(19)

**Europäisches Patentamt**
**European Patent Office**
**Office européen des brevets**

(11)  **EP 4 588 364 A1**

(12) **EUROPEAN PATENT APPLICATION**
published in accordance with Art. 153(4) EPC

(43) Date of publication:
**23.07.2025 Bulletin 2025/30**

(21) Application number: **23865625.0**

(22) Date of filing: **15.09.2023**

(51) International Patent Classification (IPC):
**A23L 33/13** (2016.01)    **A61P 25/28** (2006.01)
**A61K 31/7072** (2006.01)

(52) Cooperative Patent Classification (CPC):
**A23L 33/13; A61K 31/7072; A61P 25/28**

(86) International application number:
**PCT/JP2023/033804**

(87) International publication number:
**WO 2024/058276 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.09.2022  JP 2022147507**

(71) Applicant: **YAMASA CORPORATION
Choshi-shi, Chiba 288-0056 (JP)**

(72) Inventor: **ISHIGE,Kazuya
Choshi-shi, Chiba 288-0056 (JP)**

(74) Representative: **Maiwald GmbH
Elisenhof
Elisenstraße 3
80335 München (DE)**

(54)  **AGENT AND METHOD FOR IMPROVING COMPOSITE MEMORY OF NORMAL PERSON**

(57)    An agent for improving composite memory of healthy individuals, the agent containing uridylic acid as an active ingredient.

EP 4 588 364 A1

## Description

[Technical Field]

The present invention relates to an agent and a method for improving composite memory of healthy individuals.

[Background Art]

Cognitive functions are general intellectual functions such as understanding, judgment, and logic, and are abilities directly related to daily life. For example, in neurocognitive disorders (NCDs) classified in the Diagnostic and Statistical Manual of Mental Disorders V (DSM-5) published by the American Psychiatric Association, the cognitive function domain impaired in a patient suffering from NCD is classified into six domains: "complex attention", "executive function", "learning and memory", "language", "perceptual-motor function", and "social cognition". Improvement of the cognitive function is a universal desire shared by men and women of all ages.

Broadly divided two directions exist in research on the improvement of the cognitive function. One is "maintenance and recovery of declined cognitive function" and the other is "improvement of cognitive function in healthy individuals".

The mainstream research on the improvement of the cognitive function has so far focused on "maintenance or recovery of declined cognitive function".

"Dementia" has received the most attention in recent years as a factor contributing to the decline of the cognitive function. "Dementia" is defined as "a condition where once-acquired intellectual functions decline as a whole due to acquired brain dysfunction, causing interference with social and daily life, and is seen when there is no disturbance of consciousness" (Non-Patent Literature 1).

Along with dementia, another factor that can cause cognitive decline is "normal cognitive decline with aging (physiological amnesia)". The "normal cognitive decline with aging (physiological amnesia)" is usually distinguished from dementia, because of features that it is partial forgetting of experiences, and it does not progress or it is mild if it does progress, and that the patient retains a sense of illness, and maintains date and time awareness, and has a little disruption to daily life (Non-Patent Literature 2).

With regard to the cognitive decline with aging, it is known that while there is functional improvement in visuospatial cognition, verbal function, and verbal memory up to the 50s, numerical processing, verbal memory, perceptual speed, visuospatial cognition, reasoning, and verbal function quickly decline with aging from the 60s (Non-Patent Literature 3).

Numerous studies have been conducted on the improvement of the cognitive function, but most of them have related to recovery from or prevention of the cognitive decline. In other words, with the global increase in life expectancy and the spread of the concept of healthy life expectancy, there has been a growing demand for the recovery of the cognitive function impaired by the above "dementia" and "normal cognitive decline with aging (physiological amnesia)" as well as the maintenance of the cognitive function through the prevention of "dementia" and "normal cognitive decline with aging (physiological amnesia)", and numerous researches have been conducted. On the other hand, further cognitive enhancement from a healthy state has not been well studied.

Recently, however, much attention has been paid to "improvement of cognitive function in healthy individuals". This is a study for purposes of further improving the cognitive function originally possessed by healthy individuals, in contrast to the studies for the recovery or maintenance of the cognitive function that have been previously conducted.

In order to continue to enjoy the effect of improving the cognitive function of the healthy individuals, it is necessary to ingest an agent/material that produces the effect on a daily basis. Therefore, there has been active research on the improvement of the cognitive function by food materials that are suitable for daily ingestion.

As mentioned above, the cognitive function is an intellectual function consisting of several domains, but it is difficult to improve all of them by a single agent. Therefore, in recent research on the improvement of the cognitive function by food materials, it is expected to achieve the desired effect of improving the cognitive function by clarifying the cognitive function domains that can be improved by those food materials and ingesting foods that improve the desired functional domains or by combining multiple food materials. Examples of clarified cognitive function domains that can be improved by food materials include Patent Literature 1 which clarified that visual memory is improved by ingestion of Bifidobacterium longum N61 strain, and Non-Patent Literature 4 which clarified that attention and concentration are improved by ingestion of cassis-derived polyphenol.

Although some of food materials can improve the cognitive function across multiple cognitive function domains, there are actual situations where they are used for different purposes when the desired effect of improving cognitive function is different. For example, theanine (L-theanine), a functional food material, has been applied for a notification of Foods with Functional Claims by the same company for three cognitive function improving effects: "a function of enhancing the accuracy of attention (the ability to sustain attention and continue one action) and judgment (the accuracy and speed of judgment and the ability to process appropriately according to changing circumstances)" at a recommended daily intake of 50.3 mg or more (Non-Patent Literature 5); "supporting a verbal fluency of a person who has a low verbal fluency (the ability to recall a large number of verbal information, a part of cognitive functions, appropriately and quickly)" at a recommended daily intake of 200 mg (Non-Patent Literature 6); and "temporarily supporting working memory (the ability to sustain and correctly process multiple pieces of information necessary for solving problems), among the cognitive functions, at a recommended daily intake of 100 mg (Non-Patent Literature 7).

In the above examples, separate effects corresponding to the desired cognitive functions are produced, the content of theanine as an active ingredient is adjusted in response to the cognitive function domains, and the appeal points, consumers and sales locations of the foods and beverages containing such a food material vary, so that although there is a commonality of improving the cognitive functions, they are completely distinguished as uses.

Uridylic acid is a kind of nucleotides, and is a substance widely contained in or added to living bodies and foods, and is extremely safe and an ideal material for addition to foods.

Patent Literature 2 describes a composition for improving neuropsychological test battery [NTB] composite scores, which comprises i) uridines, etc., and ii) docosahexaenoic acid, etc. However, there is no mention in Patent Literature 2 as to whether each of the uridines, etc., produces the effect alone. In addition, the invention described in Patent Literature 2 is intended for Alzheimer's patients or dementia patients, not for healthy individuals.

Patent Literature 3 describes a composition for improving executive function, which comprises i) uridines, etc., and ii) docosahexaenoic acid, etc. However, there is no mention in Patent Literature 3 as to whether each of the uridines, etc., produces the effect alone. In addition, the invention described in Patent Literature 3 is intended for Alzheimer's patients or dementia patients, not for healthy individuals.

Patent Literature 4 describes an agent for improving learning and memory impairment, which comprises uridylic acid and so on. However, the invention described in Patent Document 4 is intended for individuals with quickly declining learning and memory ability due to aging and/or diseases, such as senile dementia.

Therefore, there were no sufficient findings as to whether uridylic acid alone has the effect of improving composite memory in healthy individuals.

[Citation List]

[Patent Literature]

[PTL 1] Japanese Patent Application Publication No. 2020-188755 A
[PTL 2] Japanese Patent Application Publication No. 2014-532696 A
[PTL 3] Japanese Patent Application Publication No. 2014-534226 A
[PTL 4] Japanese Patent Application Publication No. 2001-233776 A

[Non-Patent Literature]

[Non-PTL 1] Haruo Hanyu, et.al., "Answering Questions from Clinical Environment: Treatment for Memory Loss Q&A (p. 1)", published on July 2020, Chugai -Igakusha Co., Ltd.
[Non-PTL 2] Dementia Clinical Practice Guidelines 2017 (p. 8), published on August 1, 2017, Igaku Shoin, Inc.
[Non-PTL 3] Neuropsychol Dev Cogn B Aging Neuropsychol Cogn. 2004 June; 11(2-3): 304-324.
[Non-PTL 4] Japanese Journal of Health & Research, Vol. 2021, No. 42, p. 37-48.
[Non-PTL 5] Form I: Basic Information on Scientific Basis of Notified Food (for general consumers) Application No. F568
[Non-PTL 6] Form I: Basic Information on Scientific Basis of Notified Food (for general consumers) Application No. G600
[Non-PTL 7] Form I: Basic Information on Scientific Basis of Notified Food (for general consumers) Application No. G1323

[Summary of Invention]

[Technical Problem]

An object of the present invention is to provide a novel and highly safe agent (composite memory improving agent) that exerts an effect of improving the composite memory of healthy individuals when it is used as a food, medicine, or quasi-drug, and to provide a method for improving the composite memory of healthy individuals using that agent.

[Solution to Problem]

As a result of intensive studies to achieve the above object, the present inventors have found, for the first time, that uridylic acid has an effect of improving composite memory not only in individuals who have a rapidly deteriorated ability to learning and memory due to aging and/or diseases such as senile dementia, which are conventionally known in the art, but also in healthy individuals, and they have completed the present invention.

In other words, the present invention is an agent for improving composite memory of healthy individuals (composite memory improving agent), the agent comprising uridylic acid as an active ingredient.

The invention is also a method for improving composite memory of healthy individuals using the above agent.

[Advantageous Effects of Invention]

When used as a food, drug, or quasi-drug, the agent containing uridylic acid as an active ingredient exhibits an effect of improving composite memory of healthy individuals, and is novel and highly safe. In particular, the use of this agent will lead to provision of a novel means to improve quality of life and increase the productivity of intellectual activities through the improvement of the composite memory of the healthy individuals.

[Brief Description of Drawings]

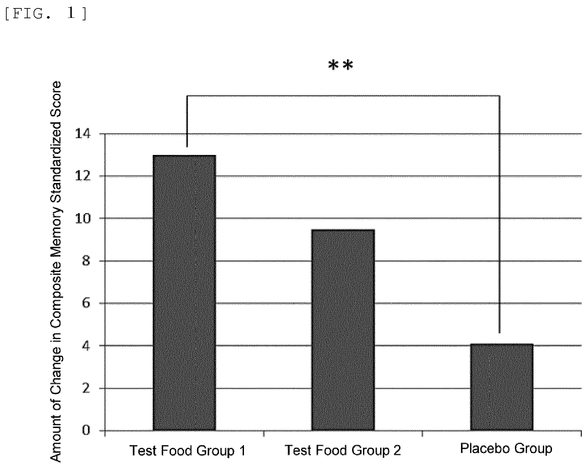

[FIG. 1] FIG. 1 shows an amount of change in composite memory standardized scores for the entire subject before and after a test, as described in Example 2-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

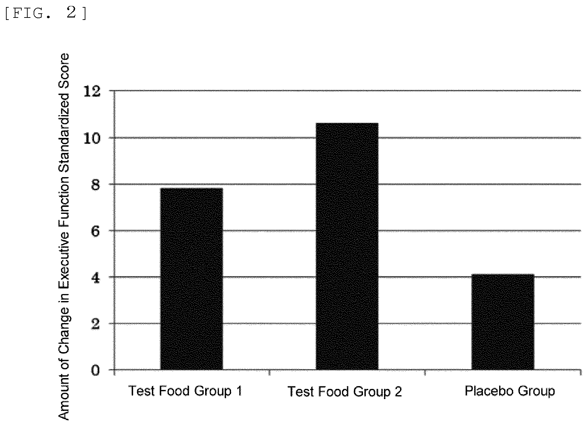

[FIG. 2] FIG. 2 shows an amount of change in executive function standardized scores for the entire subject before and after a test, as described in Example 2-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively.

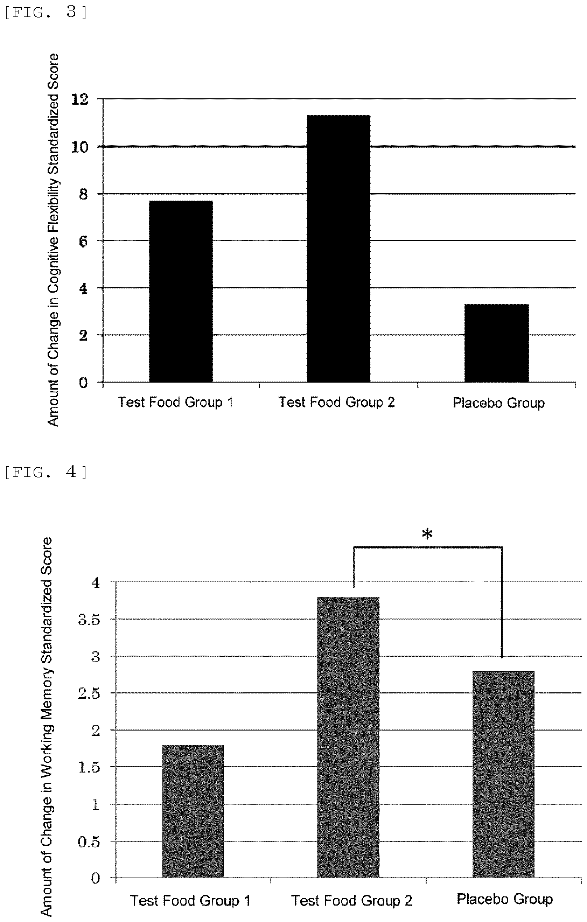

[FIG. 3] FIG. 3 shows an amount of change in cognitive flexibility standardized scores for the entire subject before and after a test, as described in Examples 2-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively.

[FIG. 4] FIG. 4 shows an amount of change in working memory standardized scores for the entire subject before and after the test, as described in Examples 2-4. The vertical axis represents the amount of change in working memory standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

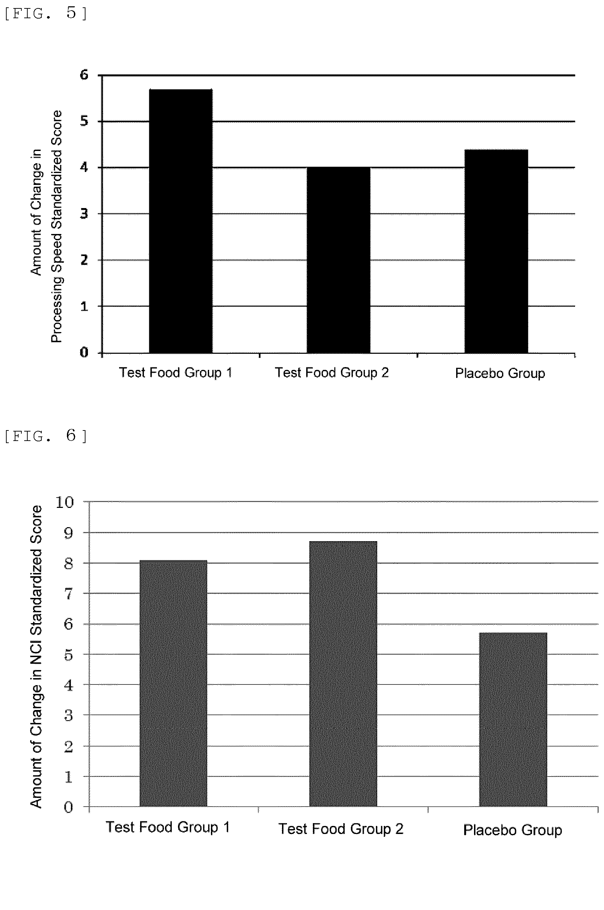

[FIG. 5] FIG. 5 shows an amount of change in processing speed standardized scores for the entire subject before and after a test, as described in Examples 2-5. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

[FIG. 6] FIG. 6 shows an amount of change in NCI standardized scores for the entire subject before and after a test, as described in Examples 2-6. The vertical axis represents the amount of change in NCI standardized scores for each test group before and after the test. In the figure, the terms "Test Food Group 1", "Test Food Group 2", and "Placebo Group" refer to corresponding groups described in Example 1, respectively.

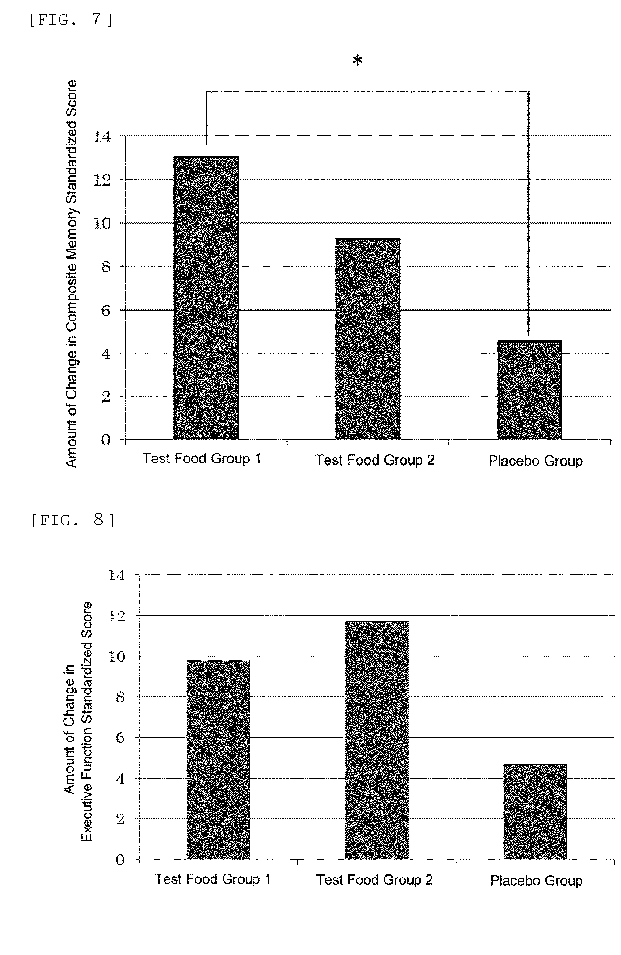

[FIG. 7] FIG. 7 shows an amount of change in composite memory standardized scores for subjects under the age of 60 before and after a test, as described in Example 3-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 8] FIG. 8 shows an amount of change in executive function standardized scores for subjects under the age of 60 before and after a test, as described in Example 3-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively.

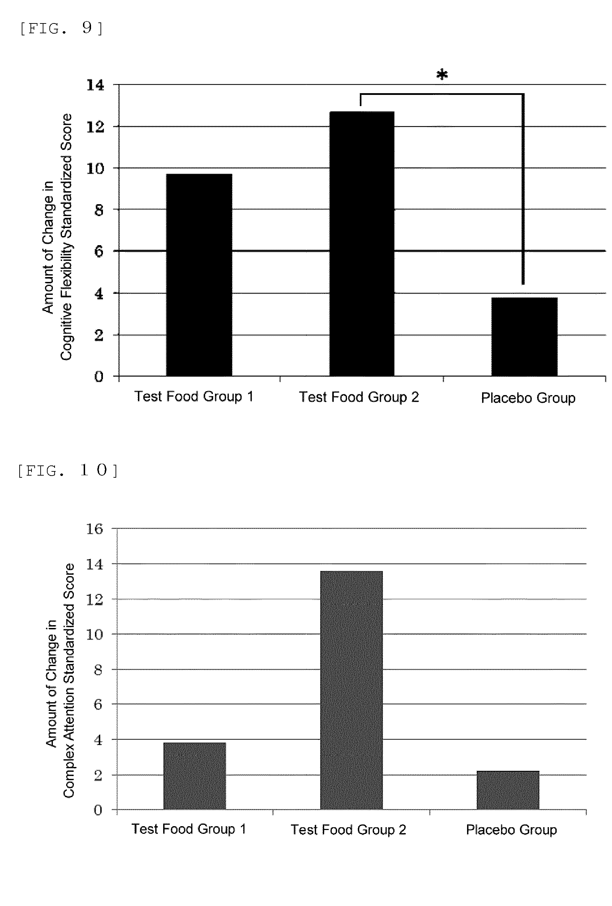

[FIG. 9] FIG. 9 shows an amount of change in cognitive flexibility standardized scores for subjects under the age of 60 before and after a test, as described in Example 3-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 10] FIG. 10 shows an amount of change in complex attention standardized scores for subjects under the age of 60 before and after a test, as described in Examples 3-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively.

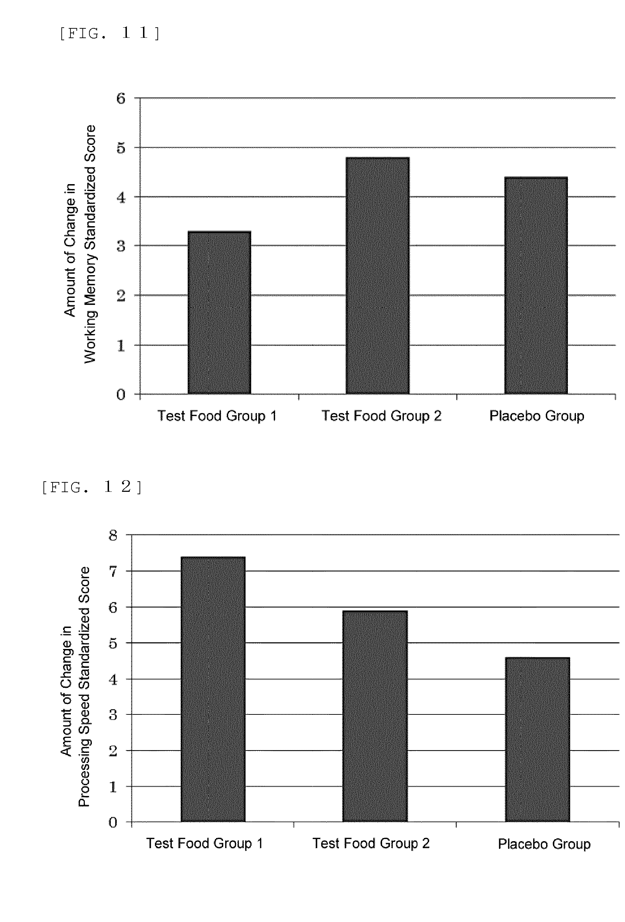

[FIG. 11] FIG. 11 shows an amount of change in working memory standardized scores for subjects under the age of 60 before and after the test, as described in Examples 3-5. The vertical axis represents the amount of change in working memory standardized scores for each test group before and after the test. In the figure, the terms "Test Food Group 1", "Test Food Group 2", and "Placebo Group" refer to corresponding groups described in Example 1, respectively.

[FIG. 12] FIG. 12 shows an amount of change in processing speed standardized scores for subjects under the age of 60 before and after a test, as described in Examples 3-6. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

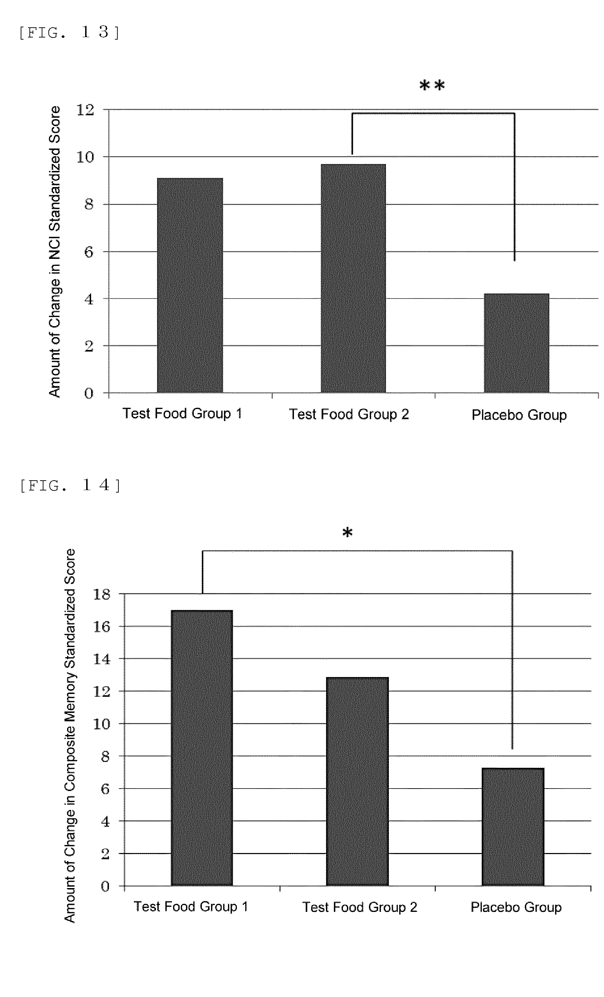

[FIG. 13] FIG. 13 shows an amount of change in NCI standardized scores for subjects under the age of 60 before and after a test, as described in Examples 3-7. The vertical axis represents the amount of change in NCI standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 14] FIG. 14 shows an amount of change in composite memory standardized scores for subjects having a composite memory standardized score of less than 90 before and after a test, as described in Example 4-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

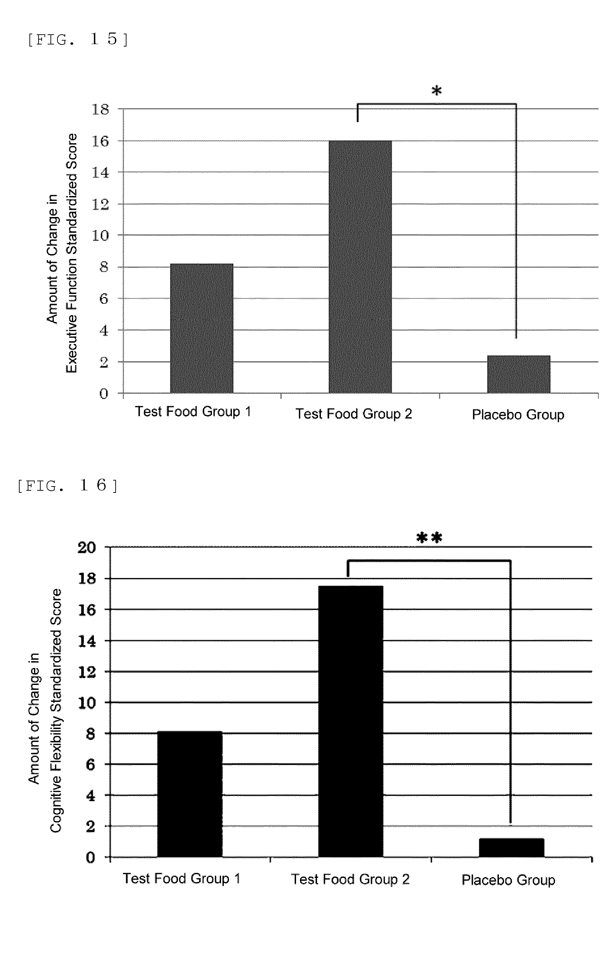

[FIG. 15] FIG. 15 shows an amount of change in executive function standardized scores for subjects having a composite memory standardized score of less than 90 before and after a test, as described in Example 4-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 16] FIG. 16 shows an amount of change in cognitive flexibility standardized scores for subjects having a composite memory standardized score of less than 90 before and after a test, as described in Example 4-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

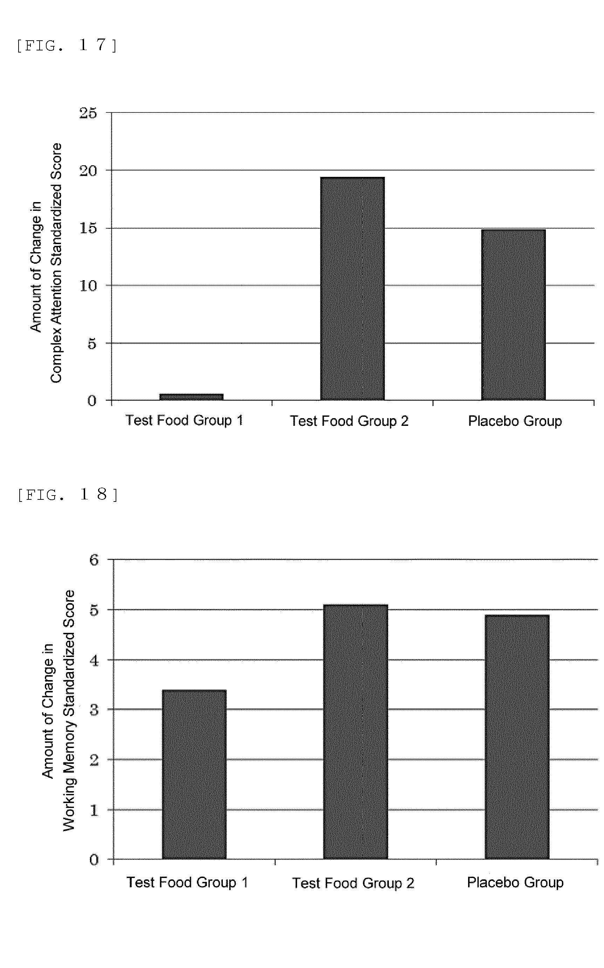

[FIG. 17] FIG. 17 shows an amount of change in complex attention standardized scores for subjects having a composite memory standardized score of less than 90 before and after a test, as described in Example 4-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively.

[FIG. 18] FIG. 18 shows an amount of change in working memory standardized scores for subjects having a composite memory standardized score of less than 90 before and after a test, as described in Examples 4-5. The vertical axis represents the amount of change in working memory standardized scores for each test group before and after the test. In the figure, the terms "Test Food Group 1", "Test Food Group 2", and "Placebo Group" refer to corresponding groups described in Example 1, respectively.

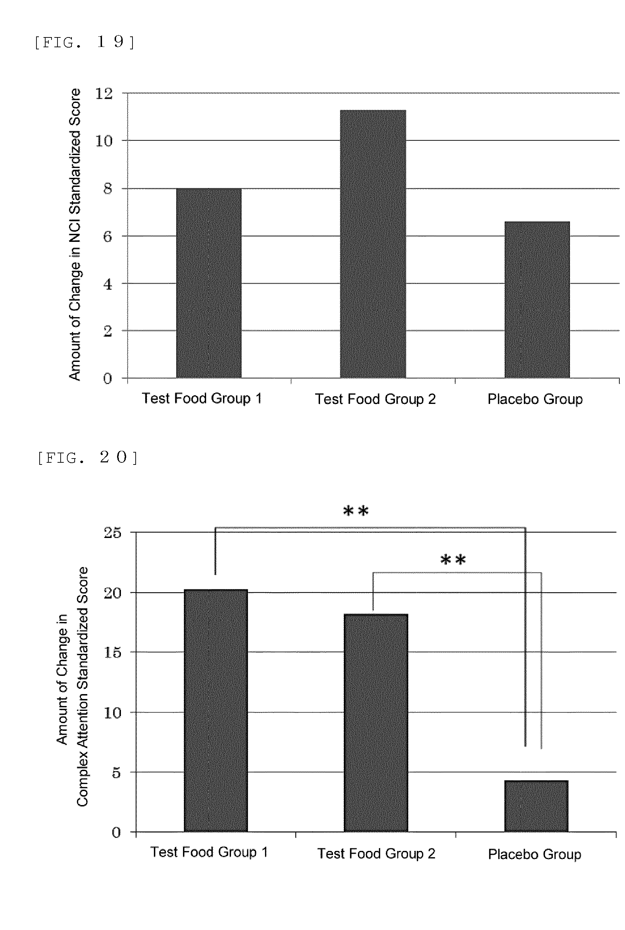

[FIG. 19] FIG. 19 shows an amount of change in NCI standardized scores for subjects having a composite memory standardized score of less than 90 before and after a test, as described in Examples 4-6. The vertical axis represents the amount of change in NCI standardized scores for each test group before and after the test. In the figure, the terms "Test Food Group 1", "Test Food Group 2", and "Placebo Group" refer to corresponding groups described in Example 1, respectively.

[FIG. 20] FIG. 20 shows an amount of change in composite memory standardized scores for subjects having a composite memory standardized score of less than 83.5 before and after the test, as described in Example 5-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

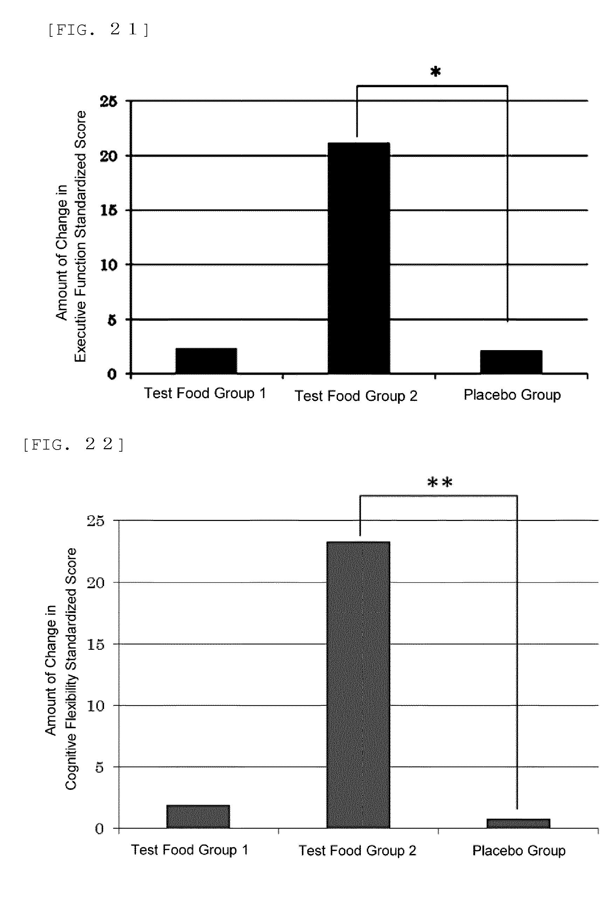

[FIG. 21] FIG. 21 shows an amount of change in executive function standardized scores for subjects having a composite memory standardized score of less than 83.5 before and after a test, as described in Example 5-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 22] FIG. 22 shows an amount of change in cognitive flexibility standardized scores for subjects having a composite memory standardized score of less than 83.5 before and after a test, as described in Example 5-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

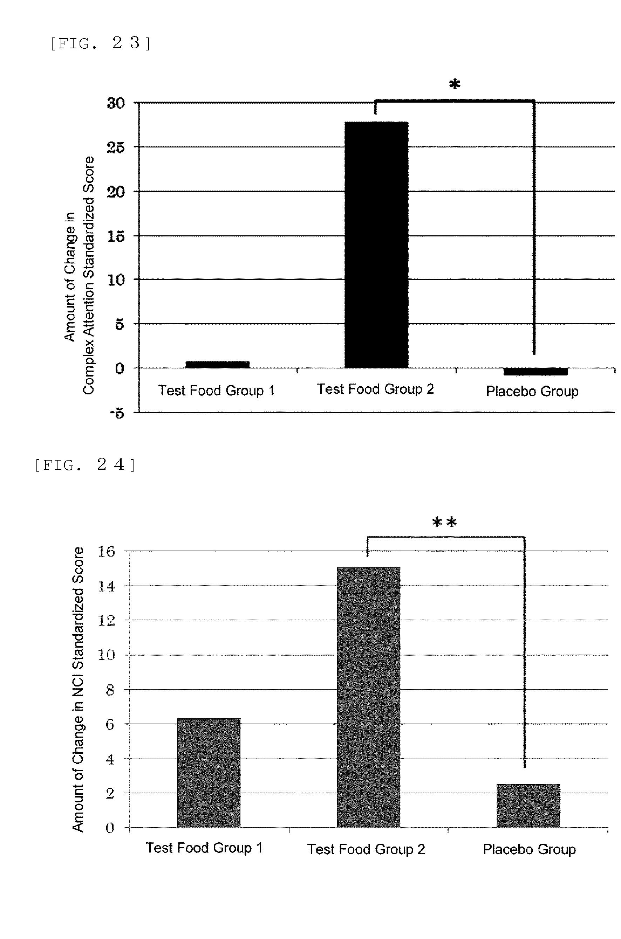

[FIG. 23] FIG. 23 shows an amount of change in complex attention standardized scores for subjects having a composite memory standardized score of less than 83.5 before and after a test, as described in Example 5-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 24] FIG. 24 shows an amount of change in NCI standardized scores for subjects having a composite memory standardized score of less than 83.5 before and after the test, as described in Example 5-5. The vertical axis represents the amount of change in NCI standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

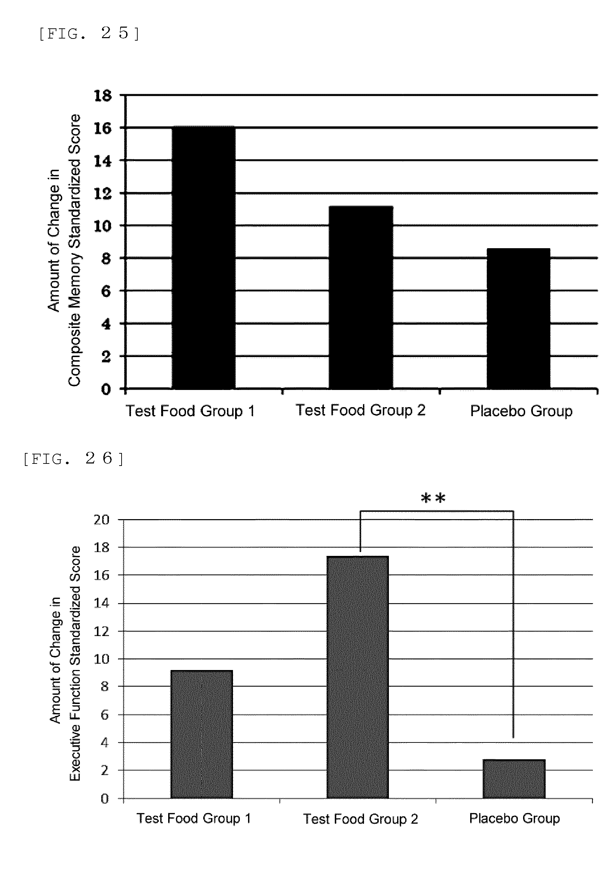

[FIG. 25] FIG. 25 shows an amount of change in composite memory standardized scores for subjects who are under the age of 60 and have a composite memory standardized score of less than 90 before and after a test, as described in Example 6-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively.

[FIG. 26] FIG. 26 shows an amount of change in executive function standardized scores for subjects who are under the age of 60 and have a composite memory standardized score of less than 90 before and after a test, as described in Example 6-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

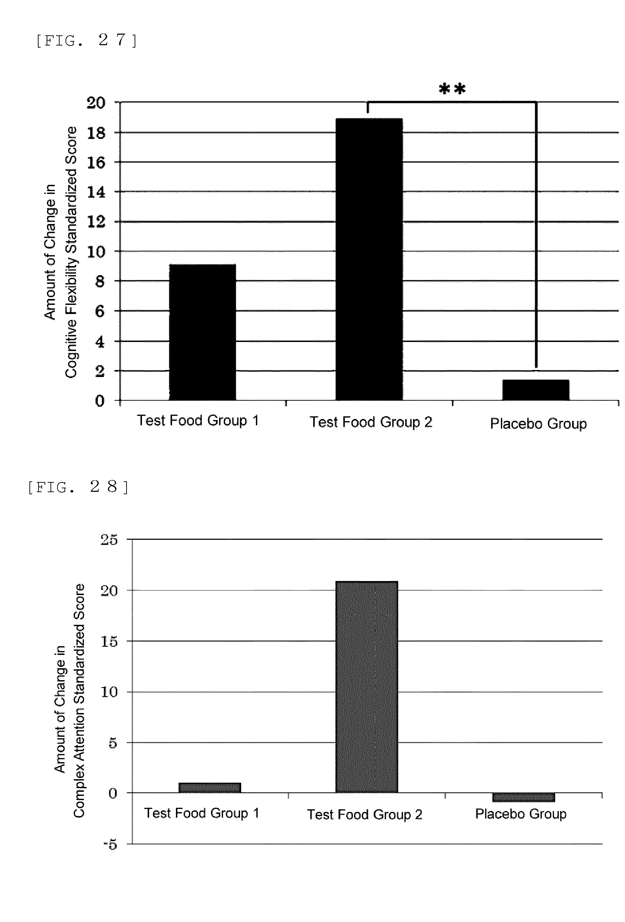

[FIG. 27] FIG. 27 shows an amount of change in cognitive flexibility standardized scores for subjects who are under the age of 60 and have a composite memory standardized score of less than 90 before and after a test, as described in Example 6-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 28] FIG. 28 shows an amount of change in a complex attention standardized scores for subjects who are under the age of 60 and have a composite memory standardized score of less than 90 before and after a test, as described in Example 6-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively.

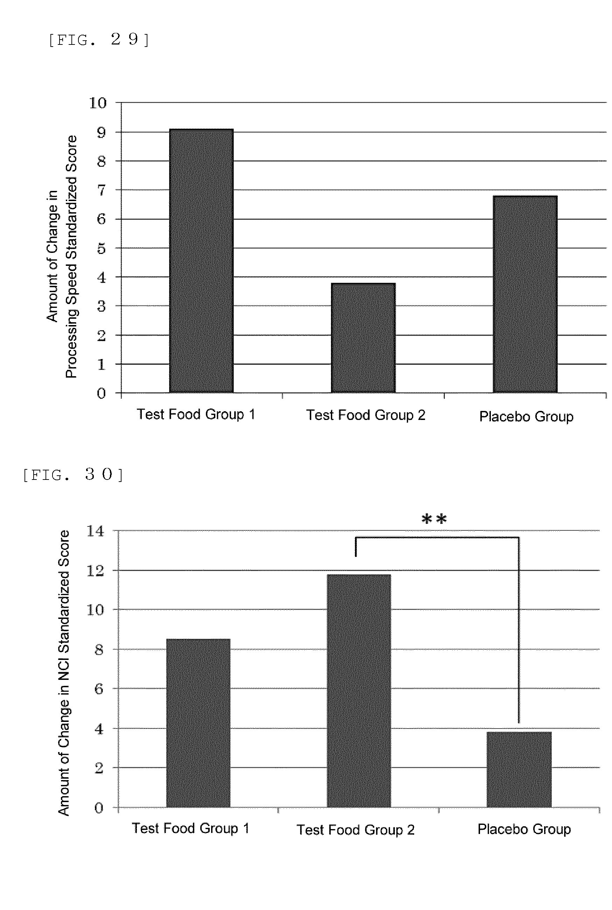

[FIG. 29] FIG. 29 shows an amount of change in processing speed standardized scores for subjects who are under the age of 60 and have a composite memory standardized score of less than 90 before and after the test, as described in Example 6-5. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

[FIG. 30] FIG. 30 shows an amount of change in NCI standardized scores for subjects who are under the age of 60 and have a composite memory standardized score of less than 90 before and after a test, as described in Example 6-6. The vertical axis represents the amount of change in NCI standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

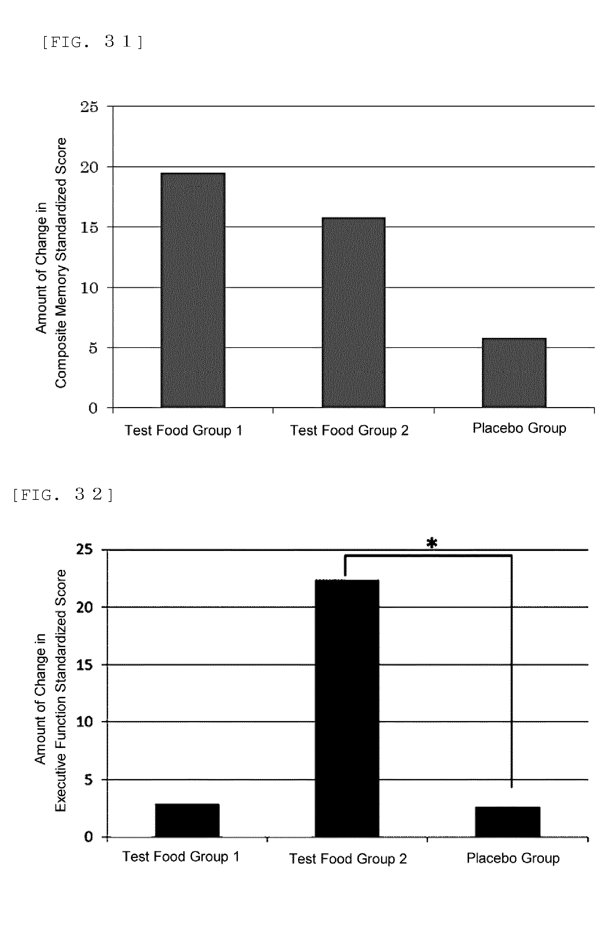

[FIG. 31] FIG. 31 shows an amount of change in composite memory standardized scores for subjects who are under the age of 60 and have a composite memory standardized score of less than 83.5 before and after a test, as described in Example 7-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively.

[FIG. 32] FIG. 32 shows an amount of change in executive function standardized scores for subjects who are under the age of 60 and have a composite memory standardized score of less than 83.5 before and after a test, as described in Example 7-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

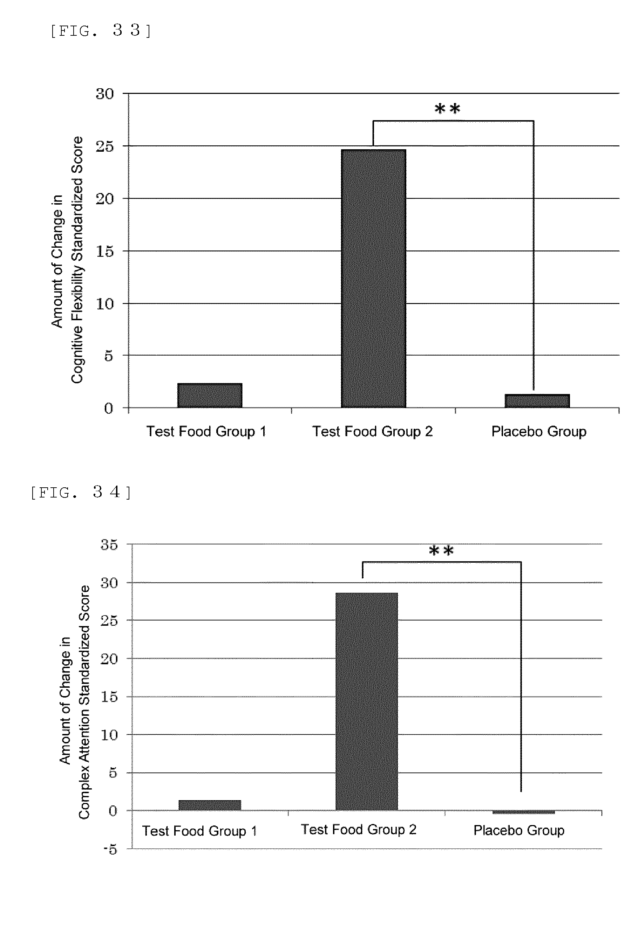

[FIG. 33] FIG. 33 shows an amount of change in cognitive flexibility standardized scores for subjects who are under the age of 60 and have a composite memory standardized score of less than 83.5 before and after a test, as described in Example 7-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 34] FIG. 34 shows an amount of change in a complex attention standardized scores for subjects who are under the age of 60 and have a composite memory standardized score of less than 83.5 before and after a test, as described in Example 7-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

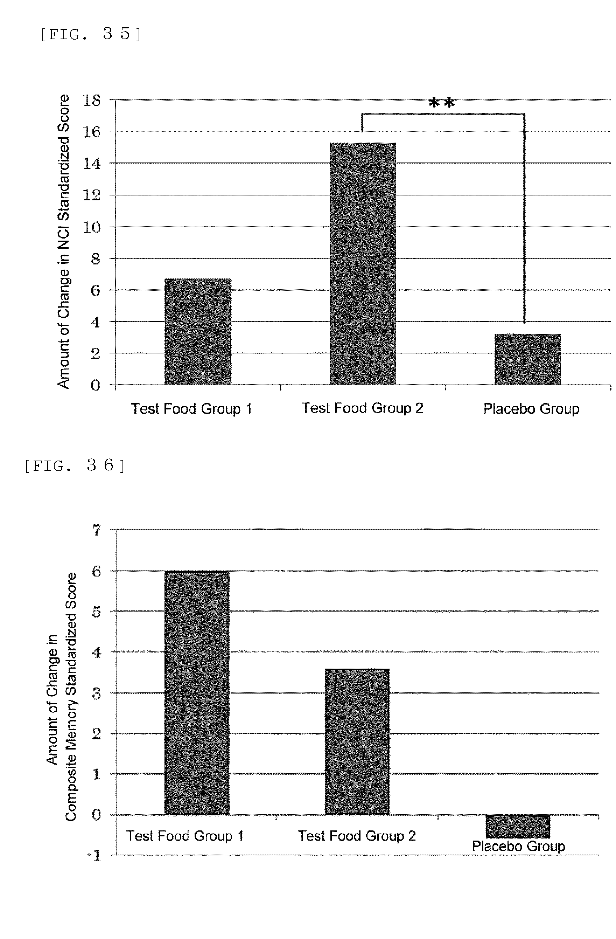

[FIG. 35] FIG. 35 shows an amount of change in NCI standardized scores for subjects who are under the age of 60 and have a composite memory standardized score of less than 83.5 before and after a test, as described in Example 7-5. The vertical axis represents the amount of change in NCI standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 36] FIG. 36 shows an amount of change in composite memory standardized scores for subjects having a composite memory standardized score of 90 or more and less than 100 before and after a test, as described in Example 8-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively.

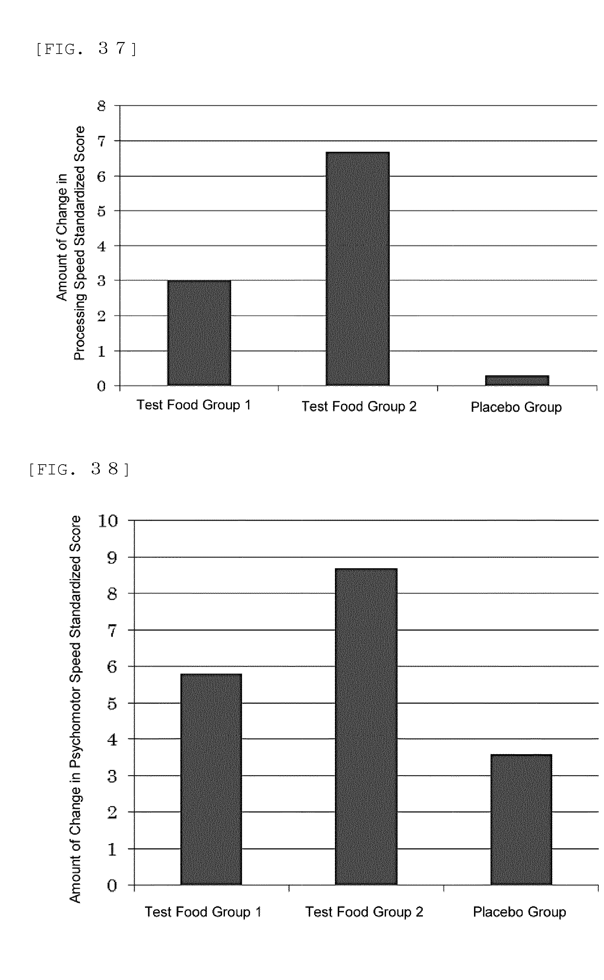

[FIG. 37] FIG. 37 shows an amount of change in processing speed standardized scores for subjects having a composite memory standardized score of 90 or more and less than 100 before and after a test, as described in Example 8-2. The vertical axis represents the amount of change in the processing speed standardized scores for each test group before and after the test. In the figure, the terms "Test Food Group 1", "Test Food Group 2", and "Placebo Group" refer to corresponding groups described in Example 1, respectively.

[FIG. 38] FIG. 38 shows an amount of change in psychomotor speed standardized scores for subjects having a composite memory standardized score of 90 or more and less than 100 before and after a test, as described in Example 8-3. The vertical axis represents the change in psychomotor speed standardized scores for each test group before and after the test. In the figure, the terms "Test Food Group 1", "Test Food Group 2", and "Placebo Group" refer to corresponding groups described in Example 1, respectively.

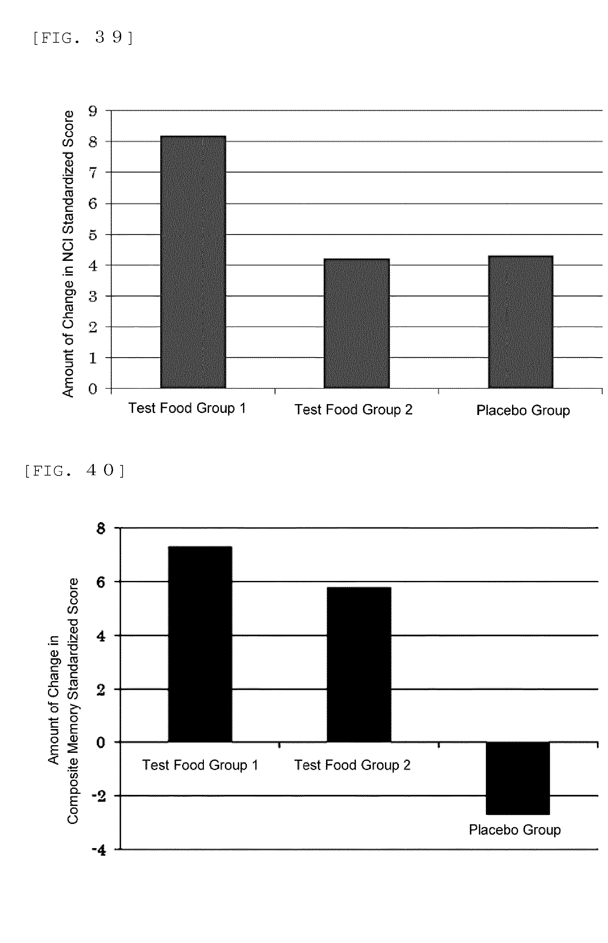

[FIG. 39] FIG. 39 shows an amount of change in NCI standardized scores for subjects having a composite memory standardized scores of 90 or more and less than 100 before and after a test, as described in Example 8-4. The vertical axis represents the amount of change in NCI standardized scores for each test group before and after the test. In the figure, the terms "Test Food Group 1", "Test Food Group 2", and "Placebo Group" refer to corresponding groups described in Example 1, respectively.

[FIG. 40] FIG. 40 shows an amount of change in composite memory standardized scores for subjects who are under the age of 60 and have a composite memory standardized scores of 90 or more and less than 100 before and after a test, as described in Example 9-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively.

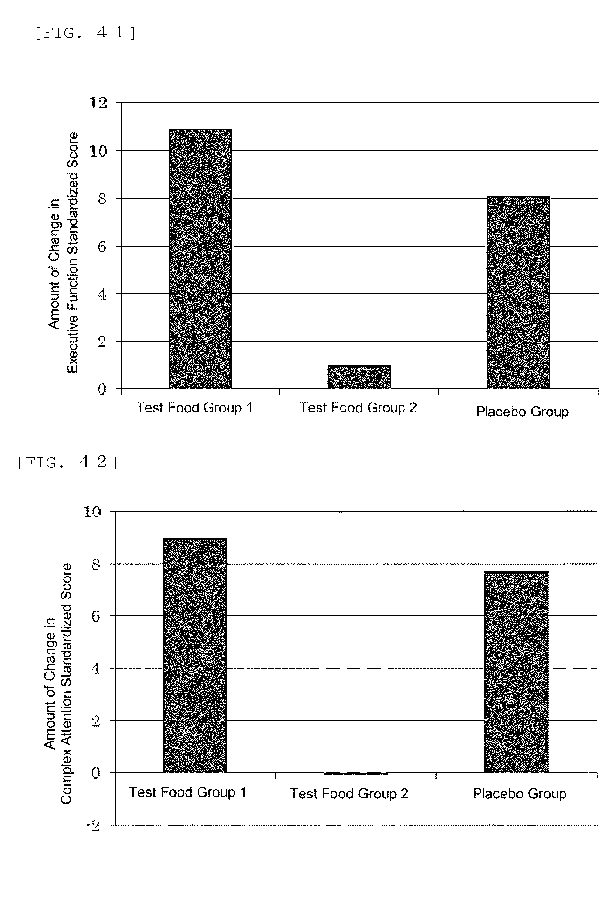

[FIG. 41] FIG. 41 shows an amount of change in executive function standardized scores for subjects who are under the age of 60 and have a composite memory standardized score of 90 or more and less than 100 before and after a test, as described in Example 9-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively.

[FIG. 42] FIG. 42 shows an amount of change in complex attention standardized scores for subjects who are under the age of 60 and have a composite memory standardized scores of 90 or more and less than 100 before and after a test, as described in Example 9-3. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively.

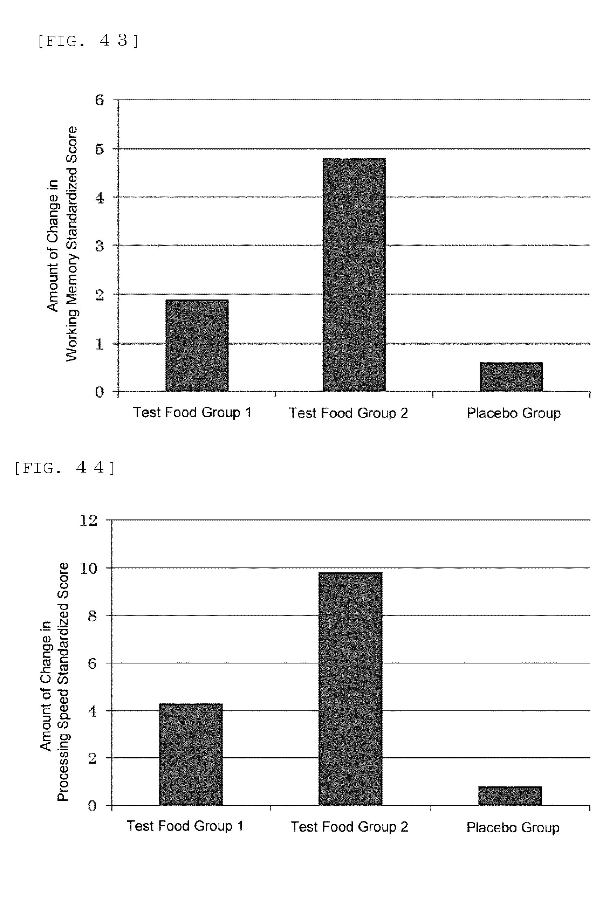

[FIG. 43] FIG. 43 shows an amount of change in working memory standardized scores for subjects who are under the age of 60 and have a composite memory standardized score of 90 or more and less than 100 before and after the test, as described in Example 9-4. The vertical axis represents the amount of change in working memory standardized scores for each test group before and after the test. In the figure, the terms "Test Food Group 1", "Test Food Group 2", and "Placebo Group" refer to corresponding groups described in Example 1, respectively.

[FIG. 44] FIG. 44 shows an amount of change in processing speed standardized scores for subjects who are under the age of 60 and have a composite memory standardized score of 90 or more and less than 100 before and after a test, as described in Example 9-5. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

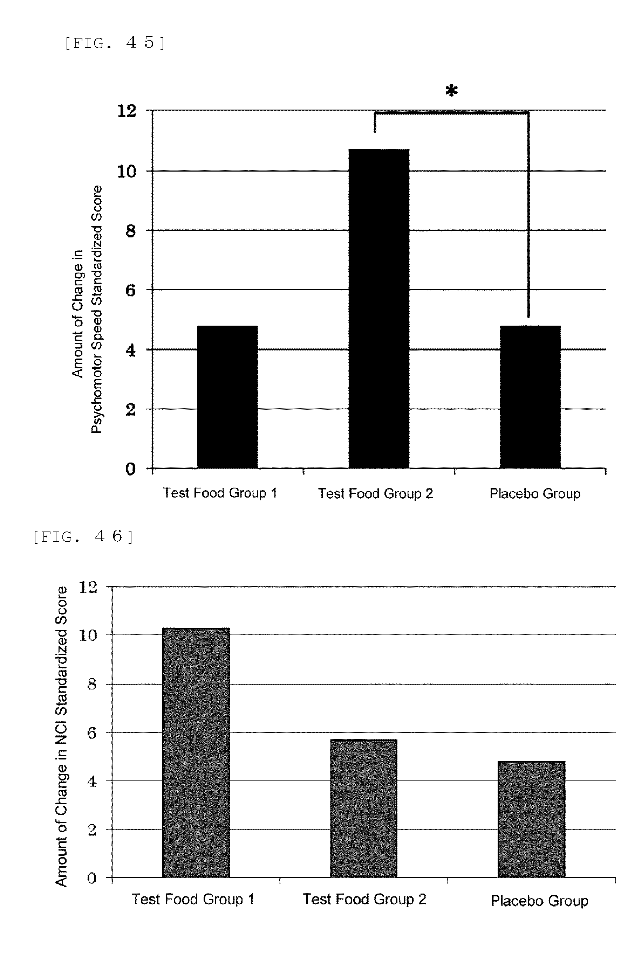

[FIG. 45] FIG. 45 shows an amount of change in psychomotor speed standardized scores for subjects who are under the age of 60 and have a composite memory standardized score of 90 or more and less than 100 before and after a test, as described in Example 9-6. The vertical axis represents the change in psychomotor speed standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 46] FIG. 46 shows an amount of change in NCI standardized scores for subjects who are under the age of 60 and have a composite memory standardized score of 90 or more and less than 100 before and after a test, as described in Example 9-7. The vertical axis represents the amount of change in NCI standardized scores for each test group before and after the test. In the figure, the terms "Test Food Group 1", "Test Food Group 2", and "Placebo Group" refer to corresponding groups described in Example 1, respectively.

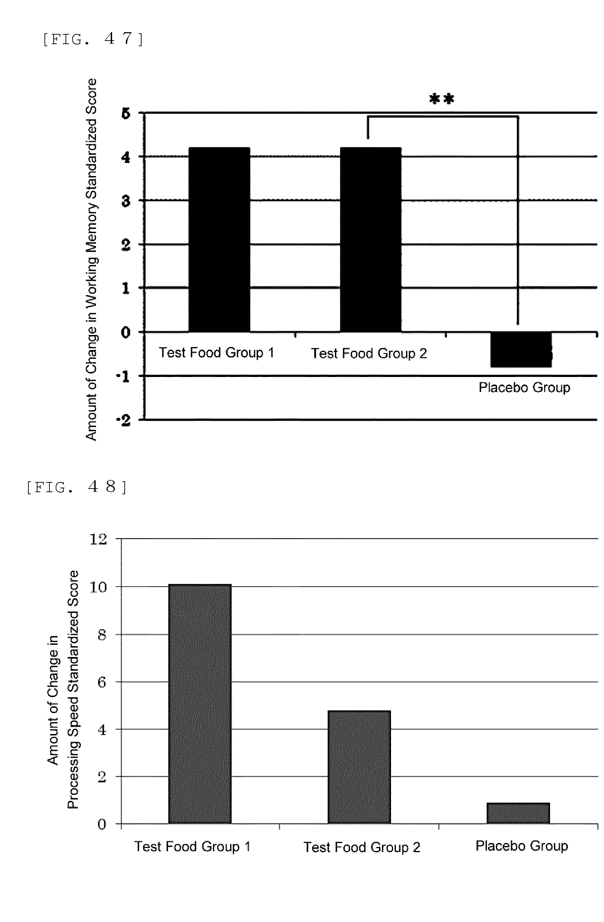

[FIG. 47] FIG. 47 shows an amount of change in working memory standardized scores for subjects having a composite memory standardized score of 83.5 or more and less than 100 before and after a test, as described in Reference Example 1. The vertical axis represents the amount of change in working memory standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 48] FIG. 48 shows an amount of change in processing speed standardized scores for subjects having a composite memory standardized score of 83.5 or more and less than 100 before and after the test, as described in Reference Example 2. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

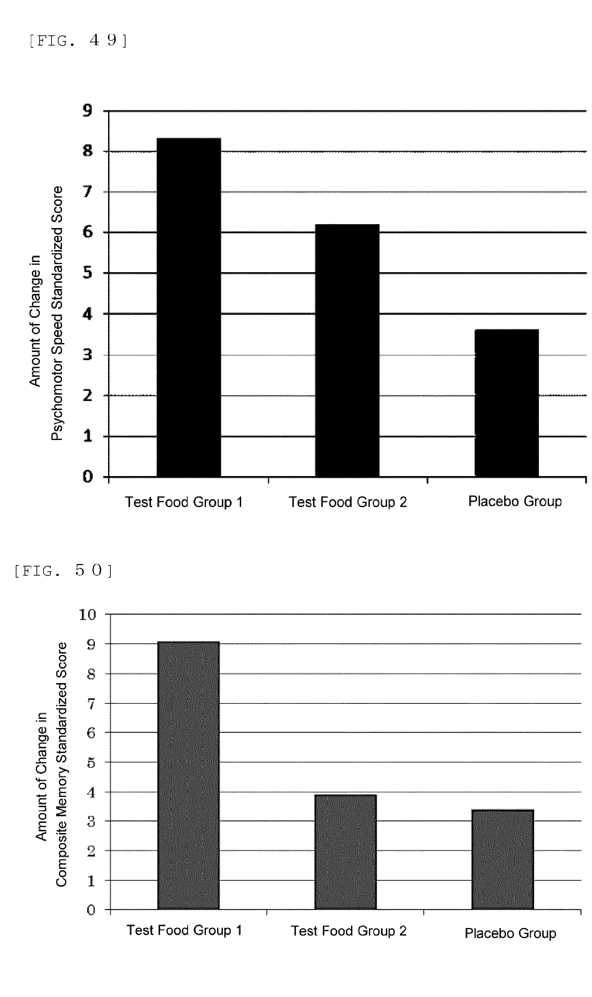

[FIG. 49] FIG. 49 shows an amount of change in psychomotor speed standardized scores for subjects having a composite memory standardized score of 83.5 or more and less than 100 before and after the test, as described in Reference Example 3. The vertical axis represents the change in psychomotor speed standardized scores for each test group before and after the test. In the figure, the terms "Test Food Group 1", "Test Food Group 2", and "Placebo Group" refer to corresponding groups described in Example 1, respectively.

FIG. 50: FIG. 50 shows an amount of change in composite memory standardized scores for subjects who are under the age of 60 and have a composite memory standardized scores of 83.5 or more and less than 100 before and after a test, as described in Reference Example 4. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively.

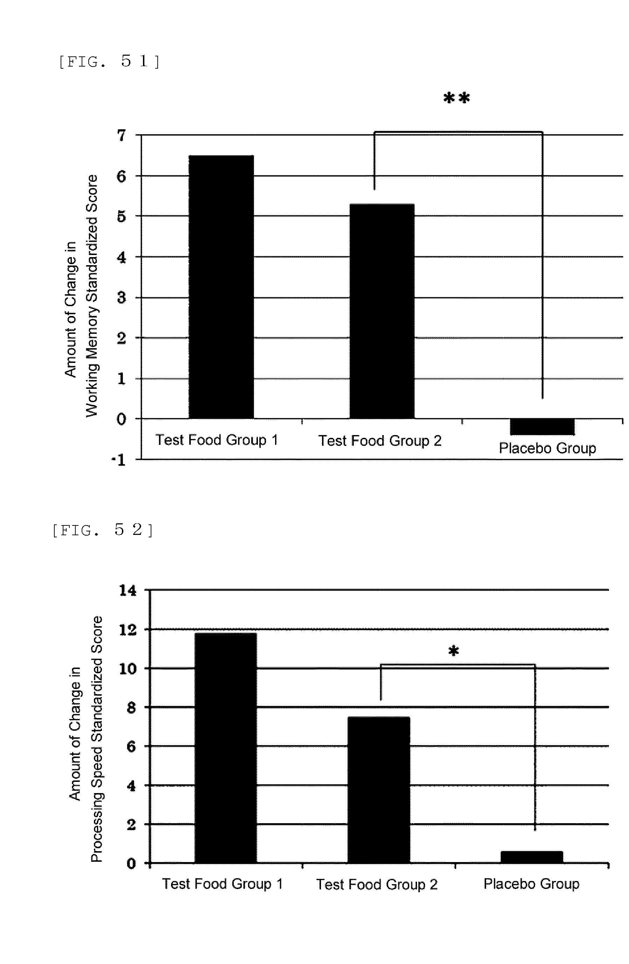

[FIG. 51] FIG. 51 shows an amount of change in working memory standardized scores for subjects who are under the age of 60 and have a composite memory standardized score of 83.5 or more and less than 100 before and after a test, as described in Reference Example 5. The vertical axis represents the amount of change in working memory standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 52] FIG. 52 shows an amount of change in processing speed standardized scores for subjects who are under the age of 60 and have a composite memory standardized score of 83.5 or more and less than 100 before and after a test, as described in Reference Example 6. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

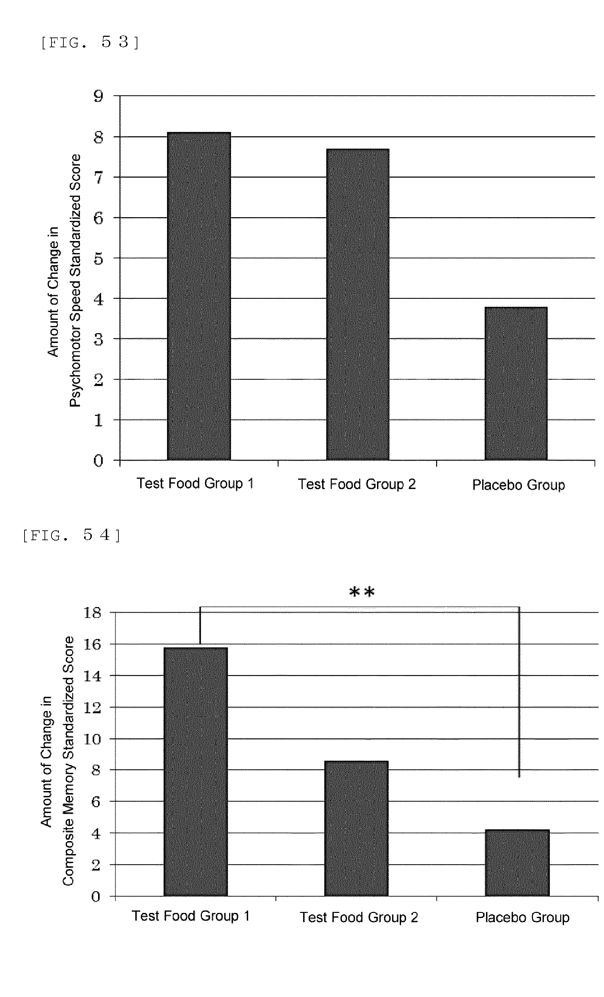

[FIG. 53] FIG. 53 shows an amount of change in psychomotor speed standardized scores for subjects who are under the age of 60 and have a composite memory standardized score of 83.5 or more and less than 100 before and after a test, as described in Reference Example 7. The vertical axis represents the change in psychomotor speed standardized scores for each test group before and after the test. In the figure, the terms "Test Food Group 1", "Test Food Group 2", and "Placebo Group" refer to corresponding groups described in Example 1, respectively.

[FIG. 54] FIG. 54 shows an amount of change in composite memory standardized scores for subject with a verbal memory standardized score of less than 90 before and after a test, as described in Example 10-1. The vertical axis represents the amount of change in the composite memory standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

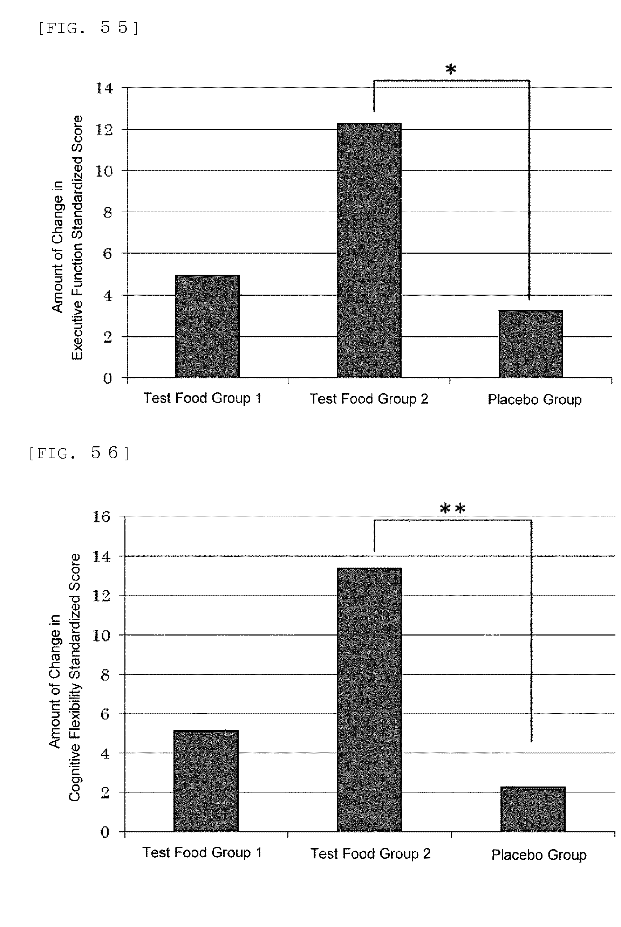

[FIG. 55] FIG. 55 shows an amount of change in executive function standardized scores for subjects having a verbal memory standardized score of less than 90 before and after a test, as described in Example 10-2. The vertical axis represents the amount of change in the executive function standardized scores for each test group before and after the test. In the figure, the terms "Test Food Group 1", "Test Food Group 2", and "Placebo Group" refer to corresponding groups described in Example 1, respectively. The symbol "*" in the figure shows a p-value < 0.10, which represents a significant trend toward the placebo group.

[FIG. 56] FIG. 56 shows an amount of change in cognitive flexibility standardized scores for subjects having a verbal memory standardized score of less than 90 before and after a test, as described in Example 10-3. The vertical axis represents the amount of change in the cognitive flexibility standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

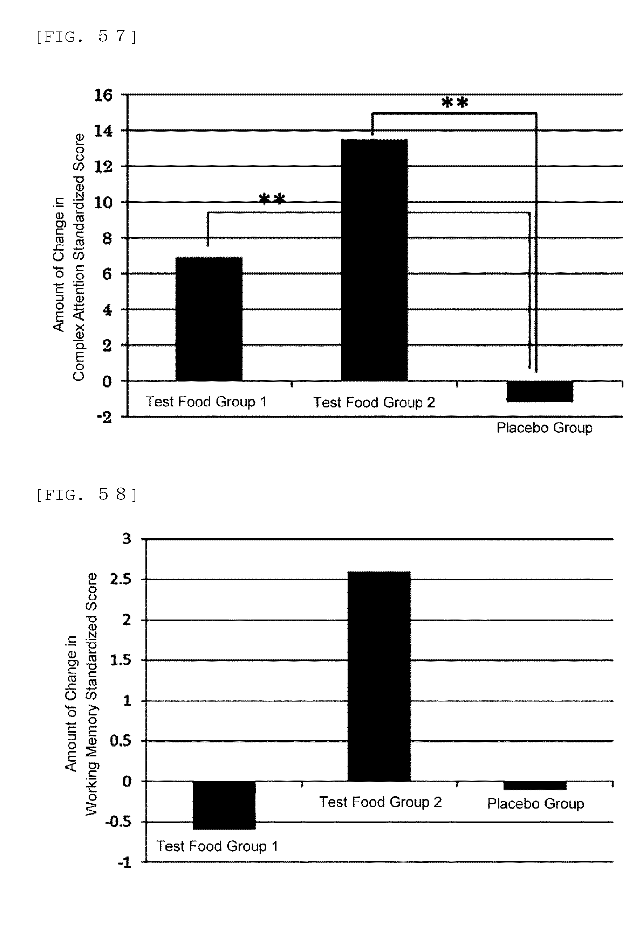

[FIG. 57] FIG. 57 shows an amount of change in complex attention standardized scores for subjects having a verbal memory standardized score of less than 90 before and after the test, as described in Example 10-4. The vertical axis represents the amount of change in the complex attention standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[FIG. 58] FIG. 58 shows an amount of change in working memory standardized scores for subjects having a verbal memory standardized score of less than 90 before and after a test, as described in Example 10-5. The vertical axis represents the amount of change in working memory standardized scores for each test group before and after the test. In the figure, the terms "Test Food Group 1", "Test Food Group 2", and "Placebo Group" refer to corresponding groups described in Example 1, respectively.

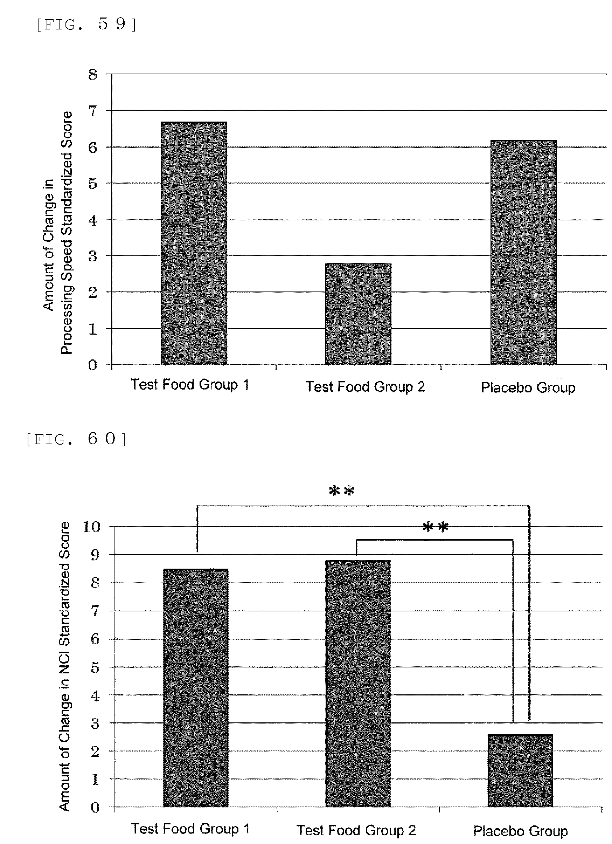

[FIG. 59] FIG. 59 shows an amount of change in processing speed standardized scores for subjects having a verbal memory standardized score of less than 90 before and after a test, as described in Example 10-6. The vertical axis represents the amount of change in the processing speed standardized score for each test group before and after the test. In the figure, the terms "Test Food Group 1", "Test Food Group 2", and "Placebo Group" refer to corresponding groups described in Example 1, respectively.

[FIG. 60] FIG. 60 shows an amount of change in NCI standardized scores for subjects having a verbal memory standardized score of less than 90 before and after a test, as described in Example 10-7. The vertical axis represents the amount of change in NCI standardized scores for each test group before and after the test. In the figure, the terms "Test Food Group 1", "Test Food Group 2", and "Placebo Group" refer to corresponding groups described in Example 1, respectively. The symbol "**" in the figure shows a p-value < 0.05, which represents a significant difference with respect to the placebo group.

[Description of Embodiments]

The present invention relates to an agent for improving composite memory of healthy individuals (composite memory improving agent), which contains uridylic acid as an active ingredient.

The composite memory as used herein refers to the overall memory, including memory for language and visual memory for figures and other objects.

Also, the composite memory as used herein refers to the cognitive function domain measured from two indices consisting of a verbal memory test (VBM) for measuring verbal memory and like in the Cognitrax testing and a visual memory test (VIM) for measuring visual memory and the like in the Cognitrax testing. The composite memory score is specifically defined as that measured by the following equation in the Cognitrax testing:

Composite Memory Score = Number of VBM Correct Hits (Immediate) + Number of VBM Correct Passes (Immediate) + Number of VBM Correct Hits (Delay) + Number of VBM Correct Passes (Delay) + Number of VIM Correct Hits (Immediate) + Number of VIM Correct Passes (Immediate) + Number of VIM Correct Hits (Delay) + Number of VIM Correct Passes (Delay)

The Cognitrax as used herein means a cognitive function testing service based on the cognitive function testing technology developed by CNS Vital Signs and designed for Japan. For the contents of the Cognitrax, the following references should also be taken into consideration.

CNS Vital Signs LLC. CNS Vital Signs Interpretation Guide. 2019. (https://www.cnsvs.com/WhitePapers/CNSVS-BriefInterpretationGuide.pdf)

As used herein, the term "Cognitrax testing" has the same meaning as the Cognitrax.

A verbal memory test (VBM) as used herein is a test conducted by the following methods: First, fifteen words are presented on the screen, and the subject memorizes them, and then finds the memorized words from the thirty words, including new words.

A visual memory test (VIM) as used herein is a test conducted by the following methods: First, fifteen geometric figures are presented on the screen, and the subject memorizes them, and then finds the memorized fitures from the thirty figures, including new figures.

The composite memory standardized score as used herein means a normalized score in a normal distribution where an average value of the composite memory scores is 100 and a standard deviation value is 15, which is calculated from the measured values. The calculations were corrected for age in 5-year increments. For example, if the measured value is the same as the average for the same age group, the standardized score is 100, and if it is better than the average by $1\sigma$, it is 115, and if it is better than the average by $2\sigma$, it is 130. The higher the composite memory standardized score, the better the composite memory.

The improvement of the composite memory as used herein means an improvement of overall memory including verbal memory and visual memory for figures, and specifically, it means an increase in the composite memory standardized score in the Cognitrax testing. The fact that the composite memory standardized score increases or decreases by 10 or more points means that the composite memory is improved or deteriorated with medical significance.

As used herein, the subjects having a composite memory standardized score of less than 90 mean individuals whose composite memory score is less than 90 when subjects are grouped in 5-year age increments and their composite memory scores standardized by the Cognitrax testing are calculated. For the calculation method for the composite memory standardized score, see Examples described below. The subjects having a composite memory standardized score of less than 90 means that they have a relatively low cognitive function for memory as compared to individuals having an average cognitive function.

As used herein, the subjects having a composite memory standardized score of less than 83.5 means individuals whose composite memory scores standardized by the method as described above are less than **83.5.** The subjects having a composite memory standardized score of less than 83.5 means that they have a significantly low cognitive function for memory as compared to individuals having an average cognitive function.

As used herein, the subjects having a composite memory standardized score of 90 or more and less than 100 means individuals whose composite memory scores are 90 or more and less than 100 when the subjects are grouped in 5-year age increments and their composite memory scores standardized by the Cognitrax testing are calculated. The subjects having a composite memory standardized score of 90 or more and less than 100 means that it is determined that the cognitive function for memory is within the normal range, although the cognitive function for memory is slightly lower.

The verbal memory as used herein refers to a cognitive function domain measured by the verbal memory test (VBM) in the Cognitrax testing. The verbal memory score is specifically defined as a score measured by the following equation in the Cognitrax testing:

Verbal Memory Score = Number of VBM Correct Hits (Immediate) + Number of VBM Correct Passes (Immediate) + Number of VBM Correct Hits (Delay) + Number of VBM Correct Passes (Delay)

As used herein, the subjects having a verbal memory standardized score of less than 90 means individuals whose verbal memory standardized scores are less than 90 when the subjects are grouped in 5-year age increments and their verbal memory standardized scores standardized by the Cognitrax testing are calculated. The subjects having a verbal memory standardized score of less than 90 means that they have a low cognitive function for verbal memory, such as a reduced ability to properly recall verbal information.

The executive function as used herein refers to an ability to recognize rules and/or concepts and make decisions rapidly.

Also, the executive function as used herein refers to overall attentional ability as measured by the shifting attention test (SAT) for measuring an ability to respond quickly and accurately to instructions in the Cognitrax testing. The executive function score is specifically defined as a score measured by the following equation in the Cognitrax testing:

$$\text{Executive Function Score = SAT Correct Responses - SAT Errors}$$

The shifting attention test (SAT) as used herein is a test conducted by the following method: Three shapes are presented on the screen, one on top and two on the bottom. Each shape is either a square or a circle, and the color is either red or blue. A subject selects a shape matched to that on the top from the bottom, but the rules change in such a manner that the "shape" is matched or the "color" is identical.

The cognitive flexibility as used herein refers to an ability to flexibly change one's thinking in response to stimuli from the external environment.

The cognitive flexibility as used herein refers to a cognitive function domain measured by two indices consisting of a stroop test (ST) for measuring cognitive flexibility and the like in the Cognitrax testing, and the shifting attention test (SAT) for measuring an ability to respond quickly and accurately to instructions. The cognitive flexibility score is specifically defined as a score measured by the following equation in the Cognitrax testing:

Cognitive Flexibility Score = SAT Correct Responses - SAT Errors - Stroop Commission Errors

The stroop test (ST) as used herein is a test conducted by the following method: The ST has three parts. In the first part, a subject presses the space bar as soon as the subject sees a word appearing on the screen. In the second part, red, yellow, blue and green words are displayed as colored characters and the subject presses the space bar when the color of the word matches the meaning of the word. In the third part, the subject presses the space bar only when the color of the word does not match the meaning of the word.

The complex attention as used herein refers to an ability to sustain attention and deal with the situation accurately.

The complex attention as used herein refers to overall attention as measured by three indices consisting of: the stroop Test (ST) for measuring executive function in the Cognitrax testing; the shifting attention test (SAT) for measuring an ability to respond quickly and accurately to instructions; and a continuous performance test (CPT) for measuring sustained attention. The complex attention score is specifically defined as a score measured by the following equation in the Cognitrax testing:

Complex Attention Score = ST Commission Errors + SAT Errors + CPT Commission Errors + CPT Omission Errors

The continuous performance test (CPT) as used herein is a test conducted by the following methods: A subject responds only when the letter **"B"** appears among the letters randomly presented on the screen for 5 minutes.

The working memory as used herein refers to a function for performing other processing tasks in parallel while temporarily holding necessary information according to diversely changing purposes.

Also, the working memory as used herein is also defined as an intellectual function as measured from the **4**-part continuous performance test (FPCPT) in the Cognitrax testing, and specifically measured by the following equation in the Cognitrax testing:

Working Memory Score = (4PCPT Part 4 Correct Responses) - --> (4PCPT Part 4 Incorrect Responses)

The 4-part continuous performance test (FPCPT) as used herein is a test conducted by the following method: The FPCPT is a test consisting of four parts. In the first part, a subject presses a space bar as soon as possible after a figure appears on the screen. In the second part, the subject presses the space bar as soon as possible after a green circle appears on the screen. In the third part, figures are presented on the screen with a combination of graphic elements (circle, triangle, rectangle, star) and color elements (red, blue, yellow, green), and the subject is asked to answer the figure presented immediately preceding. In the fourth part, figures are presented on the screen with a combination of graphic elements (circle, triangle, rectangle, star) and color elements (red, blue, yellow, green), and the subject is asked to answer the figure presented before last. The working memory is evaluated by correct/incorrect responses in the fourth part, as described above.

The psychomotor speed as used herein means an index for an ability to perceive, attend and process visual information quickly.

Also, the psychomotor speed as used herein is defined as an intellectual function as measured by a finger tapping test (FTT) and SDC test (SDC) in the Cognitrax testing, specifically as measured by the following equation in the Cognitrax testing:

Psychomotor Speed Score =FTT Right Taps Average + FTT Left Taps Average + SDC Correct Responses

An overview of the finger tapping test (FTT) is provided below. Subjects tap the space bar with their right index fingers of their left and right hands as quickly as possible for 10 seconds each.

An overview of the SDC test (SDC) is provided below. A legend representing eight symbols and corresponding numbers is presented on the top of the screen, and a table consisting of eight figures and eight corresponding empty boxes is presented on the bottom of the screen. Subjects enter the numbers corresponding to the symbols in the empty table on the bottom.

The processing speed as used herein means an index for an ability to process information quickly.

The processing speed as used herein is also defined as an intellectual function as measured from the SDC test (SDC) in the Cognitrax testing, specifically as measured by the following equation in the Cognitrax testing: Processing Speed Score = SDC Correct Responses - SDC Errors

The neurocognitive function as used herein refers to overall intellectual function that synthesizes a wide range of functional domains, including memory, psychomotor speed, executive function, attention, and cognitive flexibility.

The neurocognitive function as used herein also means a function that can be objectively measured using the Neurocognition Index (hereinafter sometimes referred to as NCI) standardized score in the Cognitrax testing as an indicator. The calculation method of the NCI standardized score is described below.

To calculate the NCI standardized scores, scores for the following five cognitive functions were calculated: Composite memory, psychomotor speed, reaction time, complex attention, and cognitive flexibility. Among these, composite memory, psychomotor speed, complex attention, and cognitive flexibility are identical to those already described.

The reaction time as used herein means an index of an ability to respond quickly to instructions.

The reaction time as used herein means an intellectual function as measured from the Stroop Test (ST) in the Cognitrax testing, and specifically as measured by the following equation in the Cognitrax testing:

Reaction Time Score = (ST Complex Reaction Time Correct + Stroop Reaction Time Correct) / 2

Each cognitive function score was converted into a standardized score by the following method: Standardized Score: a normalized score in a normal distribution with a mean value of 100 and a standard deviation value of 15 was calculated from the measured values. The calculations were corrected for age in 5-year increments. For example, if the measured value is the same as the average for the same age group, the standardized score is 100, and if it is better than the average by 1 $\sigma$, it is 115, and if it is better than the average by 2 $\sigma$, it is 130.

The NCI standardized score, which is an index for overall intellectual function that synthesizes a wide range of functional domains such as memory, cognitive speed, executive function, attention, and cognitive flexibility, was calculated using the following equation:

NCI Standardized Score = (Composite Memory Standardized Score + Psychomotor Speed Standardized Score + Reaction Time Standardized Score + Complex Attention Standardized Score + Cognitive Flexibility Standardized Score) / 5

A healthy individual as used herein means a person who does not suffer from a disease that causes impairment in cognitive functions. As used herein, the disease that causes impairment in cognitive functions mean the following disease:

Neurodegenerative central nervous system diseases: Alzheimer's disease, frontotemporal dementia, Lewy body dementia/Parkinson's disease, progressive supranuclear palsy, basal ganglia degeneration, Huntington's disease, tardive granular dementia, and senile dementia of the neurofibrillary tangle type.

Vascular dementia: multiple infarct dementia, strategic single infarct dementia, small vessel disease with dementia, hypoperfusion, cerebral vascular dementia, chronic subdural hematoma. Brain tumors: primary brain tumors, metastatic brain tumors, and cancerous meningiomas.

Normal pressure hydrocephalus.

Head trauma.

Anoxic or hypoxic encephalopathy.

Neuroinfectious diseases: acute viral encephalitis (herpes simplex encephalitis, Japanese encephalitis, etc.), HIV infection (AIDS), Creutzfeldt-Jakob disease, subacute sclerosing panencephalitis and subacute rubella panencephalitis, progressive paralysis (neurosyphilis), acute pyogenic meningitis, subacute and chronic meningitis (tuberculosis and fungal), brain abscess, brain Parasites.

Organ failure and related diseases: renal failure, dialysis encephalopathy, liver failure, portal vein hepatic shunt, chronic heart failure, chronic respiratory failure.

Endocrine dysfunction and related disorders: hypothyroidism, hypopituitarism, hypoadrenocorticism, hyperparathyroidism or hypoparathyroidism, Cushing syndrome, and repetitive hypoglycemia.

Deficiency/addictive/metabolic diseases: alcoholism, Marchiafava-Bignami disease, carbon monoxide poisoning, vitamin B1 deficiency (Wernicke-Korsakoff syndrome), vitamin B12 deficiency, vitamin D deficiency, folic acid deficiency, niacin deficiency (pellagra), drug addiction, metal poisoning (mercury, manganese, lead, etc.), Wilson's disease, late onset urea cycle enzyme deficiency, and delayed urea cycle enzyme deficiency.

Autoimmune diseases such as demyelinating diseases: multiple sclerosis, acute disseminated encephalomyelitis, Behcet's disease, and Sjogren's syndrome.

Storage diseases: delayed sphingolipidosis, adrenal leukodystrophy, cerebrotendinous xanthomatosis, intraneuronal ceroid lipofuscin [deposition] disease, and diabetes mellitus.

Other diseases: mitochondrial encephalomyopathy, progressive muscular dystrophy, Fahr's disease.

The agent according to the present invention contains uridylic acid as an active ingredient. The uridylic acid is suitable as an active ingredient of the present invention, in terms of safety as a food and the like, and ease of absorption into the body.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to disodium uridylate (UMP,2Na). When a concentration (%) of a uridylic acid solution is mentioned herein, the concentration should be expressed as a mass/volume percent concentration (w/v%) unless otherwise specified, and a mass converted to UMP,2Na is used as the mass of uridylic acid. If a salt other than the disodium salt is selected, or if it is a free acid that does not form a salt, it is a mass when converted to UMP,2Na, based on an amount of substance of the uridylic acid.

The concept of uridylic acid as used herein includes salts as described above. The salts of uridylic acid include alkali metal salts such as sodium and potassium salts; alkaline earth metal salts such as calcium, magnesium and barium salts; basic amino acid salts such as arginine and lysine; ammonium salts such as ammonium and tricyclohexylammonium salts; and various alkanolamine salts such as monoethanolamine salts, diethanolamine salts, triethanolamine salts, monoisopropanolamine salts, diisopropanolamine salts and triisopropanolamine salts. Salts of uridylic acid are preferably alkali metal salts such as sodium salts. Specific examples of such alkali metal salts of uridylic acid include monosodium uridylate and disodium uridylate, and in particular, disodium uridylate is preferred from the standpoint of handleability.

The uridylic acid used in the present invention can be used in combination with other active ingredients or used alone. In particular, it is preferable that the uridylic acid is used alone, because the uridylic acid alone can significantly improve composite memory.

There is no particular limitation on the origins of the uridylic acid used for the present invention, and those derived from natural products such as yeast, bacteria, seafood, animals, and plants are suitable.

The agent according to the present invention can be used for practical purposes as a supplement, or an additive for compositions such as food and drink products, prepared milk powders, enteral nutritional supplements, and healthy food and drink products (including special health food and functional displaying food).

When the agent according to the present invention is provided as the food and drink product, health food and drink product or prepared milk powder, it can be made into a food or drink product having a composite memory improving effect by adding the agent according to the present invention to the known food and drink product as appropriate. The food and drink products of interest include milk and dairy products, seasonings, beverages, confectioneries, breads, noodles, oils and fats, processed meat products, processed marine products, processed agricultural products, frozen foods, and instant foods.

The novel food and drink products that have the composite memory improving effect can also be produced by mixing with materials for the food and drink products. The shape of the food and drink products of interest can be selected from various forms, such as tablets, granules, capsules, powders, solutions, syrups, emulsions, and pastes. In addition to the active ingredient according to the present invention, various excipients and seasoning ingredients that can be used as foods may be added as needed in the production of those food products.

The food and drink product as described above may be provided and sold as a food and drink product labeled with the health application for improving the composite memory. The act of "labeling" includes all acts to make the above application known to users, and all expressions that may evoke or analogize the above application fall under the act of "labeling" in the present invention, regardless of the purpose of the labeling, the content of the labeling, and the object or medium to be labeled.

It is preferable that the above "labeling" be made by means of expressions that enable users to directly recognize the above application. Specific examples include the act of assigning, delivering, displaying for the purpose of assignment or delivery, or importing of goods or packages of the goods in relation to the food and drink products, which describe the above application, or the act of displaying or distributing advertisement materials, price lists or transaction documents in relation to the goods, which describes the above application, and the act of providing information about these contents, which describes the above application, through electromagnetic means (e.g., through the internet).

It is preferable that the contents of the labeling are those approved by the government or the like (e.g., labeling that has been approved based on various systems established by the government and is performed in a manner based on such approval). It is also preferable to attach such labeling to packages, containers, catalogs, pamphlets, POP, and other promotional materials at the places of sales, and other documents.

When the agent according to the present invention is practically provided as a supplement, or a pharmaceutical, enteral nutritional product, and the like, the above agent according to the present invention can be formulated alone or in combination with other components such as formulation aids. The formulations may be orally or parenterally administered, and they are preferably orally or enterally administrated.

The formulations as described above can be in the forms such as tablets, granules, capsules, pills, dispersions, solutions, syrups, and emulsions for oral administration, and injections, sprays, ointments, and patches for parenteral administration.

In addition to the active ingredient according to the present invention, the formulation may use any formulation aid such as excipients, binders, disintegrants, lubricants, taste masking agents, dissolution aids, suspending agents, coating agents, and the like, appropriately in combination, according to each dosage form.

The amount of the above active ingredient formulated in the agent according to the present invention may be appropriately selected from the range of 0.1 to 99% (w/w), depending on the purpose of use, age of the subject, method of administration or ingestion, dosage form, and the like.

The amount of uridylic acid in the agent according to the present invention can be set as desired, and a daily dosage (or intake) of uridylic acid is given as 10 mg to 1000 mg, for example. As can be seen from Examples described below, the effect of improving composite memory tends to increase as uridylic acid increases, and so the uridylic acid dosage is preferably 50 mg or more, and more preferably 100 mg or more, and even more preferably 150 mg or more, and even more preferably 200 mg or more, and even more preferably 300 mg or more, and still more preferably 400 mg or more, and still more preferably 450 mg or more, and still more preferably 500 mg or more, and most preferably 600 mg or more. From the viewpoint that a high composite memory improving effect has been demonstrated in Examples described below, it is preferably in a range of around 300 mg and/or around 600 mg, and more preferably in a range of around 600 mg, and even more preferably 450 mg to less than 750 mg, and even more preferably 500 mg to 700 mg, and particularly preferably 550 mg to 650 mg, and most preferably 600 mg.

A period of administration or intake of the agent according to the present invention is not particularly limited, but it is preferable to continuously administer or ingest uridylic acid, because the uridylic acid contained as an active ingredient is highly safety as a food or the like. A suitable period of administration or ingestion is preferably 1 week or longer, and more preferably 2 weeks or longer, and even more preferably 4 weeks or longer, and even more preferably 8 weeks or longer, and particularly preferably 12 weeks or longer in terms of the effect demonstrated Examples described below.

Subjects for improving composite memory by the agent according to the present invention are healthy individuals who do not suffer from diseases that cause cognitive decline, such as dementia.

From the viewpoint that the present invention can improve the composite memory of subjects who have not experienced age-related decline in cognitive functions, those who have not yet developed symptoms of age-related decline in normal cognitive function (physiological amnesia) are preferred, and those under the age of 60 who have a high probability of not having experienced age-related decline in the normal cognitive function (physiological amnesia) are more preferred, and those in their 40s and 50s are even more preferred from the viewpoint that a clear and significant composite memory improving effect is observed, as is clear in Examples below.

The dosage or intake of the agent according to the present invention can be selected as needed, as it will vary depending on the subject's age, weight, degree of composite memory, degree of verbal memory, desired cognitive function domains, and the like.

The cognitive function domains that are expected to be improved simultaneously with the composite memory by the present invention include executive function, cognitive flexibility, complex attention, working memory, processing speed, and psychomotor speed. It is preferable that the composite memory and one or more of the cognitive function domains illustrated above are simultaneously improved, it is more preferable that two or more be improved, it is even more preferable that three or more be improved, and it is particularly preferable that four or more be improved. Furthermore, the improvement of NCI, the overall index of neurocognitive function, is more preferable.

Specific combinations of the dosage or intake of the agent according to the present invention, the age, the degree of composite memory, the degree of verbal memory, and the desired cognitive function domains of the subjects are exemplified below.

As demonstrated in Example 2-1 below, when uridylic acid is administered or ingested in a daily dosage of 450 mg to 750 mg in healthy individuals, the composite memory of the healthy individuals is improved remarkably with medical significance and clearly enough to be determined to have a significant difference. For the dosage or intakes, the improvements of executive function, cognitive flexibility, processing speed, and neurocognitive function are expected, together with the improvement of the composite memory, as described in Examples 2-2 through 2-6 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 2-1 below, the composite memory of healthy individuals is improved when uridylic acid is administered or ingested in a daily dosage of 150 mg to less than 450 mg in healthy individuals. For the dosage or intake, the improvements of executive function, cognitive flexibility, working memory, and neurocognitive function are expected, together with the improvement of the composite memory, as described in Examples 2-2 to 2-6 below. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 3-1 below, when uridylic acid is administered or ingested in a daily dosage of 450 mg to 750 mg to the healthy individuals under the age of 60, the composite memory of the healthy individuals under the age of 60 is improved remarkably with medical significance and clearly enough to be determined to have a significant trend. For the dosage or intake, the improvements of executive function, cognitive flexibility, complex attention, processing speed, and neurocognitive function are expected, together with the improvement of the composite memory, as described in Examples 3-2 through 3-7 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 3-1 below, when a daily dose or intake of uridylic acid is 150 mg to 450 mg for the healthy individuals under the age of 60, the composite memory of the healthy individuals under the age of 60 is improved. For the dosage or intake, the improvements of executive function, cognitive flexibility, complex attention, working memory, processing speed, and neurocognitive function are expected, together with the improvement of the composite memory, as described in Examples 2-2 to 2-6 below. In particular, the cognitive flexibility and the neurocognitive function are clearly improved to such an extent that significant differences or significant trends are determined. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 4-1 below, when a daily dosage or intake of uridylic acid is 450 mg to 750 mg for healthy individuals having a composite memory standardized score of less than 90 before administration or ingestion of uridylic acid, the composite memory is clearly improved remarkably with medical significant and clearly to such an extent that significant trends are determined. For the dosage or intake, the improvements of executive function, cognitive flexibility, working memory, and neurocognitive function are expected, as well as the improvement of the composite memory, as described in Examples 4-2 to 4-6 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 4-1 below, when a daily dosage or intake of uridylic acid is 150 mg to 450 mg for healthy individuals having a composite memory standardized score of less than 90 before administration or ingestion of uridylic acid, the composite memory is clearly improved remarkably with medical significant. For the dosage or intake, the improvements of executive function, cognitive flexibility, complex attention, working memory, and neurocognitive function are expected, as well as the improvement of the composite memory, as described in Examples 4-2 to 4-6 below. In particularly, the executive function and the cognitive flexibility are improved remarkably with medical significance and clearly enough to be determined to have significant differences. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 5-1 below, when a daily dosage or intake of uridylic acid is 450 mg to 750 mg for healthy individuals having a standardized composite memory score of less than 83.5 before administration or ingestion of uridylic acid, the composite memory is clearly improved remarkably with medical significant and clearly enough to be determined to have significant differences. For the dosage or intake, the improvements of executive function, cognitive flexibility, complex attention, and neurocognitive function are expected, together with the improvement of the composite memory, as described in Examples 5-2 to 5-5 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 5-1 below, when a daily dosage or intake of uridylic acid is 150 mg to 450 mg for healthy individuals having a standardized composite memory score of less than 83.5 before administration or ingestion of uridylic acid, the composite memory is clearly improved remarkably with medical significant and clearly enough to be determined to have significant differences. As described in Examples 5-2 to 5-5 below, at that dosage or intake level, it is expected that the executive function, cognitive flexibility, complex attention, and neurocognitive function is improved, together with the improvement of the composite memory, clearly to such an extent that significant differences or significant trends are determined. Furthermore, when focusing on the composite memory, the executive function, the cognitive flexibility, and the complex attention, they are improved with remarkably with medical significant and clearly to such an extent that significant differences or trends are determined. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 6-1 below, when a daily dosage or intake of uridylic acid is 450 mg to 750 mg for healthy individuals having a composite memory standardized score of less than 90 and under the age of 60 before administration or ingestion of uridylic acid, the composite memory is clearly improved remarkably with medical significant. For the dosage or intake, the improvements of executive function, cognitive flexibility, complex attention, processing speed, and neurocognitive function are expected, together with the improvement of the composite memory, as described in Examples 6-2 to 6-6 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 6-1 below, when a daily dosage or intake of uridylic acid is 150 mg to 450 mg for healthy individuals having a composite memory standardized score of less than 90 and under the age of 60 before administration or ingestion of uridylic acid, the composite memory is clearly improved remarkably with medical significant. For the dosage or intake, the improvements of executive function, cognitive flexibility, complex attention, and neurocognitive function are expected, together with the improvement of the composite memory, as described in Examples 6-2 to 6-6 below. In particular, the executive function, the cognitive flexibility, and the neurocognitive function are clearly improved to an extent enough to be determined to have significant differences. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 7-1 below, when a daily dosage or intake of uridylic acid is 450 mg to 750 mg for healthy individuals having a composite memory standardized score of less than 83.5 and under the age of 60 before administration or ingestion of uridylic acid, the composite memory is improved. For the dosage or intake, the improvements of executive function, cognitive flexibility, complex attention, and neurocognitive function are expected, together with the improvement of the composite memory, as described in Examples 7-2 to 7-5 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 7-1 below, when a daily dosage or intake of uridylic acid is 150 mg to 450 mg for healthy individuals having a composite memory standardized score of less than 83.5 and under the age of 60 before administration or ingestion of uridylic acid, the composite memory is improved. For the dosage or intake, as described in Examples 7-2 to 7-5 below, together with the improvement of the composite memory, the executive function, cognitive flexibility, complex attention, and neurocognitive function are clearly improved to an extent enough to be determined to have significant differences. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 8-1 below, when a daily dosage or intake of uridylic acid is 450 mg to 750 mg for healthy individuals having a composite memory standardized score of 90 or more and less than 100 before administration or ingestion of uridylic acid, the composite memory is improved. For the dosage or intake, the improvements of processing speed, psychomotor speed, and neurocognitive function are expected, together with the improvement of the composite memory, as described in Examples 8-2 to 8-4 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 8-1 below, when a daily dosage or intake of uridylic acid is 150 mg to 450 mg for healthy individuals having a composite memory standardized score of 90 or more and less than 100 before administration or ingestion of uridylic acid, the composite memory is improved. For the dosage or intake, the improvements of processing speed, psychomotor speed, and neurocognitive function are expected, together with the improvement of the composite memory, as described in Examples 8-2 to 8-4 below. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 9-1 below, when a daily dosage or intake of uridylic acid is 450 mg to 750 mg for healthy individuals having a composite memory standardized score of 90 or more and less than 100 and under the age of 60 before administration or ingestion of uridylic acid, the composite memory is improved. For the dosage or intake, the improvements of executive function, complex attention, working memory, processing speed, psychomotor speed, and neurocognitive function are expected, together with the improvement of the composite memory, as described in Examples 9-2 to 9-7 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 9-1 below, when a daily dosage or intake of uridylic acid is 150 mg to 450 mg for healthy individuals having a composite memory standardized score of 90 or more and less than 100 and under the age of 60 before administration or ingestion of uridylic acid, the composite memory is improved. For the dosage or intake, as described in Examples 9-2 to 9-7 below, the executive function, complex attention, working memory, processing speed, psychomotor speed, and neurocognitive function are improved together with the improvement of the composite memory. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

As demonstrated in Example 10-1 below, when a daily dosage or intake of uridylic acid is 450 mg to 750 mg for healthy individuals having a verbal memory standardized score of less than 90 before administration or ingestion of uridylic acid, the composite memory is improved remarkably with medical significant and clearly to an extent enought to be determined to have significant trends. For the dosage or intake, the improvements of executive function, cognitive flexibility, complex attention, processing speed, and neurocognitive function are expected, together with the improvement of the composite memory, as described in Examples 10-2 through 10-6 below. From the viewpoint that the effect can be more reliably expected because the effect has been demonstrated in Examples, the daily dosage or intake of uridylic acid is preferably 500 mg to 700 mg, and more preferably 550 mg to 650 mg, and even more preferably 600 mg.

As demonstrated in Example 10-1 below, when a daily dosage or intake of uridylic acid is 150 mg to 450 mg for healthy individuals having a verbal memory standardized score of less than 90 before administration or ingestion of uridylic acid, the composite memory is improved. For the dosage or intake, the improvements of executive function, cognitive flexibility, general attention, working memory, and neurocognitive function are expected, together with the improvement of the composite memory, as described in Examples 10-2 to 10-6 below. From the viewpoint that the effect can be more reliably expected because the effect of uridylic acid has been demonstrated in Examples, the daily dosage or intake of uridylic acid is preferably 200 mg to 400 mg, and more preferably 250 mg to 350 mg, and even more preferably 300 mg.

The effects of the present invention can be summarized in terms of the improvement of the composite memory as follows:

As described below, in Example 2-1 in which all subjects were analyzed, the amount of change in the composite memory standardized score was increased as compared with the placebo group, indicating that the present invention widely improves the composite memory regardless of the attributes of the subjects. Such effects are supported by the fact that the amount of change in the composite memory standardized score was also increased in the analysis of the subject subgroups in Examples 3-1 to 10-1 described below, as compared with the placebo group.

Among others, based on the results of Examples described below, the present invention improves the composite memory with medical significance for the following subject attributes and/or the amount of uridylic acid ingested:

Overall subjects: 450 mg to less than 750 mg;
Under the age of 60: 450 mg to 750 mg;
Composite memory standardized score of less than 90 before UMP ingestion: 450 mg to 750 mg or 150 mg to 450 mg;
Composite memory standardized score of less than 83.5 before UMP ingestion: 450 mg to 750 mg or 150 mg to 450 mg;
Under the age of 60 and composite memory standardized score of less than 90 before UMP ingestion: 450 mg to 750 mg or 150 mg to 450 mg;
Under the age of 60 and composite memory standardized score of less than 83.5 before UMP ingestion: 450 mg to 750 mg or 150 mg to 450 mg;
Verbal memory standardized score of less than 90 before UMP ingestion: 450 mg to less than 750 mg.

In particular, based on the results of Examples described below, the present invention improves the composite memory remarkably with medical significance and clearly to an extent enough to be determined to have significant differences or significant trends, for the following attributes of the subjects and/or the amount of uridylic acid ingested:

Overall subjects: 450 mg to less than 750 mg;
Under the age of 60: 450 mg to 750 mg;
Composite memory standardized score of less than 90 before UMP ingestion: 450 mg to 750 mg;
Composite memory standardized score of less than 83.5 before UMP ingestion: 450 mg to 750 mg or 150 mg to 450 mg;
Verbal memory standardized score of less than 90 before UMP ingestion: 450 mg to 750 mg.

One aspect of the present invention is a method for improving composite memory of healthy individuals. As demonstrated in Examples below, the present invention can improve the composite memory of healthy individuals.

[Examples]

The present invention will be described by Examples, but the present inventio is not limited by these Examples.

(Example 1) Screening of Test Subjects and Assignment to Subgroups

To test the effect of uridylic acid on the improvement of composite memory, the following procedure was used to screen test subjects and assign them to subgroups.

[Screening of Test Subjects]

From 200 individuals who met all of criteria: Japanese (both male and female), over 40 years old, and healthy individuals were excluded those who were considered to be ineligible for the test (e.g., those who consumed foods or supplements that might affect the cognitive function such as DHA/EPA, Ginkgo biloba extract, tocotrienols, astaxanthin, GABA, phosphatidylserine, and plasmalogens, those who regularly used devices, equipment, or apps that might affect the cognitive function, and those who regularly used drugs (including herbal medicines) or supplements, and those who were pregnant, lactating, or intending to become pregnant during the test period) to select 99 test subjects.

[Assignment of Test Food Group and Placebo Group]

The test subjects were assigned to three groups having 33 persons, respectively, so that each of attributes such as age and gender were as equal as possible. Three persons in each group were excluded due to post-assignment dropouts or noncompliance with the protocol, and finally 30 persons in each group were included as the analysis subjects. Subjects assigned to Test Food Group 1 were given a 5'-UMP-containing food (4 capsules/day). Subjects assigned to Test Food Group 2 were given 5'-UMP-containing food (2 capsules/day) and placebo (2 capsules /day). Subjects assigned to the placebo group were given placebo (4 capsules /day). A table for the compositions of the test foods and placebo is shown in Table 1 below. Subjects ingested the assigned foods with water or lukewarm water at breakfast. The ingestion of the food was continued for 12 weeks. In addition, this test was conducted as a double-blind study.

[Table 1]

| Name | Form | Ingredients | Volume (mg/capsule) |
|------|------|-------------|---------------------|
| Test Food | Hard Capsule | disodium 5'-uridylate<br>crystalline cellulose<br>calcium stearate | 150<br>131<br>9 |
| Placebo | Hard Capsule | crystalline cellulose<br>calcium stearate | 221<br>9 |

In the following description, Test Food Group 1 may be referred to as a group of daily intake of UMP of 600 mg, and Test Food Group 2 as a group of daily intake of UMP of 300 mg.

[Conduction of Cognitive Function Tests]

The Cognitrax testing, which was a cognitive testing service based on the cognitive testing technology developed by CNS Vital Signs and designed for the Japanese market, was used to conduct 10 different tests (Verbal Memory Test (VBM), Visual Memory Test (VIM), Finger Tapping Test (FTT), SDC Test (SDC), Stroop Test (ST), Shifting Attention Test (SAT), Continuous Performance Test (CPT), 4-Part Continuous Performance Test (FPCPT), Perception of Emotions Test (POET), and Non-Verbal Reasoning Test (NVRT)). In the specification of this application, it is understood that the contents of the Cognitrax testing are also be taken into account as described in the following literature:
CNS Vital Signs LLC. cnn Vital Signs Interpretation Guide. 2019.

[Calculation of Score of Each Domain]

In accordance with the descriptions of the literature outlined for the above Cognitrax testing, the total score for the following eight domains and one neurocognitive function were calculated from the results of the ten tests conducted: composite memory, executive function, cognitive flexibility, complex attention, working memory, processing speed, psychomotor speed, reaction time, neurocognition index (NCI).
The calculation method for each domain score is as follows:

[Calculation of Composite Memory Score]

Composite memory, which was an index for overall composite memory capacity that combined verbal memory and visual memory, was calculated using the following equation:

Composite Memory Score = Number of VBM Correct Hits (Immediate) + Number of VBM Correct Passes (Immediate) + Number of VBM Correct Hits (Delay) + Number of VBM Correct Passes (Delay) + Number of VIM Correct Hits (Immediate) + Number of VIM Correct Passes (Immediate) + Number of VIM Correct Hits (Delay) + Number of VIM Correct Passes (Delay)

An overview of the verbal memory test (VBM) is provided below. First, fifteen words are presented on the screen, and the subject memorizes them, and then finds the memorized words from the thirty words, including new words.
An overview of the Visual Memory Test (VIM) is provided below. First, fifteen geometric figures are presented on the screen, and the subject memorizes them, and then finds the memorized figures from the thirty figures, including new figures.

[Calculation of Executive Function Score]

The executive function, which was an index for an ability to understand rules and concepts and make decisions, was calculated by the following equation:

```
Executive Function Score = SAT Correct Responses - SAT Errors
```

An overview of the Shifting Attention Test (SAT) is provided below. Three shapes are presented on a screen, one on top and two on the bottom. The shape is either a square or a circle, and the color is either red or blue. A subject selects a shape matched to that on the top from the bottom, but the rules change in such a manner that the "shape" is matched or the "color" is identical.

[Calculation of Cognitive Flexibility Score]

Cognitive flexibility, which was an index for an ability to flexibly change one's thinking in response to stimuli from the external environment, was calculated using the following equation:

Cognitive Flexibility Score = SAT Correct Responses - SAT Errors - Stroop Commission Errors

[Calculation of Working Memory Score]

The working memory score for the subjects was measured by the following equation:

Working Memory Score = (4PCPT Part 4 Correct Responses) - (4PCPT Part 4 Incorrect Responses)

An overview of the 4-Part Continuous Performance Test (FPCPT) is provided below. The FPCPT is a test consisting of four parts. In the first part, a subject presses a space bar as soon as possible after a figure appears on the screen. In the second part, the subject presses the space bar as soon as possible after a green circle appears on the screen. In the third part, figures are displayed with a combination of graphic elements (circle, triangle, rectangle, star) and color elements (red, blue, yellow, green), and the subject is asked to answer the figure displayed immediately preceding. In the fourth part, figures are presented on the screen with a combination of graphic elements (circle, triangle, rectangle, star) and color elements (red, blue, yellow, green), and the subjects are asked to answer the figure presented two times before.

[Calculation of Complex Attention Score]

Complex attention, which was an index for an ability to sustain attention and respond accurately, was calculated using the following equation:

Complex Attention Score = ST Commission Errors + SAT Errors + CPT Commission Errors + CPT Omission Errors

In this index, a lower value indicates a higher complex attention capacity, while a higher value indicates a lower complex attention capacity.

An overview of the Continuous Performance Test (CPT) is provided below. A subject responds only when the letter "B" appears among the letters randomly presented on the screen for 5 minutes.

Processing speed, which was an index for an ability to process information quickly, was calculated using the following equation:

(Processing Speed Score) = (SDC Correct Responses) - (SDC Errors)

An overview of the SDC test (SDC) is provided below. A legend representing eight symbols and corresponding numbers is presented on the top of the screen, and a table consisting of eight figures and eight corresponding empty boxes is presented on the bottom of the screen. Subjects enter the numbers corresponding to the symbols in the empty table on the bottom.

[Calculation of Psychomotor Speed Score]

Psychomotor speed, which was an index for an ability to quickly perceive, operate, and process visual information, was calculated using the following equation:

(Psychomotor Speed Score) = (FTT Right Taps Average) + (FTT --> Left Taps Average) + (SDC Left Taps Average)

An overview of the finger tapping test (FTT) is provided below. Subjects tap the space bar with their right index fingers of their left and right hands as quickly as possible for 10 seconds each.

[Calculation of Reaction Time Score]

Reaction time, which was an index for an ability to respond quickly to instructions, was calculated using the following equation:

(Reaction Time Score) = (ST Complex Reaction Time Correct + Stroop Reaction Time Correct)/2

An overview of the Stroop Test (ST) is provided below. The ST has three parts. In the first part, a subject presses the space bar as soon as the subject sees a word appearing on the screen. In the second part, red, yellow, blue and green words are displayed in colored characters and the subject presses the space bar when the color of the word matches the meaning of the word. In the third part, the subject presses the space bar only when the color of the word does not match the meaning of the word.

[Method for Standardizing Each Score]

The following method was used to convert the actual measurements for each test and each indicator into standardized scores.

Standardized Score: a normalized score in a normal distribution with a mean value of 100 and a standard deviation value of 15 was calculated from the measured values. The calculations were corrected for age in 5-year increments. For example, if the measured value is the same as the average for the same age group, the standardized score is 100, and if it is better than the average by 1 $\sigma$, it is 115, and if it is better than the average by 2 $\sigma$, it is 130. An increase or decrease of 10 or more points in the standardized score indicates improvement or worsening of the cognitive function with medical significance.

[Calculation of the Neurocognition Index (NCI) Standardized Score]

The NCI standardized score, which is an index of overall intellectual function that synthesizes a wide range of functional domains such as memory, cognitive speed, executive function, attention, and cognitive flexibility, was calculated using the following equation:

NCI Standardized Score = (Composite Memory Standardized Score + Psychomotor Speed Standardized Score + Reaction Time Standardized Score + Complex Attention Standardized Score + --> Cognitive Flexibility Standardized Score) / 5

[Statistical Processing]

Means and standard deviations were calculated for the scores among the respective test groups obtained by the above methods and compared between groups with the placebo group using ANCOVA with the scores at screening and inspection before ingestion (baseline) as covariation. All statistical analyses were performed with two-tailed tests, and a p-value of less than 0.05 was considered significant. The software used was SPSS Statistics version 23 or above, with other validated statistical software used as needed.

[Setting of Subgroup]

In order to analyze the results of this test in detail, we extracted the results of the test for subjects whose composite memory standardized score before UMP ingestion was less than 90, subjects whose composite memory standardized score before UMP ingestion was less than 83.5, subjects whose verbal memory standardized score before the test was less than 90, subjects under age 60 (40s and 50s), and combinations of each of these attributes, and statistically processed them by the same method as described above. In the Cognitrax, the composite memory standardized score of less than 90 generally means that the subject has relatively poor cognitive function relating to memory. The composite memory standardized score of less than 83.5 means that the subject has considerably low cognitive function relating to memory. The verbal memory standardized score of less than 90 means that the subject has relatively low cognitive function relating to verbal memory. For the subjects under the age of 60 (specifically, subjects in their 40s and 50s), this means subjects in their 40s and 50s who cannot find any age-related decline in cognitive function, as described in Non-Patent Literature 3.

(Example 2) Effect of Improving Standardized Score in Each Cognitive Domain across All Subjects

The improving effects of composite memory and each cognitive domain were verified for all subjects in this test.

(Example 2-1) Effect of Improving Composite Memory Standardized Scores at All Ages

For the composite memory, which is an index for the cognitive function relating to memory, each of FIG. 1 and Table 2 shows an amount of change in composite memory standardized scores for the entire subject population before and after the test.

[Table 2]

| Test Groups | Amount of Change in Composite Memory Standardized Score | Standard Deviation | p-value |
| --- | --- | --- | --- |
| Test Food Group 1 (Group of Daily UPM Intake of 600 mg) | 13.0 | 16.7 | 0.035 |
| Test Food Group 2 (Group of Daily UMP Intake of 300 mg) | 9.5 | 18.3 | 0.186 |
| Placebo Group | 4.1 | 16.2 | - |

Both groups of Test Food Group 1 (group of daily UMP intake of 600 mg) and Test Food Group 2 (group of daily UMP intake of 300 mg) expanded the increased range of the composite memory standardized scores before and after the test, as compared to the placebo group.

In particular, in Test Food Group 1 (group of daily UMP intake of 600 mg), the increase in the composite memory standardized score before and after the test was 13.0, which exceeded the medically significant value of 10, and the p-value was in the range of $p < 0.05$, which was determined to be a significant difference.

The results of this test clearly show that UMP alone has the effect of improving the composite memory, and especially the group of a daily UMP intake of 600 mg shows a remarkable and significant composite memory improving effect.

(Example 2-2) Effect of Improving Executive Function Standardization Scores at All ages

The study was conducted to see if there could be found the improvement of the executive function, an index for an ability to understand rules and concepts and make decisions rapidly.

FIG. 2 and Table 3 show an amount of change in executive function standardized scores across subjects before and after the test.

[Table 3]

| Test Groups | Amount of Change in Executive Function Standardized Score | Standard Deviation | p-value |
|---|---|---|---|
| Test Food Group 1 (Group of Daily UPM Intake of 600 mg) | 7.8 | 15.0 | 0.913 |
| Test Food Group 2 (Group of Daily UMP Intake of 300 mg) | 10.6 | 23.1 | 0.282 |
| Placebo Group | 4.1 | 12.9 | - |

The analysis across the subjects showed a trend to increase the amount of change in executive function standardized scores for Test Food Group 1 (group of daily UMP intake of 600 mg) and Test Food Group 2 (group of daily UMP intake of 300 mg), as compared to the placebo group.

When comparing Test Food Group 1 with Test Food Group 2, it was clear that the amount of change was higher for Test Food Group 2 (group of daily UMP intake of 300 mg). In the group of a daily UMP intake of 300 mg, the amount of change in the executive function standardized scores was higher than 10.

The results of this example suggest that UMP ingestion may improve the executive function. Furthermore, the results suggest that the daily UMP intake of 300 mg may have a higher effect of improving the executive function than the daily UMP intake of 600 mg. In the group of a daily UMP intake of 300 mg, the executive function was markedly improved with medical significance.

(Example 2-3) Effect of Improving Cognitive Flexibility Standardized Scores at All Ages

The study was conducted to see if there was the effect of improving the cognitive flexibility, an index for the ability to flexibly change one's thinking in response to stimuli from the external environment.

FIG. 3 and Table 4 show the results of the amount of change in the cognitive flexibility standardized scores across all subjects before and after the test.

[Table 4]

| Test Groups | Amount of Change in Cognitive Flexibility Standardized Score | Standard Deviation | p-value |
|---|---|---|---|
| Test Food Group 1 (Group of Daily UPM Intake of 600 mg) | 7.7 | 15.7 | 0.798 |
| Test Food Group 2 (Group of Daily UMP Intake of 300 mg) | 11.3 | 24.0 | 0.145 |
| Placebo Group | 3.3 | 13.0 | - |

There could be found a trend to increase the amount of change in cognitive flexibility standardized scores for Test Food Group 1 (group of daily UMP intake of 600 mg) and Test Food Group 2 (group of daily UMP intake of 300 mg), as compared to the placebo group. When comparing the group of daily UMP intake of 300 mg to the group of daily UMP intake of 600 mg, it was clear that the amount of change was higher for the group of daily UMP intake of 300 mg.

In the group of daily UMP intake of 300 mg, the amount of change in the cognitive flexibility standardized score exceeded 10, which was considered to be medically significant.

The results of this example suggest that UMP ingestion may improve the cognitive flexibility. Furthermore, the results suggest that the daily UMP intake of 300 mg may have a higher effect of improving the cognitive flexibility than the daily UMP intake of 600 mg. In the group of daily UMP intake of 300 mg, the cognitive flexibility was significantly improved with medical significance.

(Example 2-4) Effect of Improving Working Memory Standardized Scores at All Ages

The study was conducted to see if there was the effect of improving the working memory, an index for the ability to temporarily maintain necessary information according to diversely changing purposes while performing other processing tasks in parallel.

FIG. 4 and Table 5 show the results of the amount of change in working memory standardized scores across all subjects before and after the test.

[Table 5]

| Test Groups | Amount of Change in Working Memory Standardized Score | Standard Deviation | p-value |
|---|---|---|---|
| Test Food Group 1 (Group of Daily UPM Intake of 600 mg) | 1.8 | 17.9 | 0.823 |
| Test Food Group 2 (Group of Daily UMP Intake of 300 mg) | 3.8 | 11.4 | 0.099 |
| Placebo Group | 2.8 | 16.0 | - |

In Test Food Group 1 (group of daily UMP intake of 600 mg), the increase in working memory standardized scores before and after the test was lower than that in the placebo group, and no improvement of working memory could be observed. On the other hand, in Test Food Group 2 (group of daily UMP intake of 300 mg), the increase in working memory standardized scores before and after the test was higher than that in the placebo group, and the improvement of working memory could be observed with a significant trend (p-value: 0.099).

This confirmed that UMP has a working memory improving effect, and that it has a particularly strong working memory improving effect when the daily intake is around 300 mg.

(Example 2-5) Effect of Improving Processing Speed Standardized Scores at All Ages

The study was conducted to see if there was the effect of improving the processing speed, an index for the ability to process information quickly.

FIG. 5 and Table 6 show the results of the amount of change in processing speed standardized scores across all subjects before and after the test.

[Table 6]

| Test Groups | Amount of Change in Processing Speed Standardized Score | Standard Deviation | p-value |
|---|---|---|---|
| Test Food Group 1 (Group of Daily UPM Intake of 600 mg) | 5.7 | 16.0 | 0.573 |
| Test Food Group 2 (Group of Daily UMP Intake of 300 mg) | 4.0 | 11.0 | 0.558 |
| Placebo Group | 4.4 | 13.8 | - |

In Test Food Group 1 (group of daily UMP intake of 600 mg), there was an increase in processing speed standardized scores before and after the test as compared to the placebo group.

This confirmed that the effect of improving the processing speed was achieved when the daily UMP intake was around 600 mg.

(Example 2-6) Effects of Improving Neurocognition Index (NCI) Standardized Scores at All Ages

FIG. 6 and Table 7 show the amount of change in NCI standardized scores for all subjects before and after the test for the NCI, which is an index for overall intellectual function that synthesizes a wide range of functional domains, including memory, cognitive speed, executive function, attention, and cognitive flexibility.

[Table 7]

| Test Groups | Amount of Change in NCI Standardized Score | Standard Deviation | p-value |
|---|---|---|---|
| Test Food Group 1 (Group of Daily UPM Intake of 600 mg) | 8.1 | 12.2 | 0.595 |
| Test Food Group 2 (Group of Daily UMP Intake of 300 mg) | 8.7 | 10.4 | 0.161 |
| Placebo Group | 5.7 | 13.5 | - |

In both test food groups of Test Food Group 1 (group of daily UMP intake of 600 mg) and Test Food Group 2 (group of daily UMP intake of 300 mg), an increasing trend was observed to increase the Neurocognition Index (NCI) standardized score from the test before ingestion when compared to the placebo group.

(Example 3) Effect of Improving Standardized Score in Each Cognitive Domain for Individuals under Age of 60

In order to verify whether the present invention would have any effect on those who were not affected by the normal cognitive decline associated with aging (physiological amnesia), we focused on subjects under the age of 60 and analysis was conducted.

(Example 3-1) Effect of Improving Composite Memory Standardized Scores in Subjects under Age of 60

Results were extracted for the composite memory standardized scores for subjects under the age of 60 in the test conducted by the method described in Example 1. The amount of change in composite memory standardized scores for subjects under the age of 60 before and after the test is shown in FIG. 7 and Table 8.

[Table 8]

| Test Groups | Amount of Change in Composite Memory Standardized Score | Standard Deviation | p-value |
|---|---|---|---|
| Test Food Group 1 (Group of Daily UPM Intake of 600 mg) | 13.1 | 16.5 | 0.051 |
| Test Food Group 2 (Group of Daily UMP Intake of 300 mg) | 9.3 | 17.6 | 0.229 |
| Placebo Group | 4.6 | 16.6 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.
In particular, for Test Food Group 1 (group of daily UMP intake of 600 mg), the increase in the composite memory standardized score before and after the test was 13.1, which exceeded the medically significant value of 10, and the p-value was in the range of $p < 0.10$, which was considered to indicate a significant trend.
The results of this example clearly show that UMP improves the composite memory even in subjects under the age of 60, and especially the group of daily UMP intake of 600 mg showed a remarkable and significant composite memory improving effect.

(Example 3-2) Effects of Improving Executive Function Standardization Scores for Subjects under Age of 60

Results were extracted for the executive function standardized scores for subjects under the age of 60 in the test conducted by the method described in Example 1. The amount of change in executive function standardized scores for subjects under the age of 60 before and after the test is shown in FIG. 8 and Table 9.

[Table 9]

| Test Groups | Amount of Change in Executive Function Standardized Score | Standard Deviation | p-value |
|---|---|---|---|
| Test Food Group 1 (Group of Daily UPM Intake of 600 mg) | 9.8 | 15.0 | 0.519 |
| Test Food Group 2 (Group of Daily UMP Intake of 300 mg) | 11.7 | 24.7 | 0.155 |
| Placebo Group | 4.7 | 14.0 | - |

In the subgroup under the age of 60, there was also a trend to increase the amount of change in executive function standardized scores for Test Food Group 1 (group of daily UMP intake of 600 mg) and Test Food Group 2 (group of daily UMP intake of 300 mg) as compared to the placebo group.

When comparing Test Food Group 1 with Test Food Group 2, it was clear that the amount of change was higher for Test Food Group 2 (group of daily UMP intake of 300 mg). In the group of daily UMP intake of 300 mg, the increase in the executive function standardized score was 11.7, which exceeded the medically significant value of 10.

When comparing the results of Example 2-2 with those of this example, it was revealed that the increase in the executive function standardized score in the subgroup of subjects under the age of 60 in this example was higher than the increase in the executive function standardized score of all subjects in Example 2-2.

This indicates that the UMP produces a stronger executive function improving effect for subjects under the age of 60.

(Example 3-3) Effect of Improving Cognitive Flexibility Standardized Scores in Subjects under Age of 60

Results were extracted for the cognitive flexibility standardized scores for subjects under the age of 60 in the test conducted by the method described in Example 1. The amount of change in cognitive flexibility standardized scores for subjects under the age of 60 before and after the test is shown in FIG. 9 and Table 10.

[Table 10]

| Test Groups | Amount of Change in Cognitive Flexibility Standardized Score | Standard Deviation | p-value |
|---|---|---|---|
| Test Food Group 1 (Group of Daily UPM Intake of 600 mg) | 9.7 | 15.8 | 0.424 |
| Test Food Group 2 (Group of Daily UMP Intake of 300 mg) | 12.7 | 25.5 | 0.063 |
| Placebo Group | 3.8 | 14.2 | - |

The analysis of the subjects under the age of 60 showed a trend to increase the amount of change in cognitive flexibility standardized scores for Test Food Group 1 (group of daily UMP intake of 600 mg) and Test Food Group 2 (group of daily UMP intake of 300 mg), as compared to the placebo group.

Particularly, in the group of daily UMP intake of 300 mg of the subjects under the age of 60, the amount of the increase in the cognitive flexibility standardized score was 12.7, which exceeded the medically significant value of 10, with a p-value in the range of $p < 0.10$, which was considered to indicate a significant trend.

Furthermore, when comparing the results of Examples 2-3 with those of this example, the p-values were significantly lower and the amount of change in the scores was increased for both Test Food Groups 1 and 2.

This indicated that the UMP produced a higher effect of improving the cognitive flexibility for subjects under the age of 60.

(Example 3-4) Effect of Improving Complex Attention Standardized Score in Subjects under Age of 60

Results were extracted for the complex attention standardized scores for the subjects under the age of 60 in the test conducted by the method described in Example 1. The amount of change in the complex attention standardized scores for the subjects under the age of 60 before and after the test is shown in FIG. 10 and Table 11.

[Table 11]

| Test Groups | Amount of Change in Complex Attention Standardized Score | Standard Deviation | p-value |
|---|---|---|---|
| Test Food Group 1 (Group of Daily UPM Intake of 600 mg) | 3.8 | 37.6 | 0.929 |
| Test Food Group 2 (Group of Daily UMP Intake of 300 mg) | 13.6 | 28.8 | 0.155 |
| Placebo Group | 2.2 | 24.6 | - |

For the subjects under the age of 60, both Test Food Groups 1 and 2 showed an increased amount of change in the complex attention standardized scores as compared to the placebo group.

Among them, there was a remarkable trend to improve the complex attention in Test Food Group 2 (group of daily UMP intake of 300 mg).

This indicated that the UMP produced the effect of improving the complex attention for the subjects under the age of 60.

(Example 3-5) Effects of Improving Working Memory Standardized Scores in Subjects Under Age of 60

Results were extracted for working memory standardized scores for subjects under the age of 60 in the test conducted by the method described in Example 1. The amount of change in working memory standardized scores for subjects under the age of 60 before and after the test is shown in FIG. 11 and Table 12.

[Table 12]

| Test Groups | Amount of Change in Working Memory Standardized Score | Standard Deviation | p-value |
|---|---|---|---|
| Test Food Group 1 (Group of Daily UPM Intake of 600 mg) | 3.3 | 18.3 | 0.970 |
| Test Food Group 2 (Group of Daily UMP Intake of 300 mg) | 4.8 | 10.1 | 0.101 |
| Placebo Group | 4.4 | 16.4 | - |

For the subjects under the age of 60, the amount of change in working memory standardized scores was increased in the Test Food Group 2 as compared to the placebo group.

Furthermore, when comparing the results of Examples 2-4 with the results of this example, the amount of change in the working memory standardized scores was increased in this example.

This confirms that UMP has a higher working memory improving effect on subjects under the age of 60, and that it has a particularly strong working memory improving effect when the daily intake is around 300 mg.

(Example 3-6) Effect of Improving Processing Speed Standardized Scores for Subjects Under Age of 60

Results were extracted for processing speed standardized scores for subjects under the age of 60 in the test conducted by the method described in Example 1. The amount of change in processing speed standardized scores for subjects under the age of 60 before and after the test is shown in FIG. 12 and Table 13.

[Table 13]

| Test Groups | Amount of Change in Processing Speed Standardized Score | Standard Deviation | p-value |
|---|---|---|---|
| Test Food Group 1 (Group of Daily UPM Intake of 600 mg) | 7.4 | 16.4 | 0.354 |
| Test Food Group 2 (Group of Daily UMP Intake of 300 mg) | 5.9 | 10.5 | 0.255 |
| Placebo Group | 4.6 | 14.7 | - |

For the subjects under the age of 60, both Test Food Groups 1 and 2 showed the increased amount of change in processing speed standardized scores as compared to the placebo group.

In particular, there was a remarkable trend to improve the processing speed in Test Food Group 1 (group of daily UMP intake of 600 mg).

This indicates that the UMP produces the effect of improving the processing speed for the subjects under the age of 60.

(Example 3-7) Effect of Improving Neurocognition Index (NCI) Standardized Scores for Subjects Under Age of 60

Results were extracted for the Neurocognition Index (NCI) standardized scores for subjects under the age of 60 in the test conducted by the method described in Example 1. The amount of change in the Neurocognition Index (NCI) standardized scores for the subjects under the age of 60 before and after the test is shown in FIG. 13 and Table 14.

[Table 14]

| Test Groups | Amount of Change in NCI Standardized Score | Standard Deviation | p-value |
|---|---|---|---|
| Test Food Group 1 (Group of Daily UPM Intake of 600 mg) | 9.1 | 12.6 | 0.166 |
| Test Food Group 2 (Group of Daily UMP Intake of 300 mg) | 9.7 | 10.6 | 0.033 |
| Placebo Group | 4.2 | 8.5 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.

In particular, a remarkable and significant (p-value: 0.033) effect of improving the neurocognition index (NCI) standardized score was observed in the group of daily UMP intake of 300 mg.

It is revealed that the agent for improving composite memory according to the present invention produces remarkable effects on the subjects under the age of 60, specifically subjects in their 40s and 50s.

In particular, it was found that it produced a remarkable and significant effect on healthy subjects under the age of 60 when the daily intake of UMP was 300 mg.

(Example 4) Effect of Improving Standardized Scores in Each Cognitive Domain for Subjects Having Composite Memory Standardized Score of Less Than 90

In order to verify what effects the present invention will produce on subjects having a relatively low cognitive function relating to memory, we conducted an analysis focusing on subjects whose composite memory standardized score before UMP ingestion was less than 90.

(Example 4-1) Effect of Improving Composite Memory Standardized Scores in Subject Having Composite Memory Standardized Score of Less Than 90

Results were extracted for the composite memory standardized scores for subjects who had a composite memory standardized score of less than 90 prior to UMP ingestion in the test conducted by the method described in Example 1. The amount of change in the composite memory standardized scores before and after the test is shown in FIG. 14 and Table 15.

[Table 15]

| Test Groups | Amount of Change in Composite Memory Standardized Score | Standard Deviation | p-value |
|---|---|---|---|
| Test Food Group 1 (Group of Daily UPM Intake of 600 mg) | 17.0 | 16.9 | 0.071 |
| Test Food Group 2 (Group of Daily UMP Intake of 300 mg) | 12.9 | 18.1 | 0.262 |
| Placebo Group | 7.3 | 17.7 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

In both Test Food Group 1 (group of daily UMP intake of 600 mg) and Test Food Group 2 (group of daily UMP intake of 300 mg), the increase in the composite memory standardized score exceeded 10, which was considered to be medically significant. In particular, for Test Food Group 1 (group of daily UMP intake of 600 mg), the increase in the composite memory standardized score was 17.0, which significantly exceeded the medically significant value of 10, and the p-value was in the range of $p < 0.10$, which was determined to indicate a significant trend.

When compared with the results of Example 2-1, the increase in the composite memory standardized score was higher for both Test Food Groups 1 and 2.

The results of this example clearly show that UMP produces a stronger composite memory improving effect on the subjects who have a composite memory standardized score of less than 90, and in particular, the group of daily UMP intake of 600 mg shows a remarkable and significant composite memory improving effect.

(Example 4-2) Effect of Improving Executive Function Standardized Scores in Subject Having Composite Memory Standardized Scores of Less Than 90

Results were extracted for the executive function standardized scores for subjects who had a composite memory standardized score of less than 90 in the test conducted by the method described in Example 1. The amount of change in the executive function standardized scores before and after the test is shown in FIG. 15 and Table 16.

[Table 16]

| Test Groups | Amount of Change in Executive Function Standardized Score | Standard Deviation | p-value |
|---|---|---|---|
| Test Food Group 1 (Group of Daily UPM Intake of 600 mg) | 8.2 | 14.2 | 0.320 |
| Test Food Group 2 (Group of Daily UMP Intake of 300 mg) | 16.0 | 27.5 | 0.065 |
| Placebo Group | 2.4 | 11.8 | - |

Even in the subgroup with the composite memory standardized score of less than 90, there was a trend to increase the amount of change in executive function standardized scores in Test Food Group 1 (group of daily UMP intake of 600 mg) and Test Food Group 2 (group of daily UMP intake of 300 mg) as compared to the placebo group.

When comparing Test Food Group 1 with Test Food Group 2, it was clear that the amount of change was higher for Test Food Group 2 (group of daily UMP intake of 300 mg).

Particularly, in the group of daily UMP intake of 300 mg of the subjects having the composite memory standardized score of less than 90, the increase in the executive function standardized score was 16.0, which significantly exceeded the medically significant value of 10, and the p-value was in the range of $0.05 \leq p < 0.10$, which was considered to indicate a significant trend.

When comparing the results of Example 2-2 with those of this example, it is revealed that the increase in the executive function standardized scores for the subgroup of subjects having the composite memory standardized score of less than 90 in this example is higher than the increase in the executive function standardized scores for all subjects in Example 2-2.

This indicates that the UMP produces a higher effect of improving the executive function for the subjects having the composite memory standardized score of less than 90.

(Example 4-3) Effect of Improving Cognitive Flexibility Standardized Scores in Subjects Having Composite Memory Standardized Score of Less Than 90

Results were extracted for the cognitive flexibility standardized scores for subjects who had a composite memory standardized score of less than 90 in the test conducted by the method described in Example 1. The amount of change in cognitive flexibility standardized scores before and after the test is shown in FIG. 16 and Table 17.

[Table 17]

| Test Groups | Amount of Change in Cognitive Flexibility Standardized Score | Standard Deviation | p-value |
|---|---|---|---|
| Test Food Group 1 (Group of Daily UMP Intake of 600 mg) | 8.1 | 15.3 | 0.244 |
| Test Food Group 2 (Group of Daily UMP Intake of 300 mg) | 17.5 | 28.2 | 0.027 |
| Placebo Group | 1.2 | 12.1 | - |

Even in the subgroup with the composite memory standardized score of less than 90, there was a trend to increase the amount of change in cognitive flexibility standardized scores in Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects (all ages) having a composite memory standardized score of less than 90, the increase in the cognitive flexibility standardized score was 17.5, which significantly exceeded 10, which was considered to have medical significance, and the p-value was in the $p < 0.05$ range, which was determined to be a significant difference.

Furthermore, when comparing the results of Examples 2-3 with those of this example, the p-values were significantly lower and the amount of change in the scores was increased for both Test Food Groups 1 and 2.

This indicates that the UMP produces a higher effect of improving the cognitive flexibility for subjects having the composite memory standardized score of less than 90.

(Example 4-4) Effect of Improving Complex Attention Standardized Score for Subjects Having Composite Memory Standardized Score of Less Than 90

Results were extracted for the complex attention standardized scores for subjects who had the composite memory standardized score of less than 90 in the test conducted by the method described in Example 1. The amount of change in the complex attention standardized scores before and after the test is shown in FIG. 17 and Table 18.

[Table 18]

| Test Groups | Amount of Change in Complex Attention Standardized Score | Standard Deviation | p-value |
|---|---|---|---|
| Test Food Group 1 (Group of Daily UPM Intake of 600 mg) | 0.6 | 41.9 | 0.621 |
| Test Food Group 2 (Group of Daily UMP Intake of 300 mg) | 19.4 | 31.8 | 0.531 |
| Placebo Group | 14.9 | 70.3 | - |

In the subgroup with the composite memory standardized score of less than 90, there was also a trend to increase the amount of change in complex attention standardized scores in Test Food Group 2 (group of daily UMP intake of 300 mg) as compared to the placebo group.

This indicates that the UMP produces the effect of improving the complex attention for subjects having the composite memory standardized score of less than 90.

(Example 4-5) Effect of Improving Working Memory Standardized Scores for Subjects Having Composite Memory Standardized Score of Less Than 90

Results were extracted for the working memory standardized scores for subjects who had a composite memory standardized score of less than 90 in the test conducted by the method described in Example 1. The amount of change in working memory standardized scores before and after the test is shown in FIG. 18 and Table 19.

[Table 19]

| Test Groups | Amount of Change in Working Memory Standardized Score | Standard Deviation | p-value |
|---|---|---|---|
| Test Food Group 1 (Group of Daily UPM Intake of 600 mg) | 3.4 | 18.6 | 0.778 |
| Test Food Group 2 (Group of Daily UMP Intake of 300 mg) | 5.1 | 10.8 | 0.205 |
| Placebo Group | 4.9 | 16.6 | - |

In the subgroup with the composite memory standardized score of less than 90, there was also a trend to increase the amount of change in working memory standardized scores in Test Food Group 2 (group of daily UMP intake of 300 mg) as compared to the placebo group.

This indicates that the UMP produces the effect of improving the working memory for subjects having the composite memory standardized score of less than 90.

(Example 4-6) Effect of Improving Neurocognition Index (NCI) Standardized Score for Subjects Having Composite Memory Standardized Score of Less Than 90

Results were extracted for neurocognition index (NCI) standardized scores for subjects having the composite memory standardized score of less than 90 in the test conducted by the method described in Example 1. The amount of change in the neurocognition index (NCI) standardized scores before and after the test is shown in FIG. 19 and Table 20.

[Table 20]

| Test Groups | Amount of Change in NCI Standardized Score | Standard Deviation | p-value |
|---|---|---|---|
| Test Food Group 1 (Group of Daily UPM Intake of 600 mg) | 8.0 | 11.8 | 0.591 |
| Test Food Group 2 (Group of Daily UMP Intake of 300 mg) | 11.3 | 11.7 | 0.172 |
| Placebo Group | 6.6 | 16.1 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.

In particular, for the group of daily UMP intake of 300 mg, the amount of change in the neurocognition index (NCI) standardized score exceeded 10, which was considered a significant improvement with medical significance.

This indicates that the UMP produces the effect of significantly improving the neurocognitive function for healthy individuals having the composite memory standardized score of less than 90.

In particular, it was revealed that when the daily UMP intake was 300 mg, a remarkable effect was achieved for the healthy individuals having the composite memory standardized score of less than 90, and the neurocognitive function was significantly improved with medical significance.

(Example 5) Effect of Improving Standardized Scores in Each Cognitive Domain for Subjects Having Composite Memory Standardized Score of Less Than 83.5

In order to verify what effects the present invention produces on subjects having a considerably low cognitive function relating to memory, we conducted an analysis focusing on subjects having a composite memory standardized score of less than 83.5 before UMP ingestion.

(Example 5-1) Effect of Improving Composite Memory Standardized Scores in Subject Having Composite Memory Standardized Score of Less Than 83.5

Results were extracted for the composite memory standardized scores for subjects who had a composite memory standardized score of less than 83.5 prior to UMP ingestion in the test conducted by the method described in Example 1. The amount of change in the composite memory standardized scores before and after the test is shown in FIG.

20 and Table 21.

[Table 21]

| Test Groups | Amount of Change in Composite Memory Standardized Score | Standard Deviation | p-value |
|---|---|---|---|
| Test Food Group 1 (Group of Daily UPM Intake of 600 mg) | 20.3 | 19.2 | 0.033 |
| Test Food Group 2 (Group of Daily UMP Intake of 300 mg) | 18.2 | 17.3 | 0.043 |
| Placebo Group | 4.3 | 19.6 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

In both Test Food Group 1 (group of daily UMP intake of 600 mg) and Test Food Group 2 (group of daily UMP intake of 300 mg), the increase in the composite memory standardized score before and after the test significantly exceeded 10, which was considered to have medical significance. The p-values for both groups were also in the range of $p < 0.05$, which was determined to be a significant difference.

When compared to the results of Examples 2-1 and 3-1, the increase in the composite memory standardized score was higher for both Test Food Groups 1 and 2.

The results of this example show that UMP produce a remarkable and significant effect of improving the composite memory for subjects who are under the age of 60 and have a composite memory standardized score of less than 83.5.

(Example 5-2) Effect of Improving Executive Function Standardized Scores in Subject Having Composite Memory Standardized Scores of Less Than 83.5

Results were extracted for the executive function standardized scores for subjects who had a composite memory standardized score of less than 83.5 prior to UMP ingestion in the test conducted by the method described in Example 1. The amount of change in the executive function standardized scores before and after the test is shown in FIG. 21 and Table 22.

[Table 22]

| Test Groups | Amount of Change in Executive Function Standardized Score | Standard Deviation | p-value |
|---|---|---|---|
| Test Food Group 1 (Group of Daily UPM Intake of 600 mg) | 2.3 | 8.5 | 0.596 |
| Test Food Group 2 (Group of Daily UMP Intake of 300 mg) | 21.2 | 31.6 | 0.086 |
| Placebo Group | 2.1 | 13.7 | - |

In Test Food Group 2 (group of daily UMP intake of 300 mg), the increase in the composite memory standardized score before and after the test was 21.2, which significantly exceeded the medically significant value of 10. The p-values were in the range of $p < 0.10$, which was determined to have a significant trend.

In terms of Test Food Group 2 (the group of daily UMP intake of 300 mg), the increase in executive function standardized scores was higher than the results of Examples 2-2 and 4-2.

The results of this example show that UMP has a markable and significant effect of improving the executive function for subjects having a composite memory standardized score of less than 83.5, when the daily UMP intake is around 300 mg. The effects were higher than those for the subjects as a whole and for subjects having the composite memory standardized score of less than 90, indicating that a higher effect of improving the executive function is exhibited for subjects having a lower cognitive function relating to memory.

(Example 5-3) Effect of Improving Cognitive Flexibility Standardized Scores for Subjects Having Composite Memory Standardized Score of Less Than 83.5

Results were extracted for the cognitive flexibility standardized scores for subjects who had a composite memory standardized score of less than 83.5 before UMP ingestion in the test conducted by the method described in Example 1. The amount of change in cognitive flexibility standardized scores before and after the test is shown in FIG. 22 and Table 23.

[Table 23]

| Test Groups | Amount of Change in Cognitive Flexibility Standardized Score | Standard Deviation | p-value |
|---|---|---|---|
| Test Food Group 1 (Group of Daily UPM Intake of 600 mg) | 1.9 | 8.8 | 0.542 |
| Test Food Group 2 (Group of Daily UMP Intake of 300 mg) | 23.3 | 32.2 | 0.044 |
| Placebo Group | 0.8 | 13.9 | - |

In both Test Food Group 1 (group of daily UMP intake of 600 mg) and Test Food Group 2 (group of daily UMP intake of 300 mg), the increase in composite memory standardized scores before and after the test was higher than the placebo group.
In particular, for test food group 2 (group of daily UMP intake of 300 mg), the increase in composite memory standardized scores before and after the test was 23.3, which significantly exceeded the medically significant value of 10. The p-value was in the range of $p < 0.05$, which was determined to be a significant difference.
In terms of Test Food Group 2 (group of daily UMP intake of 300 mg), the increase in executive function standardized scores was higher than the results of Examples 2-3 and 4-3.
The results of this example show that UMP has a markable and significant effect of improving the cognitive flexibility for subjects having a composite memory standardized score of less than 83.5, when the daily UMP intake is around 300 mg. The effect was higher than that for the subjects as a whole and for those who had the composite memory standardized score of less than 90, indicating that a higher effect of improving the cognitive flexibility is exhibited for subjects having a lower cognitive function relating to memory.

(Example 5-4) Effect of Improving Complex Attention Standardized Scores for Subjects Having Composite Memory Standardized Score of Less Than 83.5.

Results were extracted for the complex attention standardized score for subjects having a composite memory standardized score of less than 83.5 before UMP ingestion in the test conducted by the method described in Example 1. The amount of change in the complex attention standardized scores before and after the test is shown in FIG. 23 and Table 24.

[Table 24]

| Test Groups | Amount of Change in Complex Attention Standardized Score | Standard Deviation | p-value |
|---|---|---|---|
| Test Food Group 1 (Group of Daily UPM Intake of 600 mg) | 0.7 | 13.0 | 0.350 |
| Test Food Group 2 (Group of Daily UMP Intake of 300 mg) | 27.8 | 35.0 | 0.058 |
| Placebo Group | -0.8 | 18.4 | - |

In both Test Food Group 1 (group of daily UMP intake of 600 mg) and Test Food Group 2 (group of daily UMP intake of 300 mg), the increase in composite memory standardized scores before and after the test was higher than the placebo group.
In particular, for test food group 2 (group of daily UMP intake of 300 mg), the increase in composite memory standardized scores before and after the test was 27.8, which significantly exceeded the medically significant value of 10. The p-values were in the range of $p < 0.10$, which was determined to have a significant trend.
In terms of Test Food Group 2 (the group of daily UMP intake of 300 mg), the increase in executive function standardized scores was higher than the results of Example 4-4.

The results of this example show that UMP has a markable and significant effect of improving the complex attention for subjects having the composite memory standardized score of less than 83.5, when the daily UMP intake is around 300 mg. The effect was higher than that for the subjects as a whole and for those having the composite memory standardized score of less than 90, indicating that a higher effect of improving the complex attention is exhibited for subjects having a lower cognitive function relating to memory.

(Example 5-5) Effect of Improving Neurocognition Index (NCI) Standardized Score for Subjects Having Composite Memory Standardized Score of Less Than 83.5

Results were extracted for the neurocognition index (NCI) standardized scores for subjects who had a composite memory standardized score of less than 83.5 before UMP ingestion in the test conducted by the method described in Example 1. The amount of change in the neurocognition index (NCI) standardized scores before and after the test is shown in FIG. 24 and Table 25.

[Table 25]

| Test Groups | Amount of Change in NCI Standardized Score | Standard Deviation | p-value |
|---|---|---|---|
| Test Food Group 1 (Group of Daily UPM Intake of 600 mg) | 6.3 | 5.1 | 0.129 |
| Test Food Group 2 (Group of Daily UMP Intake of 300 mg) | 15.1 | 11.8 | 0.001 |
| Placebo Group | 2.5 | 8.7 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.
Particularly, in the group of daily UMP intake of 300 mg, the increase in the neurocognition index (NCI) standardized score was 15.1, which significantly exceeded the medically significant of 10, and the p-value was in the range of $p < 0.05$, which was determined to be a significant difference.
Furthermore, in the group of daily UMP intake of 300 mg, the amount of change in the neurocognition index (NCI) standardized score was increased, as compared to Example 2-6 which was tested for all subjects, and Example 4-6 which was tested for subjects having the composite memory standardized score of less than 90.
This indicates that UMP had a pronounced and significant effect on subjects having a composite memory standardized score of less than 83.5, and that the neurocognitive function was significantly improved with medical significance for subjects who ingested UMP.

(Example 6) Effect of Improving Standardized Scores in Each Cognitive Domain for Subjects Under Age of 60 and Having Composite Memory Standardized Score of Less Than 90

In order to verify what effect the present invention produces on subjects with both attributes, in relation to the effect on subjects under the age of 60 tested in Example 3 and the effect on subjects having the relatively low cognitive function relating to memory tested in Example 4, we focused on subjects who were under the age of 60 and had a composite memory standardized score of less than 90 before UMP ingestion, and analysis was conducted.

(Example 6-1) Effect of Improving Composite Memory Standardized Scores for Subjects Under Age of 60 and Having Composite Memory Standardized Score of Less Than 90.

Results were extracted for the composite memory standardized scores for subjects who were under the age of 60 and had a composite memory standardized score of less than 90 prior to UMP ingestion in the test conducted by the method described in Example 1. The amount of change in the composite memory standardized scores before and after the test is shown in FIG. 25 and Table 26.

[Table 26]

| Test Groups | Amount of Change in Composite Memory Standardized Score | Standard Deviation | p-value |
|---|---|---|---|
| Test Food Group 1 (Group of Daily UPM Intake of 600 mg) | 16.1 | 16.8 | 0.168 |
| Test Food Group 2 (Group of Daily UMP Intake of 300 mg) | 11.2 | 17.1 | 0.502 |
| Placebo Group | 8.6 | 18.0 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

In both Test Food Group 1 (group of daily UMP intake of 600 mg) and Test Food Group 2 (group of daily UMP intake of 300 mg), the increase in the composite memory standardized score exceeded 10, which was considered to be medically significant. In particular, for Test Food Group 1 (group of daily UMP intake of 600 mg), the increase in the composite memory standardized score was 16.1, which significantly exceeded the medically significant of 10.

When compared with the results of Example 2-1, the increase in the composite memory standardized score was higher for both Test Food Groups 1 and 2.

The results of this example showed that UMP had a higher effect of improving the composite memory for subjects who were under the age of 60 and had a composite memory standardized score of less than 90, especially in the group of daily UMP intake of 600 mg.

(Example 6-2) Effect of Improving Executive Function Standardized Scores for Subjects Under Age of 60 and Having Composite Memory Standardized Score of Less Than 90

Results were extracted for the executive function standardized scores for subjects who were under the age of 60 and had a composite memory standardized score of less than 90 prior to UMP ingestion in the conducted by the method described in Example 1. The amount of change in the executive function standardized scores before and after the test is shown in FIG. 26 and Table 27.

[Table 27]

| Test Groups | Amount of Change in Executive Function Standardized Score | Standard Deviation | p-value |
|---|---|---|---|
| Test Food Group 1 (Group of Daily UPM Intake of 600 mg) | 9.2 | 14.6 | 0.232 |
| Test Food Group 2 (Group of Daily UMP Intake of 300 mg) | 17.4 | 28.8 | 0.049 |
| Placebo Group | 2.8 | 12.4 | - |

In the subgroups having the composite memory standardized score of less than 90 and under the age of 60, there was also a trend to increase the amount of change in executive function standardized scores for Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects having the composite memory standardized score of less than 90 and under the age of 60, the increase in the executive function standardized score was 17.4, which significantly exceeded the medically significant value of 10, and the p-value was in the $p < 0.05$ range, which was considered to be a significant difference.

This indicates that the UMP produces a remarkable and significant effect of improving the executive function for subjects having the composite memory standardized score of less than 90 and under the age of 60 (40s and 50s).

When comparing the results of Examples 2-2, 3-2, and 4-2 with those of this example, it was found that in Test Food Group 2 (the group of daily UMP intake of 300 mg), the increase in the executive function standardized score in this example was higher than the increase in the executive function standardized score in Examples 2-2, 3-2, and 4-2. For the p-value, this example resulted in the smallest p-value.

This indicates that the UMP produces a higher effect of improving the executive function for subjects having the composite memory standardized score of less than 90 and under the age of 60.

(Example 6-3) Effect of Improving Cognitive Flexibility Standardized Scores for Subjects Under Age of 60 and Having Composite Memory Standardized Score of Less Than 90

Results were extracted for the cognitive flexibility standardized scores for subjects who were under the age of 60 and had a composite memory standardized score of less than 90 prior to UMP ingestion in the test conducted by the method described in Example 1. The amount of change in cognitive flexibility standardized scores before and after the test is shown in FIG. 27 and Table 28.

[Table 28]

| Test Groups | Amount of Change in Cognitive Flexibility Standardized Score | Standard Deviation | p-value |
|---|---|---|---|
| Test Food Group 1 (Group of Daily UPM Intake of 600 mg) | 9.1 | 15.8 | 0.173 |
| Test Food Group 2 (Group of Daily UMP Intake of 300 mg) | 18.9 | 29.5 | 0.020 |
| Placebo Group | 1.4 | 12.8 | - |

In the subgroups having the composite memory standardized score of less than 90 and under the age of 60, there was also a trend to increase the amount of change in cognitive flexibility standardized scores for Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects having the composite memory standardized score of less than 90 and under the age of 60, the increase in the cognitive flexibility standardized score was 18.9, which significantly exceeded the medically significant value of 10, and the p-value was in the $p < 0.05$ range, which was considered to be a significant difference.

This indicates that the UMP produces a remarkable and significant effect of improving the cognitive flexibility for subjects having a composite memory standardized score of less than 90 and under the age of 60.

(Example 6-4) Effect of Improving Complex Attention Standardized Scores for Subjects Under Age of 60 and Having Composite Memory Standardized Score of Less Than 90.

Results were extracted for the complex attention standardized score for subjects who were under the age of 60 and had a composite memory standardized score of less than 90 prior to UMP ingestion in the test conducted by the method described in Example 1. The amount of change in the complex attention standardized scores before and after the test is shown in FIG. 28 and Table 29.

[Table 29]

| Test Groups | Amount of Change in Complex Attention Standardized Score | Standard Deviation | p-value |
|---|---|---|---|
| Test Food Group 1 (Group of Daily UPM Intake of 600 mg) | 1.0 | 44.4 | 0.924 |
| Test Food Group 2 (Group of Daily UMP Intake of 300 mg) | 20.9 | 33.2 | 0.184 |
| Placebo Group | -0.9 | 17.3 | - |

In the subgroups having the composite memory standardized score of less than 90 and under the age of 60, there was also a trend to increase the amount of change in complex attention standardized scores for Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects who had a composite memory standardized score of less than 90 and were under the age of 60, the increase in complex attention standardized score was 20.9, which significantly exceeded the medically significant of 10.

This indicates that the UMP produces a remarkable and significant effect of improving the complex attention for subjects having the composite memory standardized score of less than 90 and under the age of 60.

(Example 6-5) Effect of Improving Processing Speed Standardized Scores for Subjects Under Age of 60 and Having Composite Memory Standardized Score of Less Than 90

Results were extracted for the processing speed standardized scores for subjects who were under the age of 60 and had a composite memory standardized score of less than 90 prior to UMP ingestion in the test conducted by the method described in Example 1. The amount of change in processing speed standardized scores before and after the test is shown in FIG. 29 and Table 30.

[Table 30]

| Test Groups | Amount of Change in Processing Speed Standardized Score | Standard Deviation | p-value |
|---|---|---|---|
| Test Food Group 1 (Group of Daily UPM Intake of 600 mg) | 9.1 | 17.7 | 0.482 |
| Test Food Group 2 (Group of Daily UMP Intake of 300 mg) | 3.8 | 10.2 | 0.955 |
| Placebo Group | 6.8 | 9.5 | - |

In the subgroup with the composite memory standardized score of less than 90 and under the age of 60, there was also a trend to increase the amount of change in processing speed standardized scores in Test Food Group 1 (group of daily UMP intake of 600 mg) as compared to the placebo group.
When comparing the results of Examples 2-5 with the results of this example, the increase in processing speed standardized scores was increased.
This indicates that UMP produces the effect of improving the processing speed for subjects having a composite memory standardized score of less than 90 and under the age of 60.

(Example 6-6) Effect of Improving Neurocognition Index (NCI) Standardized Score for Persons Under Age of 60 and Having Composite Memory Standardized Score of Less Than 90

Results were extracted for the neurocognition index (NCI) standardized scores for subjects who were under the age of 60 and had a composite memory standardized score of less than 90 prior to UMP intake in the test conducted by the method described in Example 1. The amount of change in the neurocognition index (NCI) standardized scores before and after the test is shown in FIG. 30 and Table 31.

[Table 31]

| Test Groups | Amount of Change in NCI Standardized Score | Standard Deviation | p-value |
|---|---|---|---|
| Test Food Group 1 (Group of Daily UPM Intake of 600 mg) | 8.5 | 12.3 | 0.230 |
| Test Food Group 2 (Group of Daily UMP Intake of 300 mg) | 11.8 | 12.2 | 0.045 |
| Placebo Group | 3.8 | 7.9 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.
Particularly, in the group of daily UMP intake of 300 mg of the subjects who were under the age of 60 and had the composite memory standardized score of less than 90, the increase in the neurocognition index (NCI) standardized score was 11.8, which exceeded the medically significant value of 10, and the increase in the neurocognition index (NCI) standardized score was higher than Examples 2-6, 3-7, and 4-6. The p-value was in the range of $p < 0.05$, which was determined to be a significant difference.
It was found from the results that the agent for improving composite memory according to the present application had a remarkable effect on those who were under the age of 60 and had a composite memory standardized score of less than 90 before UMP ingestion, and that neurocognitive functions were significantly improved with medical significance.
Among them, when the daily UMP intake was around 300 mg, it was found to have a remarkable and significant effect on healthy subjects who are under the age of 60 and have a composite memory standardized score of less than 90, and neurocognitive functions were significantly improved with medical significance.

(Example 7) Effect of Improving Standardized Scores in Each Cognitive Domain for Subjects Under Age of 60 and Having Composite Memory Standardized Score of Less Than 83.5

In order to verify what effect the present invention produces on subjects with both attributes, in relation to the effect on subjects under the age of 60 tested in Example 3 and the effect on subjects having a considerably low cognitive function relating to memory tested in Example 5, we focused on subjects who were under the age of 60 and had a composite memory standardized score of less than 83.5 before UMP ingestion, and analysis was conducted.

(Example 7-1) Effect of Improving Composite Memory Standardized Scores for Subjects Under Age of 60 and Having Composite Memory Standardized Score of Less Than 83.5.

Results were extracted for the composite memory standardized scores for subjects who were under the age of 60 and had a composite memory standardized score of less than 83.5 prior to UMP ingestion in the test conducted by the method described in Example 1. The amount of change in the composite memory standardized scores before and after the test is shown in FIG. 31 and Table 32.

[Table 32]

| Test Groups | Amount of Change in Composite Memory Standardized Score | Standard Deviation | p-value |
|---|---|---|---|
| Test Food Group 1 (Group of Daily UPM Intake of 600 mg) | 19.5 | 19.9 | 0.120 |
| Test Food Group 2 (Group of Daily UMP Intake of 300 mg) | 15.8 | 15.7 | 0.103 |
| Placebo Group | 5.8 | 19.6 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.
In both Test Food Group 1 (group of daily UMP intake of 600 mg) and Test Food Group 2 (group of daily UMP intake of 300 mg), the increase in the composite memory standardized score before and after the test significantly exceeded 10, which was considered to have medical significance.
The results of this example showed that UMP had a higher effect of improving the composite memory for subjects who were under the age of 60 and had a composite memory standardized score of less than 83.5, especially in the group of daily UMP intake of 600 mg.

(Example 7-2) Effect of Improving Executive Function Standardized Scores for Subjects Under Age of 60 and Having Composite Memory Standardized Score of Less Than 83.5

Results were extracted for the executive function standardized scores for subjects who were under the age of 60 and had a composite memory standardized score of less than 83.5 prior to UMP ingestion in the conducted by the method described in Example 1. The amount of change in the executive function standardized scores before and after the test is shown in FIG. 32 and Table 33.

[Table 33]

| Test Groups | Amount of Change in Executive Function Standardized Score | Standard Deviation | p-value |
|---|---|---|---|
| Test Food Group 1 (Group of Daily UPM Intake of 600 mg) | 2.9 | 9.0 | 0.535 |
| Test Food Group 2 (Group of Daily UMP Intake of 300 mg) | 22.4 | 32.7 | 0.056 |
| Placebo Group | 2.6 | 14.2 | - |

In the group of daily UMP intake of 300 mg of the subjects who had the composite memory standardized score of

**EP 4 588 364 A1**

less than 90 and were under the age of 60, the increase in the executive function standardized score was 22.4, which significantly exceeded the medically significant value of 10, and the p-value was in the $p < 0.10$ range, which was considered to be a significant difference.

This indicates that UMP produces a remarkable and significant effect of improving the executive function for subjects having the composite memory standardized score of less than 83.5 and under the age of 60 (40s and 50s) when the daily UMP intake is around 300 mg.

This indicates that the UMP produces a higher effect of improving the executive function for subjects having the composite memory standardized score of less than 83.5 and under the age of 60.

(Example 7-3) Effect of Improving Cognitive Flexibility Standardized Scores for Subjects under the age of 60 and Having Composite Memory Standardized Score of Less Than 83.5

Results were extracted for the cognitive flexibility standardized scores for subjects who were under the age of 60 and had a composite memory standardized score of less than 83.5 prior to UMP ingestion in the test conducted by the method described in Example 1. The amount of change in cognitive flexibility standardized scores before and after the test is shown in FIG. 33 and Table 34.

[Table 34]

| Test Groups | Amount of Change in Cognitive Flexibility Standardized Score | Standard Deviation | p-value |
|---|---|---|---|
| Test Food Group 1 (Group of Daily UPM Intake of 600 mg) | 2.4 | 9.3 | 0.484 |
| Test Food Group 2 (Group of Daily UMP Intake of 300 mg) | 24.7 | 33.3 | 0.027 |
| Placebo Group | 1.3 | 14.4 | - |

In the subgroups having the composite memory standardized score of less than 83.5 and under the age of 60, there was also a trend to increase the amount of change in cognitive flexibility standardized scores for Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects having the composite memory standardized score of less than 83.5 and under the age of 60, the increase in the cognitive flexibility standardized score was 24.7, which significantly exceeded the medically significant value of 10, and the p-value was in the $p < 0.05$ range, which was considered to be a significant difference.

This indicates that the UMP produces a remarkable and significant effect of improving the cognitive flexibility for subjects having a composite memory standardized score of less than 83.5 and under the age of 60.

(Example 7-4) Effect of Improving Complex Attention Standardized Scores for Subjects Under Age of 60 and Having Composite Memory Standardized Score of Less Than 83.5.

Results were extracted for the complex attention standardized score for subjects who were under the age of 60 and had a composite memory standardized score of less than 83.5 prior to UMP ingestion in the test conducted by the method described in Example 1. The amount of change in the complex attention standardized scores before and after the test is shown in FIG. 34 and Table 35.

[Table 35]

| Test Groups | Amount of Change in Complex Attention Standardized Score | Standard Deviation | p-value |
|---|---|---|---|
| Test Food Group 1 (Group of Daily UPM Intake of 600 mg) | 1.3 | 14.0 | 0.317 |
| Test Food Group 2 (Group of Daily UMP Intake of 300 mg) | 28.7 | 36.4 | 0.041 |
| Placebo Group | -0.4 | 19.2 | - |

In subjects who had a composite memory standardized score of less than 83.5 and were under the age of 60 (40s

38

and 50s), the placebo group showed decreased complex attention standardized scores before and after the test, whereas both Test Food Groups 1 and 2 showed increased complex attention standardized scores.

Particularly, in the group of daily UMP intake of 300 mg of the subjects having the composite memory standardized score of less than 83.5 and under the age of 60, the increase in the complex attention standardized score was 28.7, which significantly exceeded the medically significant value of 10, and the p-value was in the p < 0.05 range, which was considered to be a significant difference.

This indicates that the UMP produces a remarkable and significant effect of improving the complex attention for subjects having the composite memory standardized score of less than 83.5 and under the age of 60.

Furthermore, when comparing the results of Test Food Group 2 in this example with the results of Test Food Group 2 in Examples 3-4 and 5-4, this example has the highest amount of change in the complex attention standardized score before and the after the test and the p-value in this example is the lowest. This indicates that the UMP has a particularly strong effect of improving the complex attention for subjects having the composite memory standardized score of less than 83.5 and under the age of 60.

(Example 7-5) Effect of Improving Neurocognition Index (NCI) Standardized Score for Subjects Under Age of 60 and Having Composite Memory Standardized Score of Less Than 83.5

Results were extracted for the neurocognition index (NCI) standardized scores for subjects who were under the age of 60 and had a composite memory standardized score of less than 83.5 prior to UMP intake in the test conducted by the method described in Example 1. The amount of change in the neurocognition index (NCI) standardized scores before and after the test is shown in FIG. 35 and Table 36.

[Table 36]

| Test Groups | Amount of Change in NCI Standardized Score | Standard Deviation | p-value |
|---|---|---|---|
| Test Food Group 1 (Group of Daily UPM Intake of 600 mg) | 6.7 | 5.0 | 0.158 |
| Test Food Group 2 (Group of Daily UMP Intake of 300 mg) | 15.3 | 12.3 | 0.003 |
| Placebo Group | 3.2 | 8.7 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.

Particularly, in the group of daily UMP intake of 300 mg, the increase in the neurocognition index (NCI) standardized score was 15.3, which significantly exceeded the medically significant of 10, and the p-value was in the range of p < 0.05, which was determined to be a significant difference.

Furthermore, in the group of daily UMP intake of 300 mg, the amount of change in NCI standardized scores was increased when compared to that in Examples 6-6 tested for the subjects under the age of 60 and having the composite memory standardized score of less than 90, and Examples 5-5 tested for the subjects having the composite memory standardized score of less than 83.5.

It was found from the results that the agent for improving composite memory according to the present invention had a more pronounced and significant effect on subjects who had the composite memory standardized score of less than 83.5 and were under the age of 60, and that the neurocognitive function was remarkably improved with medical significance.

(Example 8) Effect of Improving Standardized Scores in Each Cognitive Domain for Subjects Having Composite Memory Standardized Score of 90 or More and Less Than 100

In Cognitrax, the individuals having the composite memory standardized score of 90 or more and less than 100 is determined to have the composite memory cognitive function relating to memory being within the normal range, but in order to examine what effects the UMP produces on those individuals, we focused on individuals whose standardized composite memory score before UMP intake was of 90 or more and less than 100.

(Example 8-1) Effect of Improving Composite Memory Standardized Scores for Subjects Having Composite Memory Standardized Score of 90 or More and Less Than 100

Results were extracted for the composite memory standardized score for subjects who had the composite memory standardized score before UMP intake of 90 or more and less than 100 in the test conducted by the method described in Example 1. The amount of change in the composite memory standardized scores before and after the test is shown in FIG. 36 and Table 37.

[Table 37]

| Test Groups | Amount of Change in Composite Memory Standardized Score | Standard Deviation | p-value |
|---|---|---|---|
| Test Food Group 1 (Group of Daily UPM Intake of 600 mg) | 6.0 | 14.6 | 0.296 |
| Test Food Group 2 (Group of Daily UMP Intake of 300 mg) | 3.6 | 17.8 | 0.475 |
| Placebo Group | -0.6 | 12.7 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.
The results of this example clearly show that the UMP exhibits the effect of improving the composite memory even for subjects having the composite memory standardized score of 90 or more and less than 100, and especially in the group of daily UMP intake of 600 mg, it exhibits the higher effect of improving the composite memory.

(Example 8-2) Effect of Improving Processing Speed Standardized Score for Subjects Having Composite Memory Standardized Score of 90 or More and Less Than 100

Results were extracted for the processing speed standardized scores for subjects who had the composite memory standardized score of 90 or more and less than 100 before UMP ingestion in the test conducted by the method described in Example 1. The amount of change in processing speed standardized scores before and after the test is shown in FIG. 37 and Table 38.

[Table 38]

| Test Groups | Amount of Change in Processing Speed Standardized Score | Standard Deviation | p-value |
|---|---|---|---|
| Test Food Group 1 (Group of Daily UPM Intake of 600 mg) | 3.0 | 13.1 | 0.720 |
| Test Food Group 2 (Group of Daily UMP Intake of 300 mg) | 6.7 | 11.6 | 0.230 |
| Placebo Group | 0.3 | 18.6 | - |

Both Test Food Group 1 (group of daily UMP intake of 600 mg) and Test Food Group 2 (group of daily UMP intake of 300 mg) expanded the increased range of the processing speed standardized scores before and after the test as compared to the placebo group.
This indicates that the UMP produces the effect of improving the processing speed even for subjects having a composite memory standardized score of 90 or more and less than 100.

(Example 8-3) Effect of improving Psychomotor Speed Standardized Scores for Subjects Having Composite Memory Standardized Score of 90 or More and Less Than 100

Results were extracted for the psychomotor speed standardized scores for subjects who had the composite memory standardized score of 90 or more and less than 100 before UMP ingestion in the test conducted by the method described in Example 1. The amount of change in psychomotor speed standardized scores before and after the test is shown in FIG. 38 and Table 39.

[Table 39]

| Test Groups | Amount of Change in Psychomotor Speed Standardized Score | Standard Deviation | p-value |
|---|---|---|---|
| Test Food Group 1 (Group of Daily UPM Intake of 600 mg) | 5.8 | 13.0 | 0.725 |
| Test Food Group 2 (Group of Daily UMP Intake of 300 mg) | 8.7 | 6.4 | 0.115 |
| Placebo Group | 3.6 | 7.5 | - |

Both Test Food Group 1 (group of daily UMP intake of 600 mg) and Test Food Group 2 (group of daily UMP intake of 300 mg) expanded the increased range of the psychomotor speed standardized scores before and after the test as compared to the placebo group.

This indicates that UMP produces the effect of improving the psychomotor speed even for subjects who have the composite memory standardized score of 90 or more and less than 100.

(Example 8-4) Effect of Improving Neurocognition Index (NCI) Standardized Score for Subjects Having Composite Memory Standardized Score of 90 or More and Less Than 100

Results were extracted for the neurocognition index (NCI) standardized scores for subjects who had the composite memory standardized scores of 90 or more and less than 100 before UMP ingestion in the test conducted by the method described in Example 1. The amount of change in the neurocognition index (NCI) standardized scores before and after the test is shown in FIG. 39 and Table 40.

[Table 40]

| Test Groups | Amount of Change in NCI Standardized Score | Standard Deviation | p-value |
|---|---|---|---|
| Test Food Group 1 (Group of Daily UPM Intake of 600 mg) | 8.2 | 13.5 | 0.945 |
| Test Food Group 2 (Group of Daily UMP Intake of 300 mg) | 4.2 | 5.7 | 0.634 |
| Placebo Group | 4.3 | 8.6 | - |

In Test Food Group 1 (group of daily UMP intake of 600 mg), a trend to expand the increased range of the increase in the neurocognition index (NCI) standardized score was observed when compared to the placebo group.

This indicates that UMP produces the effect of improving the neurocognitive function even for subjects who have the composite memory standardized score of 90 or more and less than 100.

(Example 9) Effect of Improving Standardized Scores in Each Cognitive Domain for Subjects Under Age of 60 and Having Composite Memory Standardized Score of 90 or More and Less Than 100

We focused on subjects who were under the age of 60 and had a composite memory standardized score before UMP intake of 90 or more and less than 100, and analysis was conducted.

(Example 9-1) Effect of Improving Composite Memory Standardized Scores for Subjects Under Age of 60 and Having Composite Memory Standardized Score of 90 or More and Less Than 100

Results were extracted for the composite memory standardized score for subjects who had the composite memory standardized score before UMP intake of 90 or more and less than 100 in the test conducted by the method described in Example 1. The amount of change in the composite memory standardized scores before and after the test is shown in FIG. 40 and Table 41.

[Table 41]

| Test Groups | Amount of Change in Composite Memory Standardized Score | Standard Deviation | p-value |
|---|---|---|---|
| Test Food Group 1 (Group of Daily UPM Intake of 600 mg) | 7.3 | 15.0 | 0.168 |
| Test Food Group 2 (Group of Daily UMP Intake of 300 mg) | 5.8 | 19.2 | 0.262 |
| Placebo Group | -2.7 | 11.2 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

The results of this example clearly show that the UMP exhibits the effect of improving the composite memory even for subjects who are under the age of 60 and have the composite memory standardized score of 90 or more and less than 100, and especially in the group of daily UMP intake of 600 mg, it exhibits the higher effect of improving the composite memory.

(Example 9-2) Effect of Improving Executive Function Standardized Scores for Subjects Under Age of 60 and Having Composite Memory Standardized Score of 90 or More and Less Than 100

Results were extracted for the executive function standardized scores for subjects who had the composite memory standardized scores of 90 or more and less than 100 prior to UMP ingestion in the test conducted by the method described in Example 1. The amount of change in the executive function standardized scores before and after the test is shown in FIG. 41 and Table 42.

[Table 42]

| Test Groups | Amount of Change in Executive Function Standardized Score | Standard Deviation | p-value |
|---|---|---|---|
| Test Food Group 1 (Group of Daily UPM Intake of 600 mg) | 10.9 | 16.5 | 0.632 |
| Test Food Group 2 (Group of Daily UMP Intake of 300 mg) | 1.0 | 6.7 | 0.540 |
| Placebo Group | 8.1 | 16.7 | - |

In Test Food Group 1 (group of daily UMP intake of 600 mg), there was an increased range of the increase in the standardized executive function scores before and after the test as compared to the placebo group.

The results of this example show that the UMP produces the effect of improving the executive function even for subjects who are under the age of 60 and have the composite memory standardized score f 90 or more and less than 100.

(Example 9-3) Effect of Improving the Complex Attention Standardized Scores for Subjects Under Age of 60 and Having Composite Memory Standardized Score of 90 or More and Less Than 100

Results were extracted for the complex attention standardized scores for subjects who had the composite memory standardized score before UMP ingestion of 90 or more and less than 100 in the test conducted by the method described in Example 1. The amount of change in the complex attention standardized scores before and after the test is shown in FIG. 42 and Table 43.

[Table 43]

| Test Groups | Amount of Change in Complex Attention Standardized Score | Standard Deviation | p-value |
|---|---|---|---|
| Test Food Group 1 (Group of Daily UPM Intake of 600 mg) | 9.0 | 20.8 | 0.855 |
| Test Food Group 2 (Group of Daily UMP Intake of 300 mg) | -0.1 | 8.0 | 0.184 |

(continued)

| Test Groups | Amount of Change in Complex Attention Standardized Score | Standard Deviation | p-value |
|---|---|---|---|
| Placebo Group | 7.7 | 34.7 | - |

Test Food Group 1 (group of daily UMP intake of 600 mg) expanded the increased range of the standardized executive function scores before and after the test as compared to the placebo group.

The results of this example show that the UMP produces the effect of improving the complex attention, even for subjects who are under the age of 60 and have the composite memory standardized score of 90 or more and less than 100.

(Example 9-4) Effect of Improving Working Memory Standardized Scores for Subjects Under Age of 60 and Having Composite Memory Standardized Scores of 90 or More and Less Than 100

Results were extracted for the working memory standardized scores for subjects who had the composite memory standardized scores of 90 or more and less than 100 before UMP ingestion in the test conducted by the method described in Example 1. The amount of change in working memory standardized scores before and after the test is shown in FIG. 43 and Table 44.

[Table 44]

| Test Groups | Amount of Change in Working Memory Standardized Score | Standard Deviation | p-value |
|---|---|---|---|
| Test Food Group 1 (Group of Daily UPM Intake of 600 mg) | 1.9 | 16.8 | 0.947 |
| Test Food Group 2 (Group of Daily UMP Intake of 300 mg) | 4.8 | 11.3 | 0.128 |
| Placebo Group | 0.6 | 16.1 | - |

Both Test Food Group 1 (group of daily UMP intake of 600 mg) and Test Food Group 2 (group of daily UMP intake of 300 mg) expanded the increased range of the executive function standardized scores before and after the test as compared to the placebo group.

The results of this example show that UMP produces the effect of improving the working memory even for subjects who are under the age of 60 and has the composite memory standardized score of 90 or more and less than 100.

(Example 9-5) Effect of Improving Processing Speed Standardized Scores for Subjects Under Age of 60 and Having Composite Memory Standardized Score of 90 or More and Less Than 100

Results were extracted for the processing speed standardized scores for subjects who were under the age of 60 and had the composite memory standardized score of 90 or more and less than 100 before UMP ingestion, in the study conducted by the method described in Example 1. The amount of change in processing speed standardized scores before and after the test is shown in FIG. 44 and Table 45.

[Table 45]

| Test Groups | Amount of Change in Processing Speed Standardized Score | Standard Deviation | p-value |
|---|---|---|---|
| Test Food Group 1 (Group of Daily UPM Intake of 600 mg) | 4.3 | 14.2 | 0.568 |
| Test Food Group 2 (Group of Daily UMP Intake of 300 mg) | 9.8 | 10.5 | 0.130 |
| Placebo Group | 0.8 | 21.2 | - |

Both Test Food Group 1 (group of daily UMP intake of 600 mg) and Test Food Group 2 (group of daily UMP intake of 300 mg) expanded the increased range of the processing speed standardized scores before and after the test as compared to the placebo group.

This indicates that the UMP produces the effect of improving the processing speed even for subjects who are under the age of 60 and have the composite memory standardized score of 90 or more and less than 100.

(Example 9-6) Effect of Improving Psychomotor Speed Standardized Scores for Subjects Under Age of 60 and Having Composite Memory Standardized Score of 90 or More and Less Than 100

Results were extracted for the psychomotor speed standardized scores for subjects who were under the age of 60 and had the composite memory standardized score before UMP ingestion of 90 or more and less than 100 in the test conducted by the method described in Example 1. The amount of change in psychomotor speed standardized scores before and after the test is shown in FIG. 45 and Table 46.

[Table 46]

| Test Groups | Amount of Change in Psychomotor Speed Standardized Score | Standard Deviation | p-value |
|---|---|---|---|
| Test Food Group 1 (Group of Daily UPM Intake of 600 mg) | 4.8 | 12.0 | 0.972 |
| Test Food Group 2 (Group of Daily UMP Intake of 300 mg) | 10.7 | 5.3 | 0.065 |
| Placebo Group | 4.8 | 7.7 | - |

In Test Food Group 2 (group of daily UMP intake of 300 mg), the increased range of the psychomotor speed standardized scores before and after the test was expanded as compared to the placebo group. The increased range of the psychomotor speed standardized scores exceeded 10, which was considered to have medical significance, and the p-value was in the range of $p < 0.10$, which was determined to show a significant trend.
This indicates that the UMP produces the effect of improving the psychomotor speed even for subjects who are under the age of 60 and have the composite memory standardized score of 90 or more and less than 100.

(Example 9-7) Effect of Improving Neurocognition Index (NCI) Standardized Score for Subjects Under Age of 60 and Having Composite Memory Standardized Score of 90 or More and Less than 100

Results were extracted for the neurocognition index (NCI) standardized scores for subjects who were under the age of 60 and had the composite memory standardized score of 90 or more and less than 100 before UMP ingestion in the test conducted by the method described in Example 1. The amount of change in the neurocognition index (NCI) standardized scores before and after the test is shown in FIG. 46 and Table 47.

[Table 47]

| Test Groups | Amount of Change in NCI Standardized Score | Standard Deviation | p-value |
|---|---|---|---|
| Test Food Group 1 (Group of Daily UPM Intake of 600 mg) | 10.3 | 13.7 | 0.694 |
| Test Food Group 2 (Group of Daily UMP Intake of 300 mg) | 5.7 | 5.1 | 0.272 |
| Placebo Group | 4.8 | 10.0 | - |

Both Test Food Group 1 (group of daily UMP intake of 600 mg) and Test Food Group 2 (group of daily UMP intake of 300 mg) expanded the increased range of the neurocognition index (NCI) standardized scores before and after the test as compared to the placebo group.
This indicates that UMP produces the effect of improving the neurocognitive function even for subjects who are under the age of 60 and have the composite memory standardized score of 90 or more and less than 100.
Some cognitive domains showed high improving effects on subjects who had the composite memory standardized score before UMP ingestion of 83.5 or more and less than 100, and so this is described as a reference example.

(Reference Example 1) Effect of Improving Working Memory Standardized Score for Subjects Having Composite Memory Standardized Score of 83.5 or More and Less Than 100

Results were extracted for the working memory standardized scores for subjects who had the composite memory standardized scores were of 83.5 or more and less than 100 before UMP ingestion in the test conducted by the method described in Example 1. The amount of change in working memory standardized scores before and after the test is shown in FIG. 47 and Table 48.

[Table 48]

| Test Groups | Amount of Change in Working Memory Standardized Score | Standard Deviation | p-value |
|---|---|---|---|
| Test Food Group 1 (Group of Daily UPM Intake of 600 mg) | 4.2 | 19.1 | 0.354 |
| Test Food Group 2 (Group of Daily UMP Intake of 300 mg) | 4.2 | 12.1 | 0.031 |
| Placebo Group | -0.8 | 13.3 | - |

The analysis was conducted on subjects who had the composite memory standardized score of 83.5 or more and less than 100, indicating that the working memory standardized scores were decreased before and after the test for the placebo group, whereas the working memory standardized scores were significantly increased for Test Food Group 1 (group of daily UMP intake of 600 mg) and Test Food Group 2 (group of daily UMP intake of 300 mg).
A significant (p: 0.031) effect of improving the working memory was observed for the group of UMP intake of 300 mg.
It is revealed from the results that the UMP has a working memory improving effect on subjects who have the composite memory standardized score of 83.5 or more and less than 100, and especially when the daily intake is around 300 mg, it has a remarkable and significant working memory improving effect.

(Reference Example 2) Effect of Improving Processing Speed Standardized Score for Subjects Having Composite Memory Standardized Score of 83.5 or More and Less Than 100

Results were extracted for the processing speed standardized scores for subjects who had the composite memory standardized score of 83.5 or more and less than 100 before UMP ingestion in the test conducted by the method described in Example 1. The amount of change in processing speed standardized scores before and after the test is shown in FIG. 48 and Table 49.

[Table 49]

| Test Groups | Amount of Change in Processing Speed Standardized Score | Standard Deviation | p-value |
|---|---|---|---|
| Test Food Group 1 (Group of Daily UPM Intake of 600 mg) | 10.1 | 18.3 | 0.217 |
| Test Food Group 2 (Group of Daily UMP Intake of 300 mg) | 4.8 | 10.2 | 0.332 |
| Placebo Group | 0.9 | 16.3 | - |

Both Test Food Group 1 (group of daily UMP intake of 600 mg) and Test Food Group 2 (group of daily UMP intake of 300 mg) expanded the increased range of the processing speed standardized scores before and after the test as compared to the placebo group.
In particular, for Test Food Group 1 (group of daily UMP intake of 600 mg), the increase in the processing speed standardized score exceeded 10, which was considered to be medically significant.
This indicates that the UMP produces the effect of improving the processing speed even for subjects whose composite memory standardized score is 83.5 or more and less than 100.

(Reference Example 3) Effect of Improving Psychomotor Speed Standardized Score for Subjects Having Composite Memory Standardized Score of 83.5 or More and Less Than 100

Results were extracted for the psychomotor speed standardized scores for subjects who had the composite memory standardized score of 83.5 or more and less than 100 before UMP ingestion in the test conducted by the method described in Example 1. The amount of change in psychomotor speed standardized scores before and after the test is shown in FIG. 49 and Table 50.

[Table 50]

| Test Groups | Amount of Change in Psychomotor Speed Standardized Score | Standard Deviation | p-value |
|---|---|---|---|
| Test Food Group 1 (Group of Daily UPM Intake of 600 mg) | 8.3 | 14.4 | 0.450 |
| Test Food Group 2 (Group of Daily UMP Intake of 300 mg) | 6.2 | 8.5 | 0.399 |
| Placebo Group | 3.6 | 6.9 | - |

Both Test Food Group 1 (group of daily UMP intake of 600 mg) and Test Food Group 2 (group of daily UMP intake of 300 mg) expanded the increased range of the psychomotor speed standardized scores before and after the test as compared to the placebo group.
This indicates that UMP produces the effect of improving the psychomotor speed even for subjects who have the composite memory standardized score of 83.5 or more and less than 100.

(Reference Example 4) Effect of Improving Composite Memory Standardized Score for Subjects Under Age of 60 and Having Composite Memory Standardized Score of 83.5 or More and Less Than 100

Results were extracted for the composite memory standardized score for subjects who were under the age of 60 and had the composite memory standardized score before UMP ingestion of 83.5 or more and less than 100 in the test conducted by the method described in Example 1. The amount of change in the composite memory standardized scores before and after the test is shown in FIG. 50 and Table 51.

[Table 51]

| Test Groups | Amount of Change in Composite Memory Standardized Score | Standard Deviation | p-value |
|---|---|---|---|
| Test Food Group 1 (Group of Daily UPM Intake of 600 mg) | 9.1 | 13.1 | 0.399 |
| Test Food Group 2 (Group of Daily UMP Intake of 300 mg) | 3.9 | 17.9 | 0.980 |
| Placebo Group | 3.4 | 13.9 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.
This indicates that the UMP produces the effect of improving the composite memory for subjects who are under the age of 60 and have the composite memory standardized score of 83.5 or more and less than 100.

(Reference Example 5) Effect of Improving Working Memory Standardized Score for Subjects Under Age of 60 and Having Composite Memory Standardized Score of 83.5 or More and Less Than 100

Results were extracted for the working memory standardized scores for subjects who were under the age of 60 and had the composite memory standardized score before UMP ingestion of 83.5 or more and less than 100 in the test conducted by the method described in Example 1. The amount of change in working memory standardized scores before and after the test is shown in FIG. 51 and Table 52.

[Table 52]

| Test Groups | Amount of Change in Working Memory Standardized Score | Standard Deviation | p-value |
|---|---|---|---|
| Test Food Group 1 (Group of Daily UPM Intake of 600 mg) | 6.5 | 18.6 | 0.387 |
| Test Food Group 2 (Group of Daily UMP Intake of 300 mg) | 5.3 | 10.2 | 0.018 |
| Placebo Group | -0.4 | 13.9 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

A significant (p: 0.018) effect of improving the working memory was observed for Test Food Group 2 (group of daily UMP intake of 300 mg).

This indicates that the UMP produces the effect of improving the working memory even for subjects who are under the age of 60 and have the composite memory standardized score of 83.5 or more and less than 100.

(Reference Example 6) Effect of Improving Processing Speed Standardized Score for Subjects Under Age of 60 and Having Composite Memory Standardized Score of 83.5 or More and Less Than 100

Results were extracted for the processing speed standardized scores for subjects who were under the age of 60 and had the composite memory standardized score of 83.5 or more and less than 100 before UMP ingestion, in the test conducted by the method described in Example 1. The amount of change in processing speed standardized scores before and after the test is shown in FIG. 52 and Table 53.

[Table 53]

| Test Groups | Amount of Change in Processing Speed Standardized Score | Standard Deviation | p-value |
|---|---|---|---|
| Test Food Group 1 (Group of Daily UPM Intake of 600 mg) | 11.8 | 18.9 | 0.115 |
| Test Food Group 2 (Group of Daily UMP Intake of 300 mg) | 7.5 | 9.2 | 0.100 |
| Placebo Group | 0.6 | 18.1 | - |

Both Test Food Group 1 (group of daily UMP intake of 600 mg) and Test Food Group 2 (group of daily UMP intake of 300 mg) expanded the increased range of the processing speed standardized scores before and after the test as compared to the placebo group.

This indicates that the UMP produces the effect of improving the processing speed even for subjects who are under the age of 60 and have the composite memory standardized score of 83.5 or more and less than 100.

(Reference Example 7) Effect of Improving Psychomotor Speed Standardized Scores for Subjects Under Age of 60 and Having Composite Memory Standardized Score of 83.5 or More and Less Than 100

Results were extracted for the psychomotor speed standardized scores for subjects who were under the age of 60 and had the composite memory standardized score before UMP ingestion of 83.5 or more and less than 100 in the test conducted by the method described in Example 1. The amount of change in psychomotor speed standardized scores before and after the test is shown in FIG. 53 and Table 54.

[Table 54]

| Test Groups | Amount of Change in Psychomotor Speed Standardized Score | Standard Deviation | p-value |
|---|---|---|---|
| Test Food Group 1 (Group of Daily UPM Intake of 600 mg) | 8.1 | 14.2 | 0.503 |
| Test Food Group 2 (Group of Daily UMP Intake of 300 mg) | 7.7 | 8.6 | 0.231 |

(continued)

| Test Groups | Amount of Change in Psychomotor Speed Standardized Score | Standard Deviation | p-value |
|---|---|---|---|
| Placebo Group | 3.8 | 6.8 | - |

Both Test Food Group 1 (group of daily UMP intake of 600 mg) and Test Food Group 2 (group of daily UMP intake of 300 mg) expanded the increased range of the psychomotor speed standardized scores before and after the test as compared to the placebo group.

This indicates that the UMP produces the effect of improving the psychomotor speed even for subjects who are under the age of 60 and have the composite memory standardized score of 83.5 or more and less than 100.

(Example 10) Effect of Improving Standardized Scores in Each Cognitive Domain for Subjects Having Verbal Memory Standardized Score of Less Than 90

In order to verify what effects the present invention will produce on subjects having a relatively low cognitive function relating to verbal memory, we conducted an analysis focusing on subjects who had a verbal memory standardized score before UMP ingestion of less than 90.

(Example 10-1) Effect of Improving Composite Memory Standardized Score for Subjects Having Verbal Memory Standardized Score of Less Than 90

Results were extracted for the composite memory standardized scores for subjects who had the verbal memory standardized score before UMP ingestion of less than 90 in the test conducted by the method described in Example 1. The amount of change in the composite memory standardized scores before and after the test is shown in FIG. 54 and Table 55.

[Table 55]

| Test Groups | Amount of Change in Composite Memory Standardized Score | Standard Deviation | p-value |
|---|---|---|---|
| Test Food Group 1 (Group of Daily UPM Intake of 600 mg) | 15.8 | 17.1 | 0.025 |
| Test Food Group 2 (Group of Daily UMP Intake of 300 mg) | 8.6 | 21.0 | 0.204 |
| Placebo Group | 4.2 | 21.0 | - |

Both Test Food Group 1 (group of daily UMP intake of 600 mg) and Test Food Group 2 (group of daily UMP intake of 300 mg) expanded the increased range of the composite memory standardized scores before and after the test as compared to the placebo group.

In Test Food Group 1 (group of daily UMP intake of 600 mg), the increase in the composite memory standardized score before and after the test was 15.8, which significantly exceeded the medically significant value of 10. The p-value range was < 0.05, which was determined to be a significant difference.

The results of this example clearly show that the UMP exhibits the effect of improving the composite memory for subjects having the verbal memory standardized score of less than 90, and especially in the group of daily UMP intake of 600 mg, it exhibits a remarkable and significant effect of improving the composite memory.

(Example 10-2) Effect of Improving Executive Function Standardized Scores for Subjects Having Verbal Memory Standardized Score of Less Than 90

Results were extracted for the executive function standardized scores for subjects who had the verbal memory standardized score of less than 90 in the test conducted by the method described in Example 1. The amount of change in the executive function standardized scores before and after the test is shown in FIG. 55 and Table 56.

[Table 56]

| Test Groups | Amount of Change in Executive Function Standardized Score | Standard Deviation | p-value |
|---|---|---|---|
| Test Food Group 1 (Group of Daily UPM Intake of 600 mg) | 5.0 | 13.8 | 0.352 |
| Test Food Group 2 (Group of Daily UMP Intake of 300 mg) | 12.3 | 22.2 | 0.076 |
| Placebo Group | 3.3 | 13.2 | - |

Even in the subgroup with the verbal memory standardized score of less than 90, there was a trend to increase the amount of change in executive function standardized scores in Test Food Group 1 (group of daily UMP intake of 600 mg) and Test Food Group 2 (group of daily UMP intake of 300 mg) as compared to the placebo group.

When comparing Test Food Group 1 with Test Food Group 2, it was clear that the amount of change was higher for Test Food Group 2 (group of daily UMP intake of 300 mg).

Particularly, in the group of daily UMP intake of 300 mg of the subjects having the verbal memory standardized score of less than 90, the increase in the executive function standardized score was 12.3, which exceeded the medically significant value of 10, and the p-value was in the range of $0.05 \leq p < 0.10$, which was considered to indicate a significant trend.

When comparing the results of Test Food Group 2 in Example 2-2 with those of Test Food Group 2 in this example, it is revealed that the increase in the executive function standardized scores for the subgroup of subjects having the verbal memory standardized score of less than 90 in this example is higher than the increase in the executive function standardized scores for all subjects in Example 2-2.

This indicates that the UMP produces a higher effect of improving the executive function for subjects who have the verbal memory standardized score of less than 90.

(Example 10-3) Effect of Improving Cognitive Flexibility Standardized Scores for Subjects Having Verbal Memory Standardized Score of Less Than 90

Results were extracted for the cognitive flexibility standardized scores for subjects who had the verbal memory standardized score of less than 90 in the test conducted using the method described in Example 1. The amount of change in cognitive flexibility standardized scores before and after the test is shown in FIG. 56 and Table 57.

[Table 57]

| Test Groups | Amount of Change in Cognitive Flexibility Standardized Score | Standard Deviation | p-value |
|---|---|---|---|
| Test Food Group 1 (Group of Daily UPM Intake of 600 mg) | 5.2 | 15.2 | 0.232 |
| Test Food Group 2 (Group of Daily UMP Intake of 300 mg) | 13.4 | 23.6 | 0.037 |
| Placebo Group | 2.3 | 13.0 | - |

Even in the subgroup with the verbal memory standardized score of less than 90, there was a trend to increase the amount of change in cognitive flexibility standardized scores in Test Food Group 1 (group of daily UMP intake of 600 mg) and Test Food Group 2 (group of daily UMP intake of 300 mg) as compared to the placebo group.

Particularly, in the group of daily UMP intake of 300 mg of the subjects (all ages) having the verbal memory standardized score of less than 90, the increase in the cognitive flexibility standardized score was 13.4, which exceeded the medically significant value of 10, and the p-value was in the $p < 0.05$ range, which was determined to be a significant difference.

Furthermore, when comparing the results of Examples 2-3 with those of this example, the amount of change in scores was increased for Test Food Group 2.

This indicates that the UMP produces a higher effect of improving the cognitive flexibility for subjects having the verbal memory standardized scores of less than 90.

(Example 10-4) Effect of Improving Complex Attention Standardized Scores for Subjects Having Verbal Memory Standardized Score of Less Than 90

Results were extracted for the complex attention standardized scores for subjects who had the verbal memory standardized score of less than 90 in the test conducted by the method described in Example 1. The amount of change in the complex attention standardized scores before and after the test is shown in FIG. 57 and Table 58.

[Table 58]

| Test Groups | Amount of Change in Complex Attention Standardized Score | Standard Deviation | p-value |
|---|---|---|---|
| Test Food Group 1 (Group of Daily UPM Intake of 600 mg) | 6.9 | 25.5 | 0.038 |
| Test Food Group 2 (Group of Daily UMP Intake of 300 mg) | 13.5 | 29.4 | 0.047 |
| Placebo Group | -1.2 | 17.5 | - |

Even in the subgroup with the verbal memory standardized score of less than 90, there was a trend to increase the amount of change in complex attention standardized scores in Test Food Group 1 (group of daily UMP intake of 600 mg) and Test Food Group 2 (group of daily UMP intake of 300 mg) as compared to the placebo group.
Particularly, in the group of daily UMP intake of 300 mg of the subjects (all ages) who had a verbal memory standardized score of less than 90, the increase in complex attention standardized score was 13.5, which exceeded the medically significant of 10. The p-values were in the range of $p < 0.05$, which was determined to be a significant difference in both Test Food Group 1 (group of daily UMP intake of 600 mg) and Test Food Group 2 (group of daily UMP intake of 300 mg).
This indicates that the UMP produces the effect of improving the complex attention for subjects who have the verbal memory standardized score of less than 90.

(Example 10-5) Effect of Improving Working Memory Standardized Scores for Subjects Having Verbal Memory Standardized Score of Less Than 90

Results were extracted for the working memory standardized scores for subjects who had the verbal memory standardized score of less than 90 in the test conducted by the method described in Example 1. The amount of change in working memory standardized scores before and after the test is shown in FIG. 58 and Table 59.

[Table 59]

| Test Groups | Amount of Change in Working Memory Standardized Score | Standard Deviation | p-value |
|---|---|---|---|
| Test Food Group 1 (Group of Daily UPM Intake of 600 mg) | -0.6 | 16.5 | 0.667 |
| Test Food Group 2 (Group of Daily UMP Intake of 300 mg) | 2.6 | 9.8 | 0.181 |
| Placebo Group | -0.1 | 14.7 | - |

In the subgroup with the verbal memory standardized score of less than 90, there was also a trend to increase the amount of change in working memory standardized scores in Test Food Group 2 (group of daily UMP intake of 300 mg) as compared to the placebo group.
This indicates that the UMP produces the effect of improving the working memory for subjects who have the verbal memory standardized score of less than 90.

(Example 10-6) Effect of Improving Processing Speed Standardized Scores for Subjects Having Verbal Memory Standardized Score of Less Than 90

Results were extracted for the processing speed standardized scores for subjects who had the verbal memory standardized score of less than 90 in the test conducted by the method described in Example 1. The amount of change in processing speed standardized scores before and after the test is shown in FIG. 59 and Table 60.

[Table 60]

| Test Groups | Amount of Change in Processing Speed Standardized Score | Standard Deviation | p-value |
|---|---|---|---|
| Test Food Group 1 (Group of Daily UPM Intake of 600 mg) | 6.7 | 17.2 | 0.365 |
| Test Food Group 2 (Group of Daily UMP Intake of 300 mg) | 2.8 | 10.8 | 0.761 |
| Placebo Group | 6.2 | 10.3 | - |

In the subgroup having the verbal memory standardized score of less than 90, there was a trend to increase the amount of change in processing speed standardized scores in Test Food Group 1 (group of daily UMP intake of 600 mg) as compared to the placebo group.

This indicates that the UMP produces the effect of improving the processing speed for subjects who have the verbal memory standardized score of less than 90.

(Example 10-7) Effect of Improving Neurocognition Index (NCI) Standardized Scores for Subjects Having Verbal Memory Standardized Score of Less Than 90

Results were extracted for the neurocognition index (NCI) standardized scores for subjects who had the verbal memory standardized score of less than 90 in the test conducted by the method described in Example 1. The amount of change in the neurocognition index (NCI) standardized scores before and after the test is shown in FIG. 60 and Table 61.

[Table 61]

| Test Groups | Amount of Change in NCI Standardized Score | Standard Deviation | p-value |
|---|---|---|---|
| Test Food Group 1 (Group of Daily UPM Intake of 600 mg) | 8.5 | 10.4 | 0.023 |
| Test Food Group 2 (Group of Daily UMP Intake of 300 mg) | 8.8 | 10.9 | 0.027 |
| Placebo Group | 2.6 | 8.0 | - |

In both Test Food Group 1 (group of daily UMP intake of 600 mg) and Test Food Group 2 (group of daily UMP intake of 300 mg), a trend toward an increased range of increase in neurocognition index (NCI) standardized scores was observed when compared to the placebo group.

The p-values were in the range of $p < 0.05$, which was determined to be a significant difference in both Test Food Group 1 (group of daily UMP intake of 600 mg) and Test Food Group 2 (group of daily UMP intake of 300 mg).

This indicates that the UMP produces a significant effect of improving the neurocognitive function on healthy individuals having the verbal memory standardized score of less than 90.

The contents of Examples 2 to 10 as shown above will be summarized with a focus on the effect of improving composite memory.

As for the effect of improving the composite memory, the amount of change in the composite memory standardized score was increased as compared to the placebo group in Example 2-1 analyzed for all the subjects, indicating that the UMP improves the composite memory. The results are supported by the fact that the amount of change in the composite memory standardized score was also increased in the analysis of the subject subgroups in Examples 3-1 to 10-1 as compared to the placebo group.

In particular, for the following subject subgroups, the increase in the composite memory standardized score exceeded 10, indicating that the UMP improves the composite memory with medical significance. In addition, corresponding examples are shown in parentheses.

All subjects: 600 mg (Example 2-1):

Under Age of 60: 600 mg (Example 3-1);
Composite memory standardized score of less than 90 before UMP ingestion: 600 mg (Example 4-1) or 300 mg (Example 4-1);
Composite memory standardized score before UMP ingestion of less than 83.5: 600 mg (Example 5-1) or 300 mg (Example 5-1);

Under the age of 60 and a composite memory standardized score of less than 90 before UMP ingestion: 600 mg (Example 6-1) or 300 mg (Example 6-1);
Under the age of 60 and a composite memory standardized score of less than 83.5 before UMP ingestion: 600 mg (Example 7-1) or 300 mg (Example 7-1);
Verbal memory standardized score of less than 90 before UMP ingestion: 600 mg (Example 10-1).

In particular, for the following subject subgroups, the increase in the composite memory standardized score was higher than 10 and the p-value was less than 0.10, indicating that the UMP improved the composite memory with medical significance and clearly to an extent that is determined to be a significant difference or trend.

All subjects: 600 mg (Example 2-1):

Under Age of 60: 600 mg (Example 3-1);
Composite memory standardized score of less than 90 before UMP ingestion: 600 mg (Example 4-1);
Composite memory standardized score of less than 83.5 before UMP ingestion: 600 mg (Example 5-1) or 300 mg (Example 5-1);
Verbal memory standardized score before UMP ingestion less than 90: 600 mg (Example 10-1).

Based on the above results, the present invention can be the following aspects:

[1] An agent for improving composite memory of healthy individuals, the agent comprising uridylic acid as an active ingredient.
[2] The agent according to [1], wherein the active ingredient is only uridylic acid.
[3] The agent according to [1] or [2], wherein the agent simultaneously improves one or more selected from executive function, cognitive flexibility, complex attention, working memory, processing speed, and psychomotor speed, in addition to the composite memory of healthy individuals.
[4] The agent according to [1] or [2], wherein the agent simultaneously improves two or more selected from executive function, cognitive flexibility, complex attention, working memory, processing speed, and psychomotor speed, in addition to the composite memory of healthy individuals.
[5] The agent according to any one of [1] to [4], wherein a content of uridylic acid is 450 mg or more and less than 750 mg, as converted into a daily dosage of uridylic acid.
[6] The agent according to [5], wherein the agent improves all of composite memory, executive function, cognitive flexibility, processing speed, and neurocognitive function of healthy individuals.
[7] The agent according to any one of [1] to [4], wherein a content of uridylic acid is 150 mg or more and less than 450 mg, as converted into a daily dosage of uridylic acid.
[8] The agent according to [7], wherein the agent improves all of composite memory, executive function, cognitive flexibility, working memory, and neurocognitive function of healthy individuals.
[9] The agent according to any one of [1] to [4], wherein a content of uridylic acid is 450 mg or more and less than 750 mg as converted into a daily dosage of uridylic acid, and the agent improves the composite memory of healthy individuals under the age of 60.
[10] The agent according to [9], wherein the agent improves all of composite memory, executive function, cognitive flexibility, complex attention, processing speed, and neurocognitive function of healthy individuals.
[11] The agent according to any one of [1] to [4], wherein a content of uridylic acid is 150 mg or more and less than 450 mg as converted into a daily dosage of uridylic acid, and the agent improves the composite memory of healthy individuals under the age of 60.
[12] The agent according to [11], wherein the agent improves all of composite memory, executive function, cognitive flexibility, complex attention, working memory, processing speed, and neurocognitive function of healthy individuals.
[13] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of less than 90.
[14] The agent according to [13], wherein the agent improves all of composite memory, executive function, cognitive flexibility, and neurocognitive function of healthy subjects.
[15] The agent according to any one of [1] to [4], wherein a content of uridylic acid is 150 mg or more and less than 450 mg as converted into a daily dosage of uridylic acid, and the agent improves the composite memory of healthy subjects having a composite memory standardized score of less than 90.
[16] The agent according to [15], wherein the agent improves all of composite memory, executive function, cognitive flexibility, complex attention, working memory, and neurocognitive function of healthy individuals.
[17] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of less than 83.5.

[18] The agent according to [17], wherein the agent improves all of composite memory, executive function, cognitive flexibility, complex attention, and neurocognitive function of healthy individuals.

[19] The agent according to any one of [1] to [4], wherein a content of uridylic acid is 150 mg or more and less than 450 mg as converted into a daily dosage of uridylic acid, and the agent improves the composite memory of healthy subjects having a composite memory standardized score of less than 83.5.

[20] The agent according to [19], wherein the agent improves all of composite memory, cognitive flexibility, and complex attention of healthy individuals.

[21] The agent according to [19], wherein the agent improves all of composite memory, executive function, cognitive flexibility, complex attention, and neurocognitive function of healthy individuals.

[22] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of less than 90 and under the age of 60.

[23] The agent according to [22], wherein the agent improves all of composite memory, executive function, cognitive flexibility, complex attention, processing speed, and neurocognitive function of healthy individuals.

[24] The agent according to any one of [1] to [4], wherein a content of uridylic acid is 150 mg or more and less than 450 mg as converted into a daily dosage of uridylic acid, and the agent improves the composite memory of healthy individual who have a composite memory standardized score of less than 90 and are under the age of 60.

[25] The agent according to [24], wherein the agent improves all of composite memory, executive function, cognitive flexibility, complex attention, and neurocognitive function of healthy individuals.

[26] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of less than 83.5 and under the age of 60.

[27] The agent according to [26], wherein the agent improves all of composite memory, executive function, cognitive flexibility, complex attention, and neurocognitive function of healthy individuals.

[28] The agent according to any one of [1] to [4], wherein a content of uridylic acid is 150 mg or more and less than 450 mg as converted into a daily dosage of uridylic acid, and the agent improves the composite memory of healthy individual who have a composite memory standardized score of less than 83.5 and are under the age of 60.

[29] The agent according to [28], wherein the agent improves all of composite memory, executive function, cognitive flexibility, complex attention, and neurocognitive function of healthy individuals.

[30] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of 90 or more and less than 100.

[31] The agent according to [30], wherein the agent improves all of composite memory, processing speed, psychomotor speed, and neurocognitive function of healthy individuals.

[32] The agent according to any one of [1] to [4], wherein a content of uridylic acid is 150 mg or more and less than 450 mg as converted into a daily dosage of uridylic acid, and the agent improves the composite memory of healthy individuals having a composite memory standardized score of 90 or more and less than 100.

[33] The agent according to [32], wherein the agent improves all of composite memory, processing speed, psychomotor speed, and neurocognitive function of healthy individuals.

[34] The agent according to any one of [1] to [4], wherein a content of uridylic acid is 450 mg or more and less than 750 mg as converted into a daily dosage of uridylic acid, and the agent improves the composite memory of healthy individuals having a composite memory standardized score of 90 or more and less than 100 and under the age of 60.

[35] The agent according to [34], wherein the agent improves all of composite memory, executive function, working memory, processing speed, psychomotor speed, and neurocognitive function of healthy individuals.

[36] The agent according to any one of [1] to [4], wherein a content of uridylic acid is 150 mg or more and less than 450 mg as converted into a daily dosage of uridylic acid, and the agent improves the composite memory of healthy individuals having a composite memory standardized score of 90 or more and less than 100 and under the age of 60.

[37] The agent according to [36], wherein the agent improves all of composite memory, executive function, complex attention, working memory, processing speed, psychomotor speed, and neurocognitive function of healthy individuals.

[38] The agent according to any one of [1] to [4], wherein a content of uridylic acid is 450 mg or more and less than 750 mg as converted into a daily dosage of uridylic acid, and the agent improves the composite memory of healthy individuals having a verbal memory standardized score of less than 90.

[39] The agent according to [38], wherein the agent improves all of composite memory, executive function, cognitive flexibility, complex attention, processing speed, and neurocognitive function of healthy individuals.

[40] The agent according to any one of [1] to [4], wherein a content of uridylic acid is 150 mg or more and less than 450 mg as converted into a daily dosage of uridylic acid, and the agent improves the composite memory of healthy individuals having a verbal memory standardized score of less than 90.

[41] The agent according to [40], wherein the agent improves all of composite memory, executive function, cognitive flexibility, complex attention, working memory, and neurocognitive function of healthy individuals.

[42] The agent according to any one of [1] to [41], wherein the agent is continuously ingested for at least 12 weeks.

[43] A composition comprising the agent according to any one of [1] to [42].

[44] The composition according to [43], wherein the composition is a food or beverage, a prepared milk powder, an enteral nutrient, a health drink, or a pharmaceutical product.

[45] A method for improving composite memory of healthy individuals, comprising using the agent according to any one of [1] to [41].

[46] The method according to [45], wherein the agent is continuously ingested for at least 12 weeks.

[47] Uridylic acid for improving composite memory of healthy individuals.

[48] The uridylic acid according [47] for simultaneously improving one or more selected from executive function, cognitive flexibility, complex attention, working memory, neurocognitive function, processing speed, and psychomotor speed, in addition to the composite memory of healthy individuals.

[49] The uridylic acid according to [47], wherein the uridylic acid simultaneously improves two or more selected from executive function, cognitive flexibility, complex attention, working memory, processing speed, and psychomotor speed, in addition to the composite memory of healthy individuals.

[50] An agent comprising the uridylic acid according to any one of [47] to [49] as an active ingredient.

[51] The agent according to [50], wherein the active ingredient is only uridylic acid.

[52] The agent according to [50] or [51], wherein a content of uridylic acid is 10 mg to 1000 mg as converted into a daily dosage of uridylic acid.

[53] The agent according to [50] or [51], wherein a content of uridylic acid is 150 mg to less than 750 mg as converted into a daily dosage of uridylic acid.

[54] A composition comprising the agent according to any one of [50] to [53].

[55] The composition according to [54], wherein the composition is a food or beverage, a prepared milk powder, an enteral nutrient, a health drink, or a pharmaceutical product.

[56] Use of uridylic acid for improving composite memory of healthy individuals.

[57] The use of uridylic acid according to [56], for simultaneously improving one or more selected from executive function, cognitive flexibility, complex attention, working memory, neurocognitive function, processing speed, and psychomotor speed, in addition to the composite memory of healthy individuals.

[58] The use of uridylic acid according to [56], for simultaneously improving two or more selected from executive function, cognitive flexibility, complex attention, working memory, processing speed, and psychomotor speed, in addition to the composite memory of healthy individuals.

[59] The use of uridylic acid according to any one of [56] to [58], wherein the uridylic acid is continuously ingested for at least 12 weeks.

## Claims

1. An agent for improving composite memory of healthy individuals, the agent comprising uridylic acid as an active ingredient.

2. The agent according to claim 1, wherein the active ingredient is only uridylic acid.

3. The agent according to claim 1 or 2, wherein the agent simultaneously improves one or more selected from executive function, cognitive flexibility, complex attention, working memory, processing speed, and psychomotor speed, in addition to the composite memory of healthy individuals.

4. The agent according to claim 1 or 2, wherein the agent simultaneously improves two or more selected from executive function, cognitive flexibility, complex attention, working memory, processing speed, and psychomotor speed, in addition to the composite memory of healthy individuals.

5. The agent according to any one of claims 1 to 4, wherein a content of uridylic acid is 450 mg or more and less than 750 mg, as converted into a daily dosage of uridylic acid.

6. The agent according to claim 5, wherein the agent improves all of composite memory, executive function, cognitive flexibility, processing speed, and neurocognitive function of healthy individuals.

7. The agent according to any one of claims 1 to 4, wherein a content of uridylic acid is 150 mg or more and less than 450 mg, as converted into a daily dosage of uridylic acid.

8. The agent according to claim 7, wherein the agent improves all of composite memory, executive function, cognitive flexibility, working memory, and neurocognitive function of healthy individuals.

9. The agent according to any one of claims 1 to 4, wherein a content of uridylic acid is 450 mg or more and less than 750 mg as converted into a daily dosage of uridylic acid, and the agent improves the composite memory of healthy individuals under the age of 60.

10. The agent according to claim 9, wherein the agent improves all of composite memory, executive function, cognitive flexibility, complex attention, processing speed, and neurocognitive function of healthy individuals.

11. The agent according to any one of claims 1 to 4, wherein a content of uridylic acid is 150 mg or more and less than 450 mg as converted into a daily dosage of uridylic acid, and the agent improves the composite memory of healthy individuals under the age of 60.

12. The agent according to claim 11, wherein the agent improves all of composite memory, executive function, cognitive flexibility, complex attention, working memory, processing speed, and neurocognitive function of healthy individuals.

13. The agent according to any one of claims 1 to 4, wherein a content of uridylic acid is 450 mg or more and less than 750 mg as converted into a daily dosage of uridylic acid, and the agent improves the composite memory of healthy individuals having a composite memory standardized score of less than 90.

14. The agent according to claim 13, wherein the agent improves all of composite memory, executive function, cognitive flexibility, and neurocognitive function of healthy subjects.

15. The agent according to any one of claims 1 to 4, wherein a content of uridylic acid is 150 mg or more and less than 450 mg as converted into a daily dosage of uridylic acid, and the agent improves the composite memory of healthy individuals having a composite memory standardized score of less than 90.

16. The agent according to claim 15, wherein the agent improves all of composite memory, executive function, cognitive flexibility, complex attention, working memory, and neurocognitive function of healthy individuals.

17. The agent according to any one of claims 1 to 4, wherein a content of uridylic acid is 450 mg or more and less than 750 mg as converted into a daily dosage of uridylic acid, and the agent improves the composite memory of healthy individuals having a composite memory standardized score of less than 83.5.

18. The agent according to claim 17, wherein the agent improves all of composite memory, executive function, cognitive flexibility, complex attention, and neurocognitive function of healthy individuals.

19. The agent according to any one of claims 1 to 4, wherein a content of uridylic acid is 150 mg or more and less than 450 mg as converted into a daily dosage of uridylic acid, and the agent improves the composite memory of healthy individuals having a composite memory standardized score of less than 83.5.

20. The agent according to claim 19, wherein the agent improves all of composite memory, cognitive flexibility, and complex attention of healthy individuals.

21. The agent according to claim 19, wherein the agent improves all of composite memory, executive function, cognitive flexibility, complex attention, and neurocognitive function of healthy individuals.

22. The agent according to any one of claims 1 to 4, wherein a content of uridylic acid is 450 mg or more and less than 750 mg as converted into a daily dosage of uridylic acid, and the agent improves the composite memory of healthy individuals having a composite memory standardized score of less than 90 and under the age of 60.

23. The agent according to claim 22, wherein the agent improves all of composite memory, executive function, cognitive flexibility, complex attention, processing speed, and neurocognitive function of healthy individuals.

24. The agent according to any one of claims 1 to 4, wherein a content of uridylic acid is 150 mg or more and less than 450 mg as converted into a daily dosage of uridylic acid, and the agent improves the composite memory of healthy individuals having a composite memory standardized score of less than 90 and under the age of 60.

25. The agent according to claim 24, wherein the agent improves all of composite memory, executive function, cognitive flexibility, complex attention, and neurocognitive function of healthy individuals.

26. The agent according to any one of claims 1 to 4, wherein a content of uridylic acid is 450 mg or more and less than 750 mg as converted into a daily dosage of uridylic acid, and the agent improves the composite memory of healthy individuals having a composite memory standardized score of less than 83.5 and under the age of 60.

27. The agent according to claim 26, wherein the agent improves all of composite memory, executive function, cognitive flexibility, complex attention, and neurocognitive function of healthy individuals.

28. The agent according to any one of claims 1 to 4, wherein a content of uridylic acid is 150 mg or more and less than 450 mg as converted into a daily dosage of uridylic acid, and the agent improves the composite memory of healthy individuals having a composite memory standardized score of less than 83.5 and under the age of 60.

29. The agent according to claim 28, wherein the agent improves all of composite memory, executive function, cognitive flexibility, complex attention, and neurocognitive function of healthy individuals.

30. The agent according to any one of claims 1 to 4, wherein a content of uridylic acid is 450 mg or more and less than 750 mg as converted into a daily dosage of uridylic acid, and the agent improves the composite memory of healthy individuals having a composite memory standardized score of 90 or more and less than 100.

31. The agent according to claim 30, wherein the agent improves all of composite memory, processing speed, psychomotor speed, and neurocognitive function of healthy individuals.

32. The agent according to any one of claims 1 to 4, wherein a content of uridylic acid is 150 mg or more and less than 450 mg as converted into a daily dosage of uridylic acid, and the agent improves the composite memory of healthy individuals having a composite memory standardized score of 90 or more and less than 100.

33. The agent according to claim 32, wherein the agent improves all of composite memory, processing speed, psychomotor speed, and neurocognitive function of healthy individuals.

34. The agent according to any one of claims 1 to 4, wherein a content of uridylic acid is 450 mg or more and less than 750 mg as converted into a daily dosage of uridylic acid, and the agent improves the composite memory of healthy individuals who have a composite memory standardized score of 90 or more and less than 100 and who are under the age of 60.

35. The agent according to claim 34, wherein the agent improves all of composite memory, executive function, working memory, processing speed, psychomotor speed, and neurocognitive function of healthy individuals.

36. The agent according to any one of claims 1 to 4, wherein the uridylic acid content is 150 mg or more and less than 450 mg as converted into a daily dosage of uridylic acid, and the agent improves the composite memory of healthy individuals who have a composite memory standardized score of 90 or more and less than 100 and who are under the age of 60.

37. The agent according to according to 36, wherein the agent improves all of composite memory, executive function, complex attention, working memory, processing speed, psychomotor speed, and neurocognitive function of healthy individuals.

38. The agent according to any one of claims 1 to 4, wherein a content of uridylic acid is 450 mg or more and less than 750 mg as converted into a daily dosage of uridylic acid, and the agent improves the composite memory of healthy individuals having a verbal memory standardized score of less than 90.

39. The agent according to claim 38, wherein the agent improves all of composite memory, executive function, cognitive flexibility, complex attention, processing speed, and neurocognitive function of healthy individuals.

40. The agent according to any one of claims 1 to 4, wherein a content of uridylic acid is 150 mg or more and less than 450 mg as converted into a daily dosage of uridylic acid, and the agent improves the composite memory of healthy individuals having a verbal memory standardized score of less than 90.

41. The agent according to claim 40, wherein the agent improves all of composite memory, executive function, cognitive flexibility, complex attention, working memory, and neurocognitive function of healthy individuals.

42. The agent according to any one of claims 1 to 41, wherein the agent is continuously ingested for at least 12 weeks.

43. A composition comprising the agent according to any one of claims 1 to 42.

44. A method for improving composite memory of healthy individuals, comprising using the agent according to any one of claims 1 to 41.

45. The method according to claim 44, wherein the agent is continuously ingested for at least 12 weeks.

46. Uridylic acid for improving composite memory of healthy individuals.

47. The uridylic acid according claim 46 for simultaneously improving one or more selected from executive function, cognitive flexibility, complex attention, working memory, neurocognitive function, processing speed, and psychomotor speed, in addition to the composite memory of healthy individuals.

48. Use of uridylic acid for improving composite memory of healthy individuals.

49. The use of uridylic acid according to claim 48, for simultaneously improving one or more selected from executive function, cognitive flexibility, complex attention, working memory, neurocognitive function, processing speed, and psychomotor speed, in addition to the composite memory of healthy individuals.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 1 1]

[FIG. 1 2]

[FIG. 1 3]

[FIG. 1 4]

[FIG. 15]

[FIG. 16]

[FIG. 17]

[FIG. 18]

EP 4 588 364 A1

[FIG. 1 9]

[FIG. 2 0]

68

[FIG. 2 1]

[FIG. 2 2]

[FIG. 2 3]

[FIG. 2 4]

[FIG. 2 5]

[FIG. 2 6]

[FIG. 27]

[FIG. 28]

[FIG. 2 9]

[FIG. 3 0]

[FIG. 3 1]

[FIG. 3 2]

[FIG. 3 3]

[FIG. 3 4]

[FIG. 3 5]

[FIG. 3 6]

[FIG. 3 7]

[FIG. 3 8]

[FIG. 3 9]

[FIG. 4 0]

[FIG. 4 1]

[FIG. 4 2]

[FIG. 4 3]

[FIG. 4 4]

[FIG. 45]

[FIG. 46]

[FIG. 4 7]

[FIG. 4 8]

[FIG. 49]

Test Food Group 1    Test Food Group 2    Placebo Group

[FIG. 50]

Test Food Group 1    Test Food Group 2    Placebo Group

[FIG. 5 1]

[FIG. 5 2]

[FIG. 5 3]

[FIG. 5 4]

[FIG. 5 5]

[FIG. 5 6]

[FIG. 5 7]

[FIG. 5 8]

[FIG. 5 9]

[FIG. 6 0]

## INTERNATIONAL SEARCH REPORT

| International application No. |
| --- |
| **PCT/JP2023/033804** |

**A. CLASSIFICATION OF SUBJECT MATTER**

*A23L 33/13*(2016.01)i; *A61P 25/28*(2006.01)i; *A61K 31/7072*(2006.01)i
FI: A23L33/13; A61P25/28; A61K31/7072

According to International Patent Classification (IPC) or to both national classification and IPC

**B. FIELDS SEARCHED**

Minimum documentation searched (classification system followed by classification symbols)

A23L33/13; A61K31/7072; A61P25/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2013-64026 A (MASSACHUSETTS INSTITUTE OF TECHNOLOGY) 11 April 2013 (2013-04-11)<br>claims, experiment example 13, paragraphs [0226]-[0236] | 1-49 |
| X | WO 2016/083362 A1 (FONDEN FOR HELENE ELSASS CENTERET) 02 June 2016 (2016-06-02)<br>claims, column 7, lines 18-28 | 1, 3-49 |
| A | | 2 |
| X | JP 2001-233776 A (YAMASA SHOYU CO LTD) 28 August 2001 (2001-08-28)<br>claims, paragraphs [0013], [0017], example 1, tables 1, 2 | 1-48 |
| A | | 49 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **21 November 2023** | **05 December 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/033804**

| Patent document cited in search report | | | Publication date (day/month/year) | Patent family member(s) | | | Publication date (day/month/year) |
|---|---|---|---|---|---|---|---|
| JP | 2013-64026 | A | 11 April 2013 | US claims, example 13, paragraphs [0219]-[0229] EP CN | 2005/0203053 2145627 101072569 | A1 A1 A | |
| WO | 2016/083362 | A1 | 02 June 2016 | (Family: none) | | | |
| JP | 2001-233776 | A | 28 August 2001 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020188755 A
- JP 2014532696 A
- JP 2014534226 A
- JP 2001233776 A

**Non-patent literature cited in the description**

- Diagnostic and Statistical Manual of Mental Disorders V (DSM-5). American Psychiatric Association
- **HARUO HANYU**. Answering Questions from Clinical Environment: Treatment for Memory Loss Q&A (p. 1). Chugai -Igakusha Co., Ltd, July 2020
- Dementia Clinical Practice Guidelines. Igaku Shoin, Inc, 01 August 2017, 8
- *Neuropsychol Dev Cogn B Aging Neuropsychol Cogn*, June 2004, vol. 11 (2-3), 304-324
- *Japanese Journal of Health & Research*, vol. 2021 (42), 37-48
- *CHEMICAL ABSTRACTS*, 58-97-9